(19) Europäisches Patentamt
European Patent Office
Office européen des brevets

(11) Veröffentlichungsnummer: **0 331 609**
**A2**

# (12) EUROPÄISCHE PATENTANMELDUNG

(21) Anmeldenummer: **89710005.3**

(22) Anmeldetag: **18.01.89**

(51) Int. Cl.4: **A 61 K 37/64**
C 07 K 5/06

(30) Priorität: **27.01.88 DE 3802303**
**30.01.88 DE 3802760**
**22.04.88 DE 3813819**

(43) Veröffentlichungstag der Anmeldung:
**06.09.89 Patentblatt 89/36**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80 (DE)**

(72) Erfinder: **Rüger, Wolfgang, Dr.**
**Parkstrasse 10**
**D-6233 Kelkheim (Taunus) (DE)**

**Urbach, Hansjörg, Dr.**
**Le Lavandoustrasse 41**
**D-6242 Kronberg (Taunus) (DE)**

**Henning, Rainer, Dr.**
**Rotenhofstrasse 31**
**D-6234 Hattersheim am Main (DE)**

**Stechl, Jens, Dr.**
**Loreleistrasse 7**
**D-6230 Frankfurt am Main 80 (DE)**

**Usinger, Patricia, Dr.**
**Hauptstrasse 54**
**D-6239 Eppstein (Taunus) (DE)**

**Hock, Franz, Dr.**
**Alstadt 19**
**D-6110 Dieburg (DE)**

Patentansprüche für folgende Vertragsstaaten: ES + GR.
Die Bezeichnung der Erfindung wurde geändert (Richtlinien für die Prüfung im EPA, A-III, 7.3).

## (54) Angiotensin-Converting-Enzym-Hemmstoffe mit psychotroper Wirkung.

(57) Die Erfindung betrifft die Verwendung von Angiotensin-Converting-Enzyme-Inhibitoren als Arzneimittel mit psychotroper, insbesondere anxiolytischer Wirkung, diese enthaltende Mittel sowie deren Verwendung bei der Behandlung oder Prophylaxe von Erkrankungen des zentralen Nervensystems, insbesondere von Angstzuständen.

Die Erfindung betrifft weiterhin neue Verbindungen der Formel

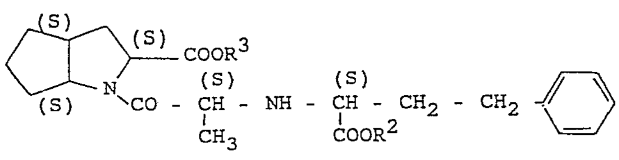

worin $R^2$ Wasserstoff oder Ethyl und $R^3$ n-Octyl bedeutet, Verfahren zu deren Herstellung, diese enthaltende Mittel und deren Verwendung als Arzneimittel.

EP 0 331 609 A2

## Beschreibung

### Verbindungen mit psychotroper Wirkung, diese enthaltende Mittel und deren Verwendung bei der Behandlung und Prophylaxe von Störungen des zentralen Nervensystems

Die Erfindung betrifft die Verwendung von Angiotensin-Converting-Enzyme-Inhibitoren (ACE-Inhibitoren) oder deren physiologisch verträglichen Salzen als Arzneimittel mit psychotroper, insbesondere anxiolytischer Wirkung, diese enthaltende Mittel sowie deren Verwendung bei der Herstellung entsprechender pharmazeutischer Zubereitungen.

Für diese neuartige Verwendung kommen beispielsweise Verbindungen der Formel I in Betracht,

$X^1 - X^2$ (I)

in welcher

$X^1$ 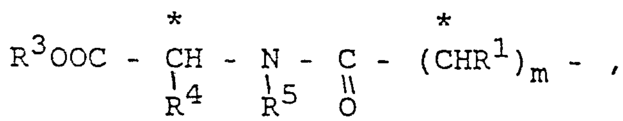

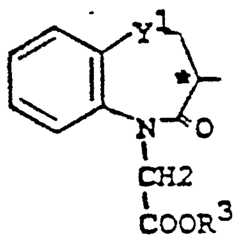 ,

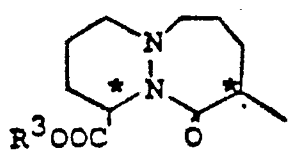

oder

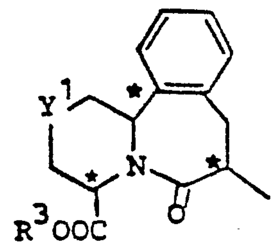 ,

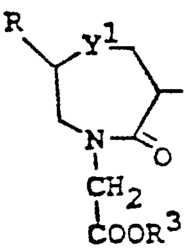

bedeutet,

$X^2$ $-CH_2SH$ , 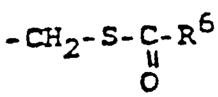 , 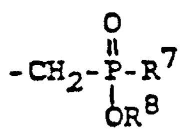 oder

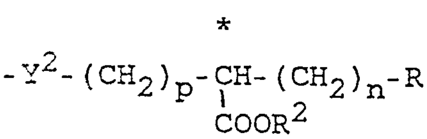 bedeutet,

$Y^1$ für -S- oder $-CH_2$-steht,
$Y^2$ für $-NR^9$- oder $-CH_2$- steht,
m = 0 oder 1 ist,
n = 0, 1 oder 2 ist,
p = 0 oder 1 ist,
R = Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,

einen gegebenenfalls substituierten araliphatischen Rest mit 7 - 32 C-Atomen,
einen gegebenenfalls substituierten alicyclisch-aliphatischen Rest mit 4 - 20 C-Atomen,
einen gegebenenfalls substituierten heteroaromatischen oder heteroaromatisch-($C_1$-$C_8$)-aliphatischen Rest mit 5 - 12 Ringatomen, oder
einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder,
falls nicht von vorstehenden Definitionen schon umfaßt, die erforderlichenfalls geschützte Seitenkette einer natürlich vorkommenden α-Aminosäure bedeutet,
$R^2$ und $R^3$ gleich oder verschieden sind und
Wasserstoff, einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen Rest der Formel

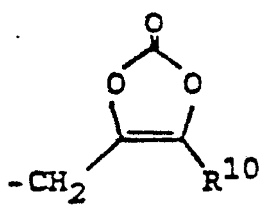

worin $R^{10}$ Wasserstoff, einen aliphatischen Rest mit 1-6 C-Atomen oder einen gegebenenfalls substituierten aromatischen Rest mit 6-12 C-Atomen bedeutet,
einen Rest der Formel

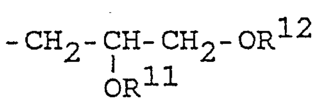

oder

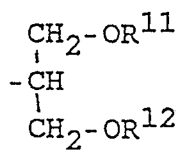

worin $R^{11}$ und $R^{12}$ gleich oder verschieden unabhängig voneinander Wasserstoff, einen gegebenenfalls substituierten Alkylrest mit 1-23 C-Atomen oder einen gegebenenfalls substituierten Acylrest mit 1-23 C-Atomen bedeuten,
bedeuten,
$R^4$ für Wasserstoff oder ($C_1$-$C_6$)Alkyl und
$R^5$ für ($C_1$-$C_6$)Alkyl, ($C_3$-$C_6$)-Cycloalkyl oder

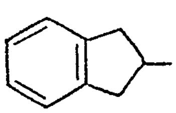

stehen oder

$R^4$ und $R^5$ zusammen mit den sie tragenden Atomen ein mono-, bi-, oder tricyclisches heterocyclisches Ringsystem mit 3 bis 15 Ring-C-Atomen bilden,
$R^6$ Wasserstoff, Amino, ($C_1$-$C_6$)-Alkyl, ($C_6$-$C_{12}$)-Aryl oder ($C_7$-$C_{13}$)-Aralkyl,
$R^7$ ($C_1$-$C_6$)-Alkyl oder ($C_7$-$C_{13}$)-Aralkyl, vorzugsweise $-(CH_2)_4-C_6H_5$,

$R^8$ $(C_1-C_6)$-Alkyl, das gegebenenfalls durch $(C_1-C_6)$-Alkanoyloxy monosubstituiert ist, vorzugsweise 2-Methyl-1-propionyloxy-propyl, und
$R^9$ Wasserstoff oder $(C_1-C_6)$-Alkyl bedeuten;
insbesondere Verbindungen der Formel II,

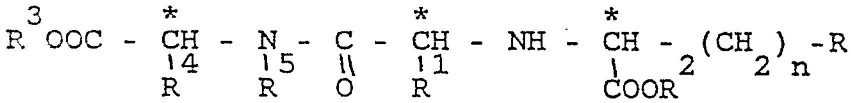 (II)

in welcher
n= 1 oder 2 ist,
R= Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclisch-aliphatischen Rest mit 7 - 14 C-Atomen,
einen gegebenenfalls substituierten heteroaromatischen oder heteroaromatisch-$(C_1-C_8)$-aliphatischen Rest mit 5 - 12 Ringatomen, oder
einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-$(C_1-C_8)$-aliphatischen Rest mit 5 - 12 Ringatomen oder,
falls nicht von vorstehenden Definitionen schon umfaßt, die erforderlichenfalls geschützte Seitenkette einer natürlich vorkommenden α-Aminosäure bedeutet,
$R^2$ und $R^3$ gleich oder verschieden sind und
Wasserstoff, einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7-32 C-Atomen,
einen Rest der Formel

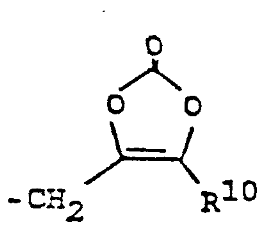

worin $R^{10}$ Wasserstoff, einen aliphatischen Rest mit 1-6 C-Atomen oder einen gegebenenfalls substituierten aromatischen Rest mit 6-12 C-Atomen bedeutet,
einen Rest der Formel

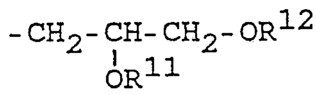

oder

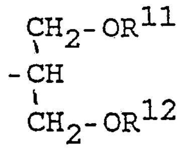

worin $R^{11}$ und $R^{12}$ gleich oder verschieden unabhängig voneinander Wasserstoff, einen gegebenenfalls substituierten Alkylrest mit 1-23 C-Atomen oder einen gegebenenfalls substituierten Acylrest mit 1-23 C-Atomen bedeuten,
bedeuten,
und
$R^4$ und $R^5$ zusammen mit den sie tragenden Atomen ein mono-, bi- oder tricyclisches heterocyclisches Ringsystem mit 3 bis 15 Ring-C-Atomen bilden.

Unter einem gegebenenfalls substituierten aliphatischen Rest versteht man einen aliphatischen acyclischen Rest, d.h. einen Rest mit einer offenen, geraden oder verzweigten Kohlenstoffkette, wie beispielsweise Alkyl, Alkenyl, Alkinyl und entsprechende mehrfach ungesättigte Reste. Er ist vorzugsweise unsubstituiert oder, wie unten beispielsweise bei Carboxy, Carbamoyl, Aminoalkyl, Alkanoylaminoalkyl, Alkoxycarbonylaminoalkyl, Arylalkoxycarbonylaminoalkyl, Arylalkylaminoalkyl, Alkylaminoalkyl, Dialkylaminoalkyl, Alkylthioalkyl, Arylthioalkyl, Carboxyalkyl, Carbamoylalkyl, Alkoxycarbonylalkyl, Alkanoyloxyalkyl, Alkoxycarbonyloxyalkyl, Aroyloxyalkyl oder Aryloxycarbonyloxyalkyl beschrieben, monosubstituiert.

Ein gegebenenfalls substituierter alicyclischer Rest und der über eine offene Kohlenstoffkette gebundene hende gegebenenfalls substituierte alicyclischaliphatische Rest ist ein vorzugsweise mono-bis pentacyclischer isocyclischer, nicht aromatischer Rest mit Einfach- oder unsymmetrisch verteilten Doppelbindungen, der auch verzweigt sein (d.h. offenkettige aliphatische Seitenketten tragen) kann, und der über ein Ring-C-Atom oder ein Seitenketten-C-Atom verknüpft ist. Er ist vorzugsweise unsubstituiert. Mehrere Ringe als Komponenten eines solchen Restes sind kondensiert, spiroverknüpft oder isoliert. Beispiele für solche Reste sind Cycloalkyl, Cycloalkenyl, Cycloalkylalkyl, Bicycloalkyl, Tricycloalkyl und von mono-, bi- oder oligocyclischen Terpenen abgeleitete Reste, wie Menthyl, Isomenthyl, Bornyl, Caranyl, Epibornyl, Epiisobornyl, Isobornyl, Neomenthyl, Neoisomenthyl, Pinanyl, Thujanyl; sie sind vorzugsweise unsubstituiert (aliphatische Seitenketten sind nach vorliegender Definition keine Substituenten).

Ein gegebenenfalls substituierter aromatischer Rest ist vorzugsweise Aryl wie Phenyl, Biphenylyl oder Naphthyl, das gegebenenfalls wie unten bei Aryl (b) 5.) angegeben mono-, di- oder trisubstituiert ist. Von Aryl abgeleitete Reste, wie Aralkyl, Aryloxy, Arylthio oder Aroyl, vorzugsweise Benzoyl, können wie Aryl substituiert sein.

Ein gegebenenfalls substituierter heteroaromatischer Rest ist vorzugsweise ein aromatischer mono- bzw. bicyclischer Heterocyclen-Rest mit 5 bis 7 bzw. 8 bis 12, bevorzugt bis 10 Ringatomen, wovon 1 bis 2 Ringatome Schwefel- oder Sauerstoffatome und/oder wovon 1 bis 4 Ringatome Stickstoffatome darstellen, wie beispielsweise Thienyl, Benzo[b]thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl. Diese Reste können auch teilweise oder vollständig hydriert sein. Ein heteroaromatischer Rest und der entsprechende heteroaromatisch-aliphatische Rest kann wie unten definiert, substituiert sein.

Unter einem gegebenenfalls substituierten araliphatischen Rest werden insbesondere Aralkylreste wie Arylalkyl, Diarylalkyl, Indanyl oder Fluorenyl verstanden, worin Aryl wie oben definiert ist und in der dort angegebenen Weise substituiert sein kann.

Ein gegebenenfalls substituierter Acylrest ist ein geradkettiger oder verzweigter, gesättigter oder ungesättigter aliphatischer Rest, vorzugsweise ein unsubstituierter gesättigter oder ungesättigter Alkanoyl-Rest, wie Formyl, Acetyl, Propionyl, Butyryl, Isobutyryl, Valeryl, Isovaleryl, Pivaloyl, Lauroyl, Myristoyl, Palmitoyl, Stearoyl, Caprinoyl, Caproyl, Capryloyl, Arachidonoyl, Sorboyl, Angeloyl, Acryloyl, Propiolyl, Methacryloyl, Crotonoyl, Isocrotonoyl, Oleoyl, Elaidoyl oder Ricinoleoyl. Bevorzugt sind Fettsäurereste, wie sie in natürlichen Triglyceriden vorkommen.

$R^4$ und $R^5$ kann mit den diese tragenden Atomen ein mono-, bi- oder tricyclisches heterocyclisches Ringsystem mit 3 bis 15 Ring-C-Atomen bilden, das im Ring vorzugsweise bis zu 2 S-Atome und bis zu 2 N-Atome, insbesondere bis zu 1 S-Atom aufweist.

Als solche Ringsysteme kommen insbesondere jene aus der folgenden Gruppe in Betracht:

Pyrrolidin (O); Thiazolidin (R); Tetrahydroisochinolin (A); Decahydroisochinolin (B); Octahydroindol (C); Indolin (Q); Octa-hydrocyclopenta-[b]pyrrol (D); 2-Azaspiro[4.5]decan (E); 2-Azaspiro[4.4]-nonan (F); Spiro[(bicyclo[2.2.1]heptan)-2,3′-pyrrolidin] (G); Spiro[(bicyclo[2.2.2]octan)-2,3′-pyrrolidin] (H); 2-Azatricyclo[4.3.0.1$^{6,9}$]decan (I); Decahydrocyclohepta-[b]pyrrol (J); Octahydroisoindol (K); Octahydrocyclopenta-[c]pyrrol (L); 2,3,3a,4,5,7a-Hexahydroindol (M); 2-Aza-bicyclo[3.1.0]-hexan (N); 1,2,3,3a,4,6a-Hexahydrocyclopenta[b]pyrrol (P), die alle gegebenenfalls substituiert sein können. Pyrrolidin (O) und Thiazolidin (R) können z.B. durch ($C_6$-$C_{12}$)-Aryl (Phenyl, 2-Hydroxyphenyl, u.a.), ($C_6$-$C_{12}$)-Arylmercapto (wie Phenylmercapto) oder

($C_3$-$C_7$)-Cycloalkyl (wie Cyclohexyl) monosubstituiert sein. Tetrahydroisochinolin (A) kann z.B. im Arylteil bis zu 2 ($C_1$-$C_6$)Alkoxyreste, vorzugsweise Methoxyreste tragen. Entsprechendes gilt für die anderen Ringsysteme. Bevorzugt sind jedoch die unsubstituierten Systeme.

Die in Betracht kommenden heterocyclischen Ringsysteme weisen die folgenden Strukturformeln auf:

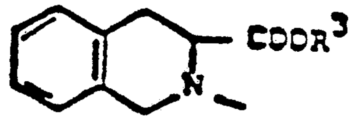

A

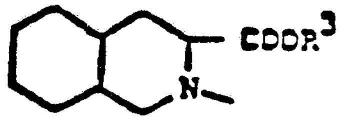

B

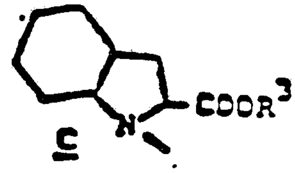

C

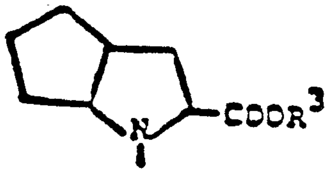

D

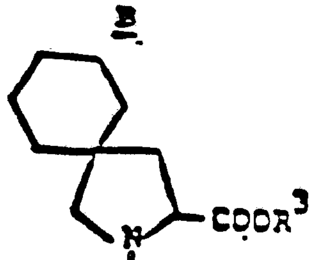

E

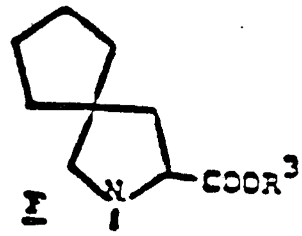

F

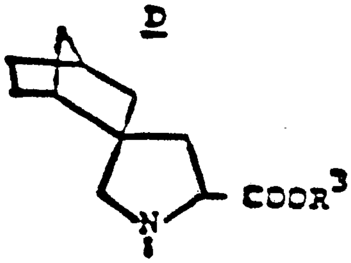

G

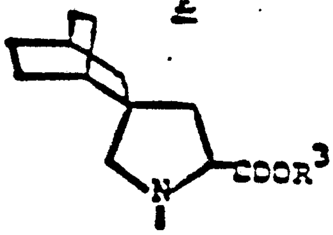

H

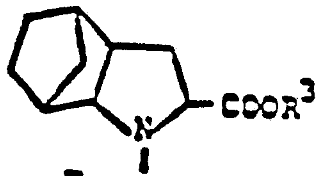

I

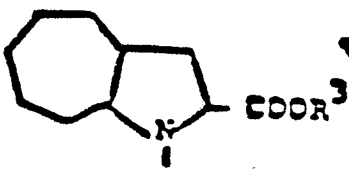

J

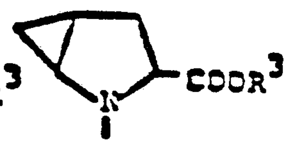

K

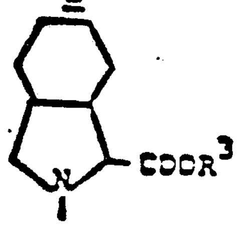

L

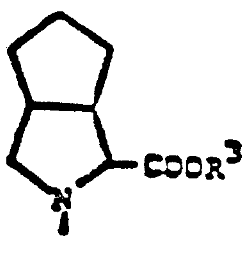

M

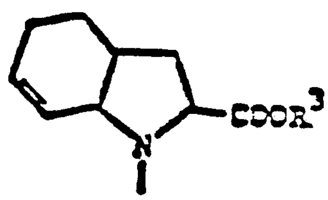

N

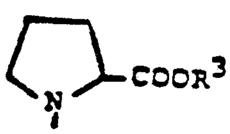

O

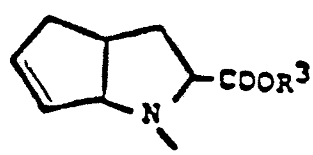

P

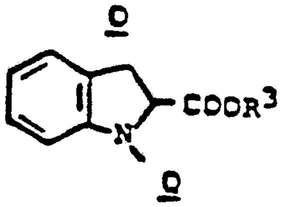

Q

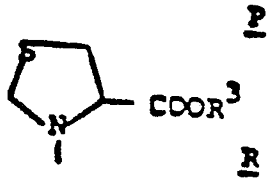

R

Natürlich vorkommende α-Aminosäuren sind beispielsweise Ala, Ser, Thr, Val, Leu, Ile, Asp, Asn, Glu, Gln, Arg, Lys, Hyl, Orn, Cit, Tyr, Phe, Trp und His.

Falls $R^1$ für eine Seitenkette einer geschützten natürlich vorkommenden α-Aminosäure steht, z.B. geschütztes Ser, Thr, Asp, Asn, Glu, Gln, Arg, Lys, Hyl, Cys, Orn, Cit, Tyr, Trp oder His, sind als Schutzgruppen die in der Peptidchemie üblichen Gruppen bevorzugt (vgl. Houben-Weyl, Bd. XV/1 und XV/2). Im Falle, daß $R^1$ die geschützte Lysin-Seitenkette bedeutet, werden die bekannten Amino-Schutzgruppen, insbesondere aber Z, Boc oder $(C_1-C_6)$-Alkanoyl bevorzugt. Als O-Schutzgruppen für Tyrosin kommen bevorzugt $(C_1-C_6)$-Alkyl, insbesondere Methyl oder Ethyl in Frage.

Die Verbindungen der Formel I bzw. II weisen asymmetrische Kohlenstoffatome auf und können daher als Enantiomere und Diastereomere auftreten. Die Erfindung umfaßt sowohl die reinen Enantiomeren als auch die Racemate.

Bei Verbindungen der Formel I bzw. II, die mehrere chirale Atome besitzen, kommen alle möglichen Diastereomere als Racemate oder Enantiomere, oder Gemische verschiedener Diastereomere in Betracht.

Eine bevorzugte Ausführungsform ist dadurch gekennzeichnet, daß Verbindungen der Formel I, vorzugsweise solche der Formel II verwendet werden, in welchen

a) n = 1 oder 2 ist;

b) R

1. Wasserstoff bedeutet;
2. Alkyl mit 1 - 18 C-Atomen bedeutet;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine ganze Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;
5. Aryl mit 6 - 12 C-Atomen bedeutet, das durch $(C_1-C_8)$-Alkyl, $(C_1-C_4)$-Alkoxy, Hydroxy, Halogen, Nitro, Amino, Aminomethyl, $(C_1-C_4)$-Alkylamino, Di-$(C_1-C_4)$-alkylamino, $(C_1-C_4)$-Alkanoylamino, Methylendioxy, Carboxy, Cyano und/oder Sulfamoyl, mono-, di- oder trisubstituiert sein kann;
6. falls n = 2 ist, $(C_6-C_{12})$-Aryl-$(C_1-C_8)$-alkyl oder Di-$(C_6-C_{12})$-aryl-$(C_1-C_8)$-alkyl bedeutet, die im Arylteil jeweils wie unter b)5. beschrieben substituiert sein können; oder
7. Alkoxy mit 1 - 4 C-Atomen;
8. Aryloxy mit 6 - 12 C-Atomen, das wie unter b)5. beschrieben substituiert sein kann;
9. mono- bzw. bicyclisches Heteroaryloxy oder Heteroaryl-$(C_1-C_8)$-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen, die im Heteroaryl wie unter b)5. beschrieben substituiert sein können;
10. Amino-$(C_1-C_8)$-alkyl;
11. $(C_1-C_4)$-Alkanoylamino-$(C_1-C_8)$-alkyl;
12. $(C_7-C_{13})$-Aroylamino-$(C_1-C_8)$-alkyl;
13. $(C_1-C_4)$-Alkoxy-carbonylamino-$(C_1-C_8)$-alkyl;
14. $(C_6-C_{12})$-Aryl-$(C_1-C_4)$-alkoxycarbonylamino-$(C_1-C_8)$-alkyl;
15. $(C_6-C_{12})$-Aryl-$(C_1-C_4)$-alkylamino-$(C_1-C_8)$-alkyl;
16. $(C_1-C_4)$-Alkylamino-$(C_1-C_8)$-alkyl;
17. Di-$(C_1-C_4)$-alkylamino-$(C_1-C_8)$-alkyl;
18. Guanidino-$(C_1-C_8)$-alkyl,
19. Imidazolyl;
20. Indolyl;
21. $(C_1-C_4)$-Alkylthio;
22. falls n = 2 ist, $(C_1-C_4)$-Alkylthio-$(C_1-C_8)$-alkyl;
23. $(C_6-C_{12})$-Arylthio-$(C_1-C_8)$-alkyl; dam im Arylteil wie unter b) 5. beschrieben, substituiert sein kann,
24. $(C_6-C_{12})$-Aryl-$(C_1-C_8)$-alkylthio, das im Arylteil wie unter b) 5. beschrieben substituiert sein kann;
25. falls n = 2 ist, Carboxy-$(C_1-C_8)$-alkyl;
26. Carboxy;
27. Carbamoyl;
28. falls n = 2 ist, Carbamoyl-$(C_1-C_8)$-alkyl;
29. $(C_1-C_4)$-Alkoxy-carbonyl-$(C_1-C_8)$-alkyl;
30. falls n = 2 ist, $(C_6-C_{12})$-Aryloxy-$(C_1-C_8)$-alkyl, das im Arylteil wie unter b) 5. beschrieben substituiert sein kann; oder
31. $(C_6-C_{12})$-Aryl-$(C_1-C_8)$-alkoxy, das in Arylteil wie unter b) 5. beschrieben substituiert sein kann, bedeutet;

c) $R^1$

1. Wasserstoff bedeutet;
2. Alkyl mit 1 - 18 C-Atomen bedeutet;
3. einen aliphatischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine ganze Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;
5. Aryl mit 6 - 12 C-Atomen,
das wie unter I. b) 5. beschrieben substituiert sein kann;
6. $(C_6-C_{12})$-Aryl-$(C_1-C_8)$-alkyl oder $(C_7-C_{13})$-Aroyl-$(C_1-C_8)$-alkyl,
die beide in Arylteil wie unter b) 5. beschrieben substituiert sein können;
7. mono- bzw. bicyclisches, gegebenenfalls teilhydriertes Heteroaryl oder Heteroaryl-$(C_1-C_8)$-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen,
die im Heteroaryl wie bei Aryl unter b) 5. beschrieben substituiert sein können; oder
8. falls von c) 1. - 8. nicht umfaßt,
die gegebenenfalls geschützte Seitenkette einer natürlich vorkommenden $\alpha$-Aminosäure der Formel $R^1$-CH($NH_2$)-COOH bedeutet;

d) $R^2$ und $R^3$ gleich oder verschieden sind und
1. Wasserstoff bedeutet;
2. Alkyl mit 1 - 18 C-Atomen;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine ganze Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;
5. Di-$(C_1-C_4)$-alkylamino-$(C_1-C_8)$-alkyl;
6. $(C_1-C_5)$-Alkanoyloxy-$(C_1-C_8)$-alkyl;
7. $(C_1-C_6)$-Alkoxy-carbonyloxy-$(C_1-C_8)$-alkyl;
8. $(C_7-C_{13})$-Aroyloxy-$(C_1-C_8)$-alkyl;
9. $(C_6-C_{12})$-Aryloxycarbonyloxy$(C_1-C_8)$-alkyl;
10. Aryl mit 6 - 12 C-Atomen;
11. $(C_7-C_{20})$-Aralkyl;
12. Phthalidyl;
13. einen Rest der Formel

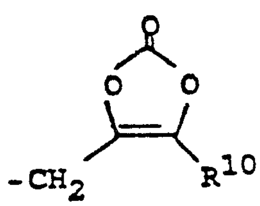

bedeutet,
worin $R^{10}$ Wasserstoff, $(C_1-C_6)$-Alkyl oder Aryl mit 6-12 C-Atomen ist,
14. einen Rest der Formel

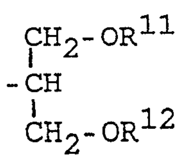

bedeutet, worin $R^{11}$ und $R^{12}$ gleich oder verschieden unabhängig voneinander Wasserstoff, einen gegebenenfalls substituierten Alkylrest mit 1-23 C-Atomen oder einen gegebenenfalls substituierten Acylrest mit 1-23 C-Atomen bedeuten,
wobei die unter d) 8., 9., 10. und 11. genannten Reste wie unter b) 5. beschrieben im Arylteil substituiert

sein können; und

e) $R^4$ und $R^5$ zusammen mit den sie tragenden Atomen ein mono-,bi- oder tricyclisches heterocyclisches Ringsystem mit 3 bis 15 Ring-C-Atomen bilden.

Eine besonders bevorzugte Ausführungsform ist dadurch gekennzeichnet, daß Verbindungen der Formel I, vorzugsweise solche der Formel II verwendet werden, in welchen

n= 1 oder 2 ist

R Wasserstoff,

Alkyl mit 1 - 8 C-Atomen,

Alkenyl mit 2 - 6 C-Atomen,

Cycloalkyl mit 3 - 9 C-Atomen,

Aryl mit 6 - 12 C-Atomen,

das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl, mono-, di- oder trisubstituiert sein kann,

Alkoxy mit 1 - 4 C-Atomen,

Aryloxy mit 6 - 12 C-Atomen,

das wie oben bei Aryl beschrieben substituiert sein kann,

mono-bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen,

das wie oben bei Aryl beschrieben substituiert sein kann,

Amino-($C_1$-$C_4$)-alkyl,

($C_1$-$C_4$)-Alkanoylamino-($C_1$-$C_4$)alkyl,

($C_7$-$C_{13}$)-Aroylamino-($C_1$-$C_4$)-alkyl,

($C_1$-$C_4$)-Alkoxy-carbonylamino-($C_1$-$C_4$)-alkyl,

($C_6$-$C_{12}$)-Aryl-($C_1$-$C_4$)-alkoxycarbonylamino-($C_1$-$C_4$)-alkyl,

($C_6$-$C_{12}$)-Aryl-($C_1$-$C_4$)-alkylamino-($C_1$-$C_4$)-alkyl,

($C_1$-$C_4$)-Alkylamino-($C_1$-$C_4$)-alkyl,

Di-($C_1$-$C_4$)-alkylamino-($C_1$-$C_4$)-alkyl,

Guanidino-($C_1$-$C_4$)-alkyl,

Imidazolyl, Indolyl,

($C_1$-$C_4$)-Alkylthio,

($C_1$-$C_4$)-Alkylthio-($C_1$-$C_4$)-alkyl,

($C_6$-$C_{12}$)-Arylthio-($C_1$-$C_4$)-alkyl,

das im Arylteil wie oben bei Aryl beschrieben, substituiert sein kann,

($C_6$-$C_{12}$)-Aryl-($C_1$-$C_4$)-alkylthio,

das im Arylteil wie oben bei Aryl beschrieben substituiert sein kann,

Carboxy-($C_1$-$C_4$)-alkyl,

Carboxy, Carbamoyl,

Carbamoyl-($C_1$-$C_4$)-alkyl,

($C_1$-$C_4$)-Alkoxy-carbonyl-($C_1$-$C_4$)-alkyl,

($C_6$-$C_{12}$)-Aryloxy-($C_1$-$C_4$)-alkyl,

das im Arylteil wie oben bei Aryl beschrieben substituiert sein kann oder

($C_6$-$C_{12}$)-Aryl-($C_1$-$C_4$)-alkoxy,

das im Arylteil wie oben bei Aryl beschrieben substituiert sein kann,

$R^1$ Wasserstoff,

Alkyl mit 1 - 6 C-Atomen,

Alkenyl mit 2 - 6 C-Atomen,

Alkinyl mit 2 - 6 C-Atomen,

Cycloalkyl mit 3 - 9 C-Atomen,

Cycloalkenyl mit 5 - 9 C-Atomen,

($C_3$-$C_9$)-Cycloalkyl-($C_1$-$C_4$)-alkyl,

($C_5$-$C_9$)-Cycloalkenyl-($C_1$-$C_4$)-alkyl,

gegebenenfalls teilhydriertes Aryl mit 6 - 12 C-Atomen, das wie oben bei R beschrieben substituiert sein kann,

($C_6$-$C_{12}$)-Aryl-($C_1$-$C_4$)-alkyl oder ($C_7$-$C_{13}$)-Aroyl-($C_1$ oder $C_2$)alkyl

die beide wie das vorstehende Aryl substituiert sein können

mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen, das wie das vorstehende Aryl substituiert sein kann oder

die gegebenenfalls geschützte Seitenkette einer natürlich vorkommenden α-Aminosäure $R^1$-$CH(NH_2)$-$COOH$ bedeuten,

$R^2$ und $R^3$ gleich oder verschieden sind und

Wasserstoff,

Alkyl mit 1 - 12 C-Atomen,

Alkenyl mit 2 - 12 C-Atomen,

Di-($C_1$-$C_4$)-alkylamino-($C_1$-$C_8$)-alkyl,

($C_1$-$C_5$)-Alkanoyloxy-($C_1$-$C_8$)-alkyl,
($C_1$-$C_6$)-Alkoxy-carbonyloxy-($C_1$-$C_8$)-alkyl,
($C_7$-$C_{13}$)-Aroyloxy-($C_1$-$C_8$)-alkyl,
($C_6$-$C_{12}$)-Aryloxycarbonyloxy($C_1$-$C_8$)-alkyl,
Aryl mit 6 - 12 C-Atomen,
($C_6$-$C_{12}$)-Aryl-($C_1$-$C_8$)-alkyl,
($C_3$-$C_9$)-Cycloalkyl oder
($C_3$-$C_9$)-Cycloalkyl-($C_1$-$C_8$)-alkyl
bedeuten und
$R^4$ und $R^5$ die oben angegebene Bedeutung haben.

Eine ganz besonders bevorzugte Ausführungsform ist dadurch gekennzeichnet, daß Verbindungen der Formel I, vorzugsweise solche der Formel II verwendet werden, in welchen
n = 1 oder 2 ist,
R ($C_1$-$C_6$)-Alkyl, ($C_2$-$C_6$)-Alkenyl, ($C_3$-$C_9$)-Cycloalkyl, Amino-($C_1$-$C_4$)-alkyl, ($C_2$-$C_5$)-Acylamino-($C_1$-$C_4$)-alkyl, ($C_7$-$C_{13}$)-Aroylamino-($C_1$-$C_4$)-alkyl, ($C_1$-$C_4$)-Alkoxy-carbonylamino-($C_1$-$C_4$)-alkyl, ($C_6$-$C_{12}$)-Aryl-($C_1$-$C_4$)-alkoxycarbonylamino-($C_1$-$C_4$)-alkyl, ($C_6$-$C_{12}$)-Aryl, das durch ($C_1$-$C_4$)-Alkyl, ($C_1$-$C_4$)-Alkoxy, Hydroxy, Halogen, Nitro, Amino, ($C_1$-$C_4$)-Alkylamino, Di-($C_1$-$C_4$)-alkylamino und/oder Methylendioxy mono-, di- oder trisubstituiert sein kann, oder 3-Indolyl, insbesondere Methyl, Ethyl, Cyclohexyl, tert. Butoxycarbonylamino-($C_1$-$C_4$)-alkyl, Benzoyloxycarbonylamino-($C_1$-$C_4$)-alkyl oder Phenyl, das durch Phenyl, ($C_1$-$C_2$)-Alkyl, ($C_1$ oder $C_2$)-Alkoxy, Hydroxy, Fluor, Chlor, Brom, Amino, ($C_1$-$C_4$)-Alkylamino, Di-($C_1$-$C_4$)alkylamino, Nitro und/oder Methylendioxy mono- oder disubstituiert oder im Falle von Methoxy, trisubstituiert sein kann, bedeutet,
$R^1$ Wasserstoff oder ($C_1$-$C_6$)-Alkyl, das gegebenenfalls durch Amino, ($C_1$-$C_6$)-Acylamino oder Benzoylamino substituiert sein kann, ($C_2$-$C_6$)-Alkenyl, ($C_3$-$C_9$)-Cycloalkyl, ($C_5$-$C_9$)-Cycloalkenyl, ($C_3$-$C_7$)-Cycloalkyl-($C_1$-$C_4$)-alkyl, ($C_6$-$C_{12}$)-Aryl oder teilhydriertes Aryl, das jeweils durch ($C_1$-$C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)-alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,
$R^2$ und $R^3$ gleich oder verschiedene Reste Waserstoff, ($C_1$-$C_{12}$)-Alkyl, ($C_2$-$C_{12}$)-Alkenyl oder ($C_6$-$C_{12}$)-Aryl-($C_1$-$C_8$)-alkyl, bedeuten und
$R^4$ und $R^5$ die oben angegebene Bedeutung haben.

Besonders vorteilhaft können die folgenden Verbindungen erfindungsgemäß verwendet werden:
2-[N-(1-S-Carbethoxy-3-phenyl-propyl)-S-alanyl]-S-1,2,3,4-tetrahydroisochinolin-3-carbonsäure
2-[N-(1-S-Carbethoxy-3-cyclohexyl-propyl)-S-alanyl]-S-1,2,3,4-tetrahydroisochinolin-3-carbonsäure
2-[N-(1-S-Carbethoxy-3-phenyl-propyl)-S-lysyl]-S-1,2,3,4-tetrahydroisochinolin-3-carbonsäure
2-[N-(1-S-Carbethoxy-3-phenyl-propyl)-O-ethyl-S-tyrosyl]-S-1,2,3,4-tetrahydroisochinolin-3-carbonsäure
2-[N-(1-S-Carbethoxy-3-phenyl-propyl)-S-alanyl]-(3S)-decahydroisochinolin-3-carbonsäure
1-[N-(1-S-Carbethoxy-3-phenyl-propyl)-S-alanyl]-(2S,3aS,7aS)-octahydroindol-2-carbonsäure
1-[N-(1-S-Carbethoxy-3-cyclohexyl-propyl)-S-alanyl]-(2S,3aS,7aS)-octahydroindol-2-carbonsäure
1-[N-(1-S-Carbethoxy-3-phenyl-propyl)-S-lysyl]-(2S,3aS,7aS)-octahydroindol-2-carbonsäure
1-[N-(1-S-Carbethoxy-3-cyclohexyl-propyl)-S-lysyl]-(2S,3aS,7aS)-octahydroindol-2-carbonsäure
1-[N-(1-S-Carbethoxy-3-cyclohexyl-propyl)-S-lysyl]-(2S,3aS,7aS)-octahydroindol-2-carbonsäure
1-[N-(1-S-Carbethoxy-3-phenyl-propyl)-O-methyl-S-tyrosyl]-(2S,3aS,7aS)-octahydroindol-2-carbonsäure
1-[N-(1-S-Carbethoxy-3-phenyl-propyl)-O-ethyl-S-tyrosyl]-(2S,3aS,7aS)-octahydroindol-2-carbonsäure
1-[N-(1-S-Carbethoxy-3-(3,4-dimethylphenyl-propyl)-S-alanyl]-(2S,3aS,7aS)-octahydroindol-2-carbonsäure
1-[N-[1-S-Carbethoxy-3-(4-fluorphenyl)-propyl]-S-alanyl]-(2S,3aS,7aS)-octahydroindol-2-carbonsäure
1-[N-[1-S-Carbethoxy-3-(4-methoxyphenyl)-propyl]-S-alanyl]-(2S,3aS,7aS)-octahydroindol-2-carbonsäure
1-[N-[1-S-Carbethoxy-3-(3,4-dimethoxyphenyl)-propyl]-S-alanyl]-(2S,3aS,7aS)-octahydroindol-2-carbonsäure
1-[N-(1-S-Carbethoxy-3-cyclopentylpropyl)-S-alanyl]-(2S,3aS,7aS)-octahydroindol-2-carbonsäure
1-[N-(1-S-Carbethoxy-3-phenyl-propyl)-S-alanyl]-(2S,3aR,7aS)-octahydroindol-2-carbonsäure
1-[N-(1-S-Carbethoxy-3-cyclohexyl-propyl)-S-alanyl]-(2S,3aR,7aS)-octahydroindol-2-carbonsäure
1-[N-(1-S-Carbethoxy-3-phenyl-propyl)-S-lysyl]-(2S,3aR,7aS)-octahydroindol-2-carbonsäure
1-[N-(1-S-Carbethoxy-3-cyclohexyl-propyl)-S-lysyl]-(2S,3aR,7aS)-octahydroindol-2-carbonsäure
1-[N-(1-S-Carbethoxy-3-phenyl-propyl)-O-ethyl-S-tyrosyl]-(2S,3aS,7aR)-octahydroindol-2-carbonsäure
1-[N-(1-S-Carbethoxy-3-phenyl-propyl)-S-alanyl]-(2S,3aR,7aR)-octahydroindol-2-carbonsäure
1-[N-(1-S-Carbethoxy-3-phenyl-propyl)-S-lysyl]-(2S,3aR,7aS)-octahydroindol-2-carbonsäure
1-[N-(1-S-Carbethoxy-3-cyclohexyl-propyl)-S-alanyl]-(2S,3aR,7aR)-octahydroindol-2-carbonsäure
1-[N-(1-S-Carbethoxy-3-cyclohexyl-propyl)-O-ethyl-S-tyrosyl]-(2S,3aR,7aR)-octahydroindol-2-carbonsäure
1-[N-(1-S-Carbethoxy-3-phenyl-propyl)-S-alanyl]-(2S,3aS,7aR)-octahydroindol-2-carbonsäure
1-[N-(1-S-Carbethoxy-3-phenyl-propyl)-O-ethyl-S-tyrosyl]-(2S,3aS,7aS)-octahydroindol-2-carbonsäure
1-[N-(1-S-Carbethoxy-3,4-dimethylphenyl-propyl)-S-alanyl]-(2S,3aS,7aS)-octahydroindol-2-carbonsäure

1-[N-[1-S-Carbethoxy-3-(4-fluorphenyl)-propyl]-S-alanyl]-(2S,3aS,7aS)-octahydroindol-2-carbonsäure
1-[N-[1-S-Carbethoxy-3-(4-methoxyphenyl)-propyl]-S-alanyl]-(2S,3aS,7aS)-octahydroindol-2-carbonsäure
1-[N-[1-S-Carbethoxy-3-(3,4-dimethoxyphenyl)-propyl]-S-alanyl]-(2S,3aS,7aS)-octahydroindol-2-carbonsäure
1-[N-(1-S-Carbethoxy-3-cyclopentylpropyl)-S-alanyl]-(2S,3aS,7aS)-octahydroindol-2-carbonsäure
2-[N-(1-S-Carbethoxy-3-phenyl-propyl)-S-alanyl]-cis-endo-2-azabicyclo[3.3.0]octan-3-S-carbonsäure
2-[N-(1-S-Carbethoxy-3-phenyl-propyl)-S-lysyl]-cis-endo-2-azabicyclo[3.3.0]octan-3-S-carbonsäure
2-[N-(1-S-Carbethoxy-3-cyclohexyl-propyl)-S-alanyl]-cis-endo-2-azabicyclo[3.3.0]octan-3-S-carbonsäure
2-[N-(1-S-Carboxy-3-cyclohexyl-propyl)-S-alanyl]-cis-endo-2-azabicyclo[3.3.0]octan-3-S-carbonsäure
2-[N-(1-S-Carbethoxy-butyl)-S-alanyl]-cis-endo-2-azabicyclo[3.3.0]octan-3-S-carbonsäure
2-[N-(1-S-Carbethoxy-3-(3,4-dimethoxyphenylpropyl)-S-alanyl]-cis-endo-2-azabicyclo[3.3.0]octan-3-S-carbonsäure
2-[N-(1-S-Carbethoxy-3-cyclopentyl-propyl)-S-alanyl]-cis-endo-azabicyclo-[3.3.0]octan-3-S-carbonsäure
2-[N-(1-S-Carbethoxy-3-phenyl-propyl)-O-methyl-S-tyrosyl]-cis-endo-2-azabicyclo[3.3.0]octan-3-S-carbonsäure
2-[N-(1-S-Carbethoxy-3-phenyl-propyl)-O-ethyl-S-tyrosyl]-cis-endo-2-azabicyclo[3.3.0]octan-3-S-carbonsäure
2-[N-(1-S-Carbethoxy-3-(4-fluorphenyl-propyl)-S-alanyl]-cis-endo-2-azabicyclo[3.3.0]octan-3-S-carbonsäure
2-[N-(1-S-Carbethoxy-3-(4-methoxyphenyl-propyl)-S-alanyl]-cis-endo-2-azabicyclo[3.3.0]octan-3-S-carbonsäure
1-[N-(1-S-Carbethoxy-3-phenyl-propyl)-S-lysyl]-(2S,3aR,6aS)-octahydrocyclopenta[b]pyrrol-2-carbonsäure
1-[N-(1-S-Carbethoxy-3-cyclohexylpropyl)-S-lysyl]-(2S,3aR,6aS)-octahydrocyclopenta[b]pyrrol-2-carbonsäure
1-[N-(1-S-Carbethoxy-3-phenyl-propyl)-O-ethyl-S-tyrosyl]-(2S,3aR,6aS)-octahydrocyclopenta[b]pyrrol-2-carbonsäure
1-[N-(1-S-Carbethoxy-3-phenyl-propyl)-S-alanyl]-2-(2S,3aR,6aS)-octahydrocyclopenta[b]pyrrol-2-carbonsäure
2-[N-(1-S-Carbethoxy-3-phenyl-propyl)-S-alanyl]-2-azaspiro[4.5]decan-3-S-carbonsäure
2-[N-(1-S-Carbethoxy-3-phenyl-propyl)-O-ethyl-2-tyrosyl]-2-azaspiro-[4.5]decan-3-S-carbonsäure
2-[N-(1-S-Carbethoxy-3-phenyl-propyl)-S-lysyl]-2-azaspiro[4.5]decan-3-S-carbonsäure
2-[N-(1-S-Carbethoxy-3-cyclohexylpropyl)-S-alanyl]-2-azaspiro[4.5]decan-3-S-carbonsäure
2-[N-(1-S-Carbethoxy-3-cyclohexylpropyl)-S-lysyl]-2-azaspiro[4.5]decan-3-S-carbonsäure
2-[N-(1-S-Carbethoxy-3-phenyl-propyl)-S-alanyl]-2-azaspiro[4.4]nonan-3-S-carbonsäure
2-[N-(1-S-Carbethoxy-3-phenyl-propyl)-O-ethyl-S-tyrosyl]-2-azaspiro[4.4]nonan-3-S-carbonsäure
2-[N-(1-S-Carbethoxy-3-phenyl-propyl)-S-lysyl]-2-azaspiro[4.4]nonan-3-S-carbonsäure
2-[N-(1-S-Carbethoxy-3-cyclohexyl-propyl)-S-alanyl]-2-azaspiro[4.4]nonan-3-S-carbonsäure
2-[N-(1-S-Carbethoxy-3-cyclopentyl-propyl)-S-alanyl]-2-azaspiro[4.4]nonan-3-S-carbonsäure
2-[N-(1-S-Carbethoxy-3-cyclopentyl-propyl)-S-lysyl]-2-azaspiro[4.4]nonan-3-S-carbonsäure
1′-[N-(1-S-Carbethoxy-3-phenyl-propyl)-S-alanyl]-spiro[bicyclo[2.2.1]heptan-2,3′-pyrrolidin]-5′-S-carbonsäure
1′-[N-(1-S-Carbethoxy-3-phenyl-propyl)-O-ethyl-S-tyrosyl]-spiro[bicyclo[2.2.1]heptan-2,3′-pyrrolidin]-5′-S-carbonsäure
1′-[N-(1-S-Carbethoxy-3-phenyl-propyl)-S-lysyl]-spiro[bicyclo[2.2.1]heptan-2,3′-pyrrolidin]-5′-S-carbonsäure
1′-[N-(1-S-Carbethoxy-3-cyclohexyl-propyl)-S-alanyl]-spiro[bicyclo[2.2.1]heptan-2,3′-pyrrolidin]5′-S-carbonsäure
1′-[N-(1-S-Carbethoxy-3-cyclohexyl-propyl)-S-lysyl]-spiro[bicyclo[2.2.1]heptan-2,3′-pyrrolidin]-5′-S-carbonsäure
1′-[N-(1-S-Carbethoxy-3-phenyl-propyl)-S-alanyl]-spiro[bicyclo[2.2.2]heptan-2,3′-pyrrolidin]-5′-S-carbonsäure
1′-[N-(1-S-Carbethoxy-3-phenyl-propyl)-O-ethyl-tyrosyl]-spiro-[bicyclo[2.2.2]octan-2,3′-pyrrolidin]-5′-S-carbonsäure
1′-[N-(1-S-Carbethoxy-3-phenyl-propyl)-S-lysyl]-spiro[bicyclo[2.2.2]octan-2,3′-pyrrolidin]-5′-S-carbonsäure
1′-[N-(1-S-Carbethoxy-3-cyclohexyl-propyl)-S-alanyl]-spiro[bicyclo[2.2.2]octan-2,3′-pyrrolidin]-5′-S-carbonsäure
2-[N-(1-S-Carbethoxy-3-phenyl-propyl)-S-alanyl]-2-azatricyclo[4.3.0.$1^{6,9}$]decan-3-S-carbonsäure
2-[N-(1-S-Carbethoxy-3-phenyl-propyl)-O-ethyl-S-tyrosyl]-2-azatricyclo[4.3.0.$1^{6,9}$]decan-3-S-carbonsäure
2-[N-(1-S-Carbethoxy-3-phenyl-propyl)-S-lysyl]-2-azatricyclo[4.3.0.$1^{6,9}$]decan-3-S-carbonsäure
2-[N-(1-S-Carbethoxy-3-phenyl-propyl)-S-alanyl]-2-azatricyclo[4.3.0.$1^{6,9}$]decan-3-S-carbonsäure
2-[N-(1-S-Carbethoxy-3-cyclohexyl-propyl)-S-lysyl]-2-azatricyclo[4.3.0.$1^{6,9}$]decan-3-S-carbonsäure
1-[N-(1-S-Carbethoxy-3-phenyl-propyl)-S-alanyl]-decahydrocyclohepta[b]pyrrol-2-S-carbonsäure
1-[N-(1-S-Carbethoxy-3-phenyl-propyl)-O-ethyl-S-tyrosyl]-decahydrocyclohepta[b]pyrrol-2-S-carbonsäure
1-[N-(1-S-Carbethoxy-3-phenyl-propyl)-S-lysyl]-decahydrocyclohepta[b]pyrrol-2-S-carbonsäure
1-[N-(1-S-Carbethoxy-3-cyclohexyl-propyl)-S-alanyl]-decahydrocyclohepta[b]pyrrol-2-S-carbonsäure
1-[N-(1-S-Carbethoxy-3-cyclohexyl-propyl)-S-lysyl]-decahydrocyclohepta[b]pyrrol-2-S-carbonsäure
2-[N-(1-S-Carbethoxy-3-phenyl-propyl)-S-alanyl]-trans-octahydroisoindol-1-S-carbonsäure

2-[N-(1-S-Carbethoxy-3-phenyl-propyl)-S-alanyl]-cis-octahydroisoindol-1-S-carbonsäure
2-[N-(1-S-Carbethoxy-3-cyclohexyl-propyl)-S-alanyl]-trans-octahydroisoindol-1-S-carbonsäure
2-[N-(1-S-Carbethoxy-3-cyclohexyl-propyl)-S-alanyl]-cis-octahydroisoindol-1-S-carbonsäure
2-[N-(1-S-Carbethoxy-3-phenyl-propyl)-S-alanyl]-cis-octahydrocyclopenta[c]pyrrol-1-S-carbonsäure
2-[N-(1-S-Carbethoxy-3-cyclohexyl-propyl)-S-alanyl]-cis-octahydrocyclopenta[c]pyrrol-1-S-carbonsäure-benzylester
2-[N-(1-S-Carbethoxy-3-cyclohexyl-propyl)-S-lysyl]-cis-octahydrocyclopenta[c]pyrrol-1-S-carbonsäure
1-[N-(1-S-Carbethoxy-3-phenyl-propyl)-S-alanyl]-2,3,3a,4,5,7a-hexahydroindol-cis-endo-2-S-carbonsäure
1-[N-(1-S-Carbethoxy-3-phenyl-propyl)-S-lysyl]-2,3,3a,4,5,7a-hexahydroindol-cis-endo-2-S-carbonsäure
2-[N-(1-S-Carbethoxy-3-cyclohexyl-propyl)-S-lysyl]-2-azabicyclo[3.1.0]hexan-3-S-carbonsäure
2-[N-(1-S-Carboxy-3-phenyl-propyl)-S-lysyl]-2-azabicyclo[3.1.0]hexan-cis-endo-3-S-carbonsäure
2-[N-(1-S-Carbethoxy-3-cyclopentylpropyl)-S-alanyl]-2-azabicyclo[3.1.0]hexan-3-carbonsäure
2-[N-(1-S-Carbethoxy-3-phenyl-propyl)-S-alanyl]-cis-endo-2-azabicyclo[3.1.0]hexan-3-S-carbonsäure
2-[N-(1-S-Carbethoxy-3-cyclohexyl-propyl)-S-alanyl]-cis-endo-2-azabicyclo[3.1.0]hexan-3-S-carbonsäure
1′-[N-(1-S-Carbethoxy-3-phenylpropyl)-S-alanyl]-(3′S,5′S)-spiro-bicyclo[2.2.2]-octan-2,3′-pyrrolidin-5′-carbonsäure
2-[N-(1-S-Carbethoxy-3-phenylpropyl)-S-alanyl]-cis-endo-2-azabicyclo[3.3.0]octan-3-S-carbonsäureoctylester
2-[N-(1-S-Carbethoxy-3-phenylpropyl)-S-alanyl]-cis-endo-2-azabicyclo[3.3.0]octan-3-S-carbonsäuredecylester
2-[N-(1-S-Carbethoxy-3-phenylpropyl)-S-alanyl]-cis-endo-2-azabicyclo[3.3.0]octan-3-S-carbonsäure-(5-nonyl)ester
2-[N-(1-S-Octyloxy-3-phenylpropyl)-S-alanyl]-cis-endo-2-azabicyclo[3.3.0]octan-3-S-carbonsäureoctylester
2-[N-(1-S-Menthyloxy-3-phenylpropyl)-S-alanyl]-cis-endo-2-azabicyclo[3.3.0]octan-3-S-carbonsäure-benzhydrylester

Diese Verbindungen lassen sich z.B. nach dem in der deutschen Patentanmeldung P 33 33 455.2 beschriebenen Verfahren herstellen, indem die in der Anmeldung beschriebenen tert. Butyl- oder Benzylderivate in bekannter Weise durch saure oder alkalische Hydrolyse oder durch edelmetallkatalysierte Hydrogenolyse in die Monocarbonsäurederivate überführt werden. Die $N^6$-Benzyloxycarbonylschutzgruppe der Lysinderivate wird durch Edelmetallkatalysierte Hydrogenolyse entfernt.

Die Herstellung der Verbindungen der allgemeinen Formel I oder II kann beispielsweise auch unter Anwendung dem Fachmann vertrauter Veresterungsmethoden erfolgen (siehe z.B. Buehler, Pearson, Survey of Organic Syntheses, Vol. 1, New York 1970, S. 802-825; Houben-Weyl, Methoden der Organischen Chemie, Band E5, 1985, S. 656-773), beispielsweise durch

a) Umsetzung einer Mono- oder Dicarbonsäure der allgemeinen Formel I oder II, worin mindestens einer der Reste $R^2$ und $R^3$ Wasserstoff bedeutet, mit einem entsprechenden Alkohol unter saurer Katalyse (Mineralsäure oder saurer Ionenaustauscher).

b) Alkylierung einer Mono- oder Dicarbonsäure der allgemeinen Formel I oder II, worin mindestens einer der Reste $R^2$ und $R^3$ Wasserstoff bedeutet, mit einer Verbindung $R^2Z$ oder $R^3Z$, worin Z eine nukleophil zu verdrängende Abgangsgruppe (wie Halogen, Tosylat) bedeutet, in einem polaren protischen oder dipolaren aprotischen Lösungsmittel in Anwesenheit einer Base wie Alkalimetallhydroxide oder -alkoholat.

c) Umsetzung einer Mono- oder Dicarbonsäure der allgemeinen Formel I oder II, worin mindestens einer der Reste $R^2$ und $R^3$ Wasserstoff bedeutet, mit einem Diazoalkan in einem inerten organischen Lösungsmittel wie $CH_2Cl_2$.

Die oben aufgeführten Verbindungen lassen sich leicht mit physiologisch verträglichen Säuren oder Basen (im Falle von Mono- oder Dicarbonsäuren) in die entsprechenden Salze (z.B. Hydrochloride, Maleinate, Fumarate, etc.) überführen und als Salze die erfindungsgemäße Verwendung finden.

Die Verbindungen der Formel I und II sind Hemmstoffe des Angiotensin-Converting-Enzymes (ACE) beziehungsweise Zwischenprodukte bei der Herstellung von solchen Hemmstoffen und können auch zur Bekämpfung des Bluthochdrucks verschiedener Genese eingesetzt werden. Die Verbindungen der Formel I und II und Verfahren zu deren Herstellung sind teilweise bekannt, beispielsweise aus US-Patent 4 129 571, US-Patent 4 374 829, EP-A-79522, EP-A-79022, EP-A-49658, EP-A-51301, US-Patent 4 454 292, US-Patent 4 374 847, EP-A-72352, US-Patent 4 350 704, EP-A-50800, EP-A-46953, US-Patent 4 344 949, EP-A-84164, US-Patent 4 470 972, EP-A-65301 und EP-A-52991. Neue Verbindungen der Formel I und II werden in analoger Weise hergestellt.

Vorteilhaft sind auch oral wirksame ACE-Inhibitoren (Wirkstoffe z.T. schon oben erwähnt), wie z.B. Ramipril, Enalapril(f), Captopril(a), Lisinopril(g), Cilazapril(o), RHC 3659, CGS 13945, CGS 13928C(1), CGS 14824A(h), CI-906(j), Zofenopril(e), Fosenopril(p), Alacepril CI-925(k), Pentopril(q), CV 3317(m), Indolapril(h), YS 980(b), Fentiapril(c), Pivopril(d), Perindopril(i), MDL 27088(r), MDL 27788(s), RS-5142(t) und andere. Oral wirksame ACE-Inhibitoren sind beispielsweise in Brunner et al., J. Cardiovasc. Pharmacol. 7 (Suppl. I) [1985] S2-S11 beschrieben.

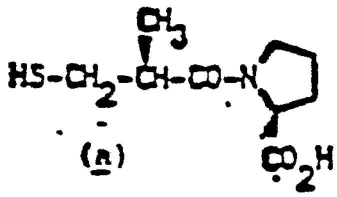

(a)

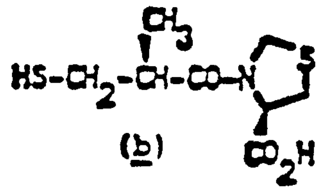

(b)

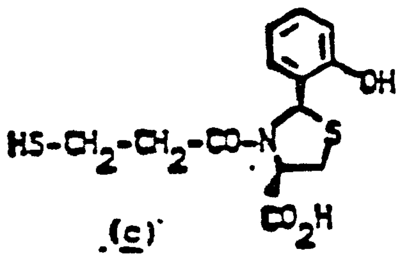

(c)

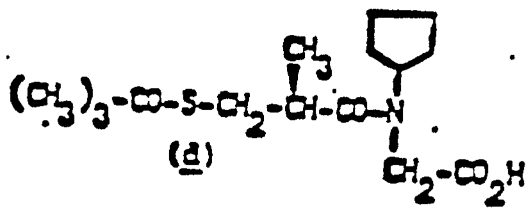

(d)

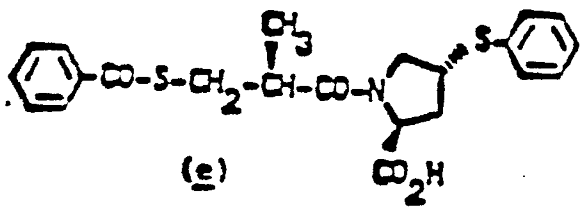

(e)

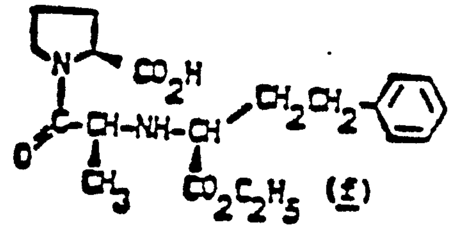

(f)

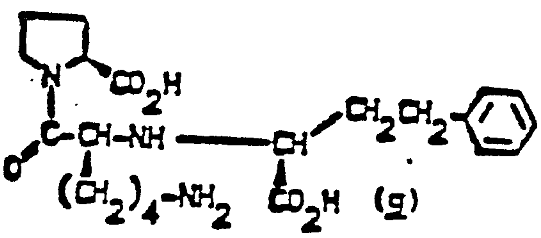

(g)

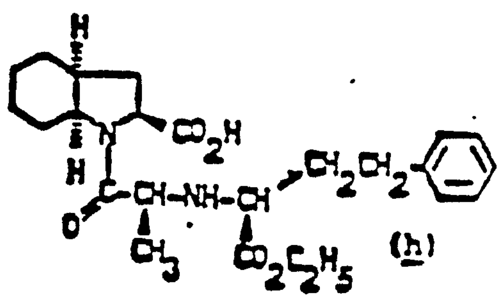

(h)

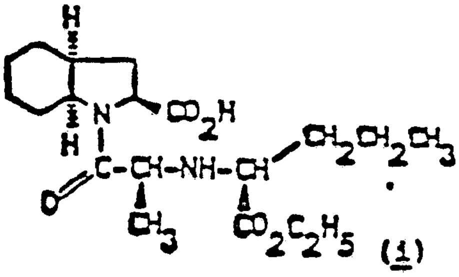

(i)

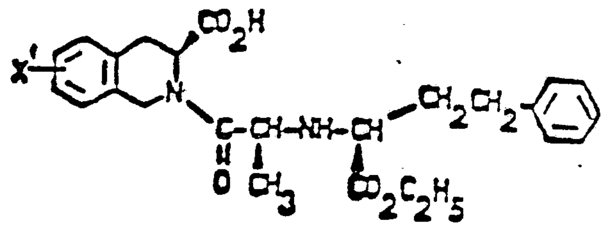

X′ = H (j)

X′ = 3,4 $OCH_3$ (k)

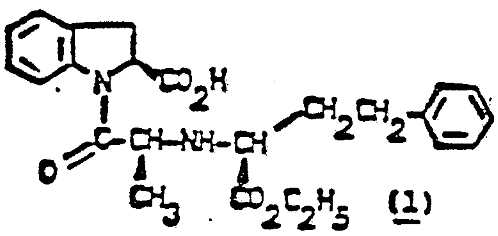

(l)

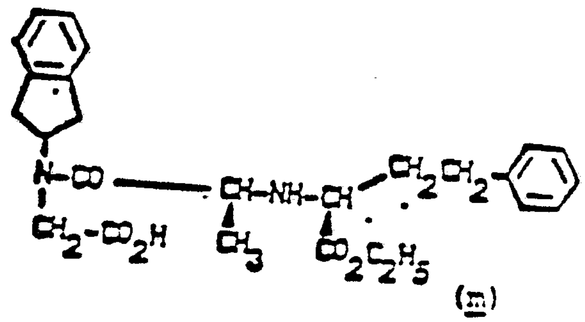

(m)

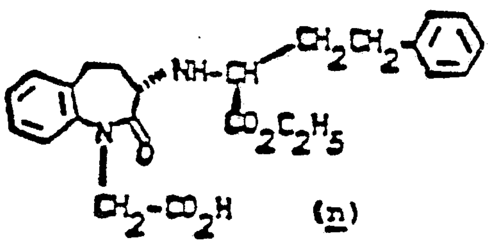

(n)

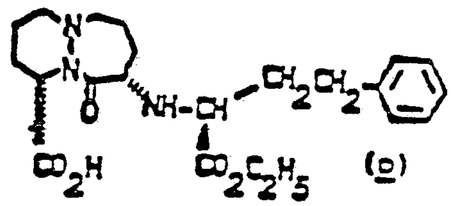

(o)

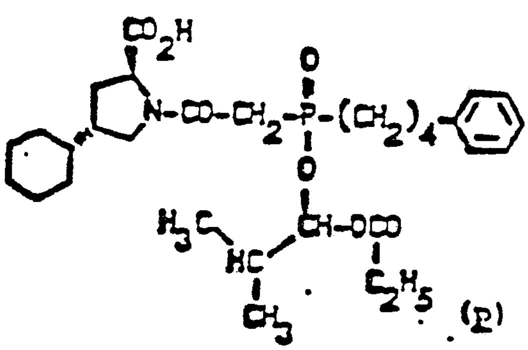

(p)

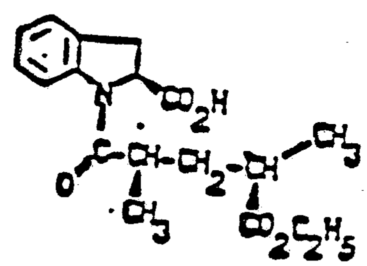

(q)

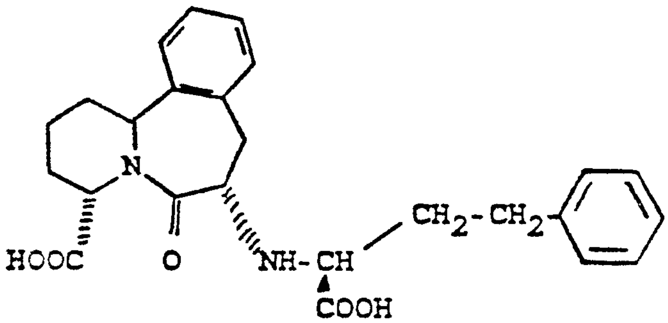

(r)

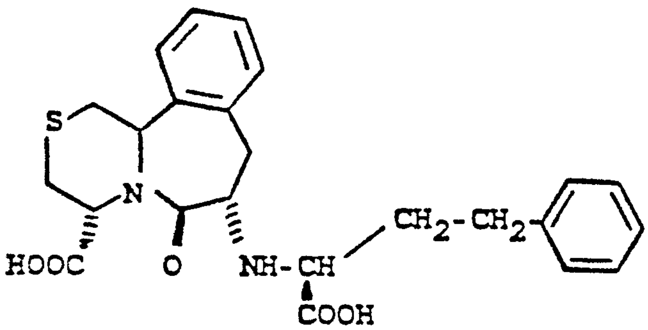

(s)

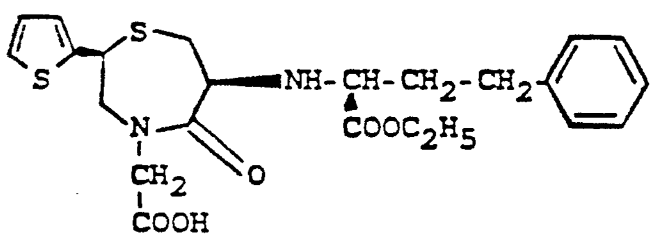

(t)

Von den u.a. aus der EP-A-24365 bekannten ACE-Inhibitoren der Formel III

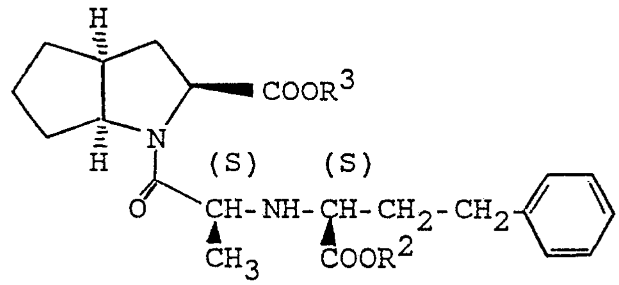

(III)

in welcher
$R^2$ und $R^3$ gleich oder verschieden sind und Wasserstoff, einen aliphatischen Rest mit 1-21 C-Atomen, einen alicyclischen Rest mit 3-20 C-Atomen oder einen araliphatischen Rest mit 7-32 C-Atomen bedeuten, sind jene bevorzugt, worin
$R^2$ Wasserstoff, Methyl, Ethyl, Benzyl, Menthyl oder n-Octyl und
$R^3$ Wasserstoff, Benzhydryl, n-Octyl, n-Decyl oder 5-Nonyl bedeutet.

Weiterhin sind von dem aus der EP-A-84164 und der EP-A-24365 bekannten ACE-Inhibitoren der Formeln IVa und IVb

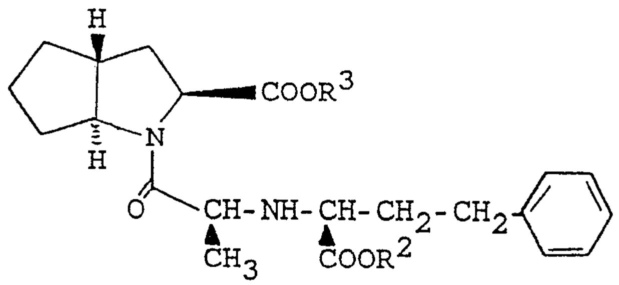

(IVa)

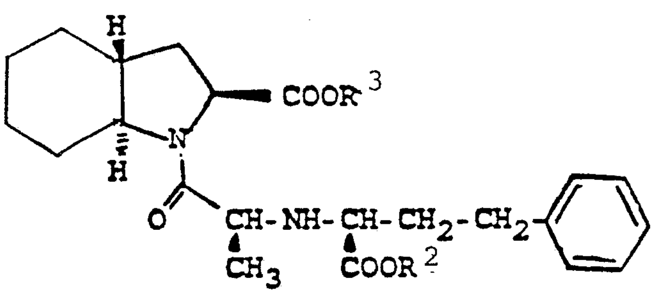

(IVb)

in welchen $R^2$ und $R^3$ wie oben bei Formel III definiert sind, jene bevorzugt, worin
$R^2$ Wasserstoff, $(C_1-C_8)$-Alkyl, Benzyl oder Menthyl und
$R^3$ Wasserstoff, Benzhydryl oder $(C_1-C_{10})$-Alkyl bedeutet.

Darüber hinaus bevorzugt sind die aus US-Patent 4620012 und EP-A-243645 bekannten ACE-Inhibitoren der Formel V

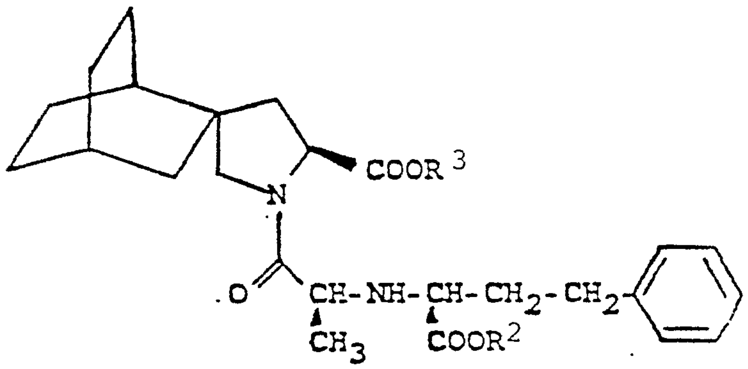

(V)

in welchen
$R^1$ Wasserstoff, ($C_1$-$C_8$)-Alkyl, Benzyl oder Menthyl und
$R^2$ Wasserstoff, Benzhydryl oder ($C_1$-$C_{10}$)-Alkyl bedeutet
und ihre Isomeren.

Aus der EP-A-243645 ist außerdem bekannt, daß die Verbindungen der Formel I und II außerdem eine nootrope (die cognitive Funktion verbessernde) Wirkung aufweisen und daher auch für die Behandlung cognitiver Dysfunktionen unterschiedlicher Genese, wie sie z.B. bei der Alzheimer'schen Krankheit oder der senilen Demenz auftreten, geeignet sind.

Es wurde nun überraschend gefunden, daß die Verbindungen der Formel I und II eine psychotrope, insbesondere eine anxiolytische Wirkung aufweisen. Sie sind daher für die Behandlung und Prophylaxe von Störungen des zentralen Nervensystems, insbesondere von Angstzuständen, geeignet.

Die anxiolytische Wirkung wurde in verschiedenen Testmodellen geprüft so, z.B. im Lick-shock-conflict-Test nach VOGEL und im "Geller-Seifter-Konflikt-Test" nach GELLER und SEIFTER.

**Lick-shock-conflict-Test**

Methode (VOGEL, J.R., Psychopharmacologia 21, (1971), 1-7)

Männliche Wistar Ratten aus eigener Aufzucht (SPF Hattersheim) im Gewicht zwischen 90 und 120 g werden verwendet. Den Tieren wird für 48 Stunden vor Testbeginn das Trinkwasser entzogen. Zum Test werden die Tiere in eine Plastikbox (14x12x28 cm, BxTxH) gesetzt, die mit einer Wasserflasche mit Metall-Trinkrohr ausgestattet ist und die außerdem erlaubt, über eine elektronische Schaltung die Anzahl der Kontakte der Zunge des Tieres mit dem Trinkrohr zu messen. Der Boden der Box besteht aus Metallstäben, die durch die Steuerungselektronik unter Strom gesetzt werden können.

Die Tiere haben nach dem Einsetzen in die Box 5 min. Zeit, um das Trinkrohr zu finden und 50-mal daran zu lecken. Tiere, die das Trinkrohr innerhalb dieser Zeit nicht gefunden haben, werden für den Versuch nicht verwendet. Nach diesen 50 Leckvorgängen (lickings) werden Trinkrohr und Bodenstäbe für jeweils 5 sec unter Strom gesetzt (Gleichstrom 300 µA) und dann für weitere 5 sec. wieder freigegeben. Diese Folge wird alternierend für eine Zeit von 5 min. weitergeführt, wobei die Zahl der Trinkrohr-Kontakte des Tieres während der Reizstrom- und der reizstromfreien Phase auf verschiedenen elektronischen Zählern registriert werden.

Gruppen von jeweils acht Tieren pro Dosis werden mit den Prüfsubstanzen, auf unterschiedlichen Applikationswegen, z.B. oral, intraperitoneal oder subcutan, behandelt. Bei oraler Applikation per Schlundsonde werden die Prüfsubstanzen in einen 1 %igen Tylose-Gel aufgeschwemmt und 5 ml/kg Körpergewicht über die Schlundsonde appliziert. Die Prüfung in der obg. Testapparatur erfolgt bei oraler Gabe 1 Stunde, bei subcutaner oder intraperitonealer Gabe 30 Minuten nach Applikation der Prüfsubstanzen. Die Anzahl der Trinkrohr-Kontakte in der Reizstrom-Phase dient als Prüf-Variable. Die mittlere Anzahl der Kontakte in dieser Phase bei der Kontrollgruppe wird zu 100 % gesetzt und Zu-oder Abnahme der Kontaktzahl bei den mit Prüfsubstanz behandelten Tieren wird prozentual in Bezug auf die Kontrollgruppe ausgedrückt.

Anxiolytika bewirken in diesem Test gewöhnlich eine deutliche Zunahme der Wasseraufnahme (lickings) in der Reizstrom-Phase gegenüber den unbehandelten Kontrollen.

Falls eine lineare oder logarithmische Beziehung zwischen Dosis und Wirkung gegeben ist, wird eine ED + 100 (d.i. die Dosis, die eine Zunahme der Wasseraufnahme um 100 % gegenüber der Kontrollgruppe bewirkt) mittels Regressionsanalyse errechnet. Ist eine lineare Dosisabhängigkeit nicht gegeben, so wird eine minimal effektive Dosis (MED) bestimmt, d.i. die niedrigste Dosis der Prüfsubstanz, die noch eine statistisch signifikante Zunahme der Wasseraufnahme im Vergleich zur Kontrollgruppe bewirkt ($p = 0,05$, DUNNETT-Test).

Zahl der Trinkrohr-Kontakte in der Reizstromphase 30 Minuten nach Verabreichung der Prüfsubstanz im Vergleich zu der Kontrollgruppe

| Verbindung | Dosis (mg/kg i.p.) | | | | | |
|---|---|---|---|---|---|---|
| | 0,1 | 0,3 | 1 | 3 | 10 | 30 |
| Captopril | | | | + 46 | + 109 | + 117* |
| Analapril | | | | + 42 | + 114 | + 130* |
| Ramipril | | | | + 88 | + 148* | + 113 |
| A | | | | + 1 | + 32 | + 65 |
| B | | | | + 53 | + 116* | + 176* |
| C | + 16 | + 36/+ 28 | + 73/+ 108* | + 124 | | |

*$p < 0.05$ (Dunnett-Test)

$$R^3OOC-\overset{*}{C}H(R^4)-N(R^5)-C(O)-\overset{*}{C}H(R^1)-NH-\overset{*}{C}H(COOR^2)-(CH_2)_n-R \qquad (II)$$

| Verbindung | n | R | $R^1$ | $R^2$ | $R^3$ | -CH-N- $R^4$ $R^5$ |
|---|---|---|---|---|---|---|
| A | 2 | $C_6H_5$ | $CH_3$ | $C_2H_5$ | H | 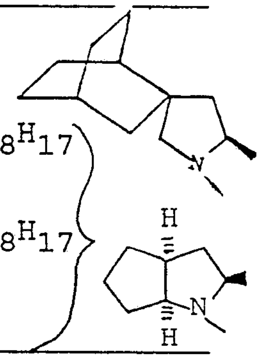 |
| B | 2 | $C_6H_5$ | $CH_3$ | H | n-$C_8H_{17}$ | |
| C | 2 | $C_6H_5$ | $CH_3$ | $C_2H_5$ | n-$C_8H_{17}$ | |
| Ramipril | 2 | $C_6H_5$ | $CH_3$ | $C_2H_5$ | H | |

**Geller-Seifter-Konflikt-Test**

Methode (Geller, I. und Seifter, J., Psychopharmacologia 1 (1962), 482):

Männliche Wistar Ratten aus eigener Aufzucht (SPF Hattersheim) im Gewicht zwischen 240 und 370 g werden verwendet und Versuchsgruppen zu je 8 Tieren zugeordnet. Je 4 Tiere werden in Plastikkäfigen (56x38x20 cm) zusammengesetzt und durch abgewogene Futtermengen auf ca. 80 % ihres normalen Körpergewichts gehalten. Die Tiere werden trainiert, in einer SKINNER-Box eine Taste zu drücken um eine Belohnung in Form von gesüßter Kondensmilch zu erhalten. Die Box enthält zwei Tasten mit Mikroschaltern, einen Lautsprecher, ein Hauslicht, zwei Signallichter über den Tasten sowie einen Fußbcden aus Metallstäben. Das Trainingsschema wurde der Arbeit von GELLER und SEIFTER (1962) in der Modifikation von DAVIDSON & COOK (Psychopharmacologia 15 (1969), 159-168) entlehnt: Jede Sitzung besteht aus vier 15-Minuten-Abschnitten, die alle aus einer 12-minütigen "Variable Interval" (VI)-Phase und einer 3-minütigen "Fixed ration" (FR)-Phase zusammengesetzt sind. Während der VI-Phase erhalten die Tiere auf Tastendruck Milchbelohnungen in einem durch einem Zufallsgenerator gesteuerten Intervall von 10-110 sec. mit einem Mittelwert um 60 ±15 sec. Während der FR-Phase erhalten die Tiere für jeden Tastendruck eine Belohnung, zusätzlich wird jedoch bei jedem 3. Tastendruck ein schmerzhafter elektrischer Reiz über die Fußbodenstäbe verabreicht, um eine Konfliktsituation zu erzeugen. Die Stromstärke des elektrischen Reizes wird für jedes Tier individuell so eingestellt (0,3-0,6 mA), daß die Tastendruckrate in der gesamten FR-Phase zwischen 5 und 15 liegt.

Das Training findet an 5 Tagen in der Woche statt, Versuche mit Prüfsubstanzen werden einmal wöchentlich durchgeführt. Da die Tiere als ihre eigene Kontrolle dienen, werden vor jedem Versuch mit Prüfsubstanz mindestens zwei Vorwerte ohne Prüfsubstanz durchgeführt. Die zu prüfenden Verbindungen werden in einem 1 %igen Tylose-Gel suspendiert und 30 min vor Testbeginn oral der Schlundsonde in einem Volumen von 2 ml/kg verabreicht. Veränderungen der Tastendruckrate in der VI-Phase werden als Beeinflussung der motorischen Aktivität und Steigerungen der Tastendruckrate in der FR-Phase werden als Anzeichen für eine

"Antikonflikt-" bzw. "anxiolytische" Wirkung gewertet.

Bestimmt wird meist die minimal effektive Dosis (MED) der Prüfsubstanz, d.i. die niedrigste geprüfte Dosis, die noch eine statistisch signifikante Änderung der Tastendruckrate bewirkt (p = 0,05; WILCOXON matched pairs signed rank test).

Die in diesen beiden Versuchsmodellen ermittelten MED's nach intraperitonealer Gabe liegen zwischen 0,1 und 30 mg/kg Versuchstier.

Die Erfindung betrifft daher weiter die Anwendung der erfindungsgemäßen Verbindungen bei der Behandlung und Prophylaxe von Störungen des zentralen Nervensystems, insbesondere von Angstzuständen.

Die Erfindung betrifft außerdem neue Verbindungen der Formel III

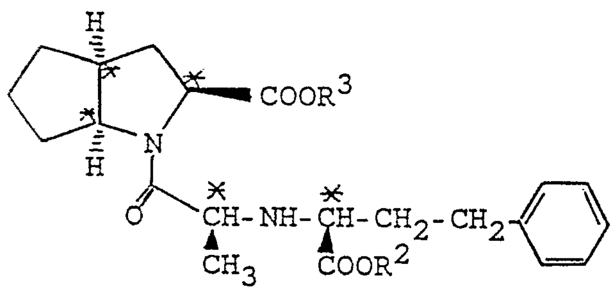

(III)

in welcher die fünf chiralen C-Atome (*) jeweils die S-Konfiguration aufweisen,
$R^2$ Ethyl oder Wasserstoff bedeutet und
$R^3$ n-Octyl bedeutet,
sowie deren physiologisch verträglichen Salze, vorzugsweise Maleinate.

Ein erfindungsgemäßes Verfahren zur Herstellung dieser neuen Verbindungen ist dadurch gekennzeichnet, daß man

a) eine Verbindung der Formel III, in welcher die fünf chiralen C-Atome (*) jeweils die S-Konfiguration aufweisen, $R^2$ Ethyl oder eine basisch, sauer oder hydrogenolytisch leicht abspaltbare Carboxyl-Schutzgruppe bedeutet und $R^3$ Wasserstoff bedeutet, mit n-Octanol umsetzt, vorzugsweise unter Anwendung dem Fachmann vertrauter Veresterungsmethoden (siehe z.B. Buehler, Pearson, Survey of Organic Synthesis, Vol. 1, New York 1970, S. 802-825; Houben-Weyl, Methoden der Organischen Chemie, Band E5, 1985, S.656-773), z.B. unter saurer Katalyse oder nach Aktivierung der Carbonsäurefunktion von III ($R^3 = H$) bzw. der Hydroxyfunktion von n-Octanol, insbesondere unter den Bedingungen einer Mitsunobu-Reaktion, in einem geeigneten Lösungsmittel bei einer Temperatur bis zum Siedepunkt des Reaktionsgemisches,

oder daß man

b) eine Verbindung der Formel III, in der die Konfiguration sowie $R^2$ und $R^3$ wie oben unter (a) definiert sind, mit einer Verbindung der Formel VI

$H_3C-[CH_2-]_7X$ (VI)

in der X eine Abgangsgruppe, die nucleophil verdrängt werden kann, insbesondere ein Cl, Br, I-Atom oder einen Sulfonsäurerest, bedeutet, unter Bedingungen einer nucleophilen Substitution, vorzugsweise in einem polaren organischen Lösungsmittel, wie einem Alkohol, vorzugsweise Methanol, Ethanol, Propanol oder Isopropanol oder einem niederen Keton, vorzugsweise Aceton, Methylethylketon oder Methylisobutylketon oder in Acetonitril, Dimethylformamid, Dimethylsulfoxid oder Sulfolan oder einem Kohlenwasserstoff, vorzugsweise Toluol, mit oder ohne Gegenwart einer Hilfsbase zum Abfangen der sich bildenden Säure, vorzugsweise in Gegenwart von Kaliumhydrogencarbonat, Natriumcarbonat, Triethylamin, Pyridin, 1.5-Diazabicyclo[5.4.0]undec-5-en oder 1,5-Diazabicyclo[4.3.0]non-5-en, sowie mit oder ohne Gegenwart eines Alkalihalogenids, vorzugsweise Natriumjodid oder Kaliumjodid, bei einer Temperatur zwischen -50 und +100 °C, vorzugsweise zwischen -20 und +60 °C, umsetzt,

oder daß man

c) eine Verbindung der Formel III, die wie oben unter (a) konfiguriert ist und in welcher $R^2$ Wasserstoff bedeutet und $R^3$ n-Octyl bedeutet, mit Ethanol wie unter Verfahrensvariante (a) beschrieben, umsetzt,

oder daß man

d) eine Verbindung der Formel III, in der die Konfiguration, $R^2$ und $R^3$ wie oben unter (c) definiert sind, mit einer Verbindung der Formel VII

$H_3C-CH_2-X$ (VII)

in der X eine Abgangsgruppe, die nucleophil verdrängt werden kann, bedeutet, wie unter Verfahrensvariante (b) beschrieben, umsetzt,

oder daß man

e) eine Verbindung der Formel VIII

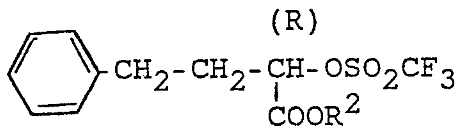

(VIII)

in der $R^2$ wie oben unter (a) definiert ist, mit einer Verbindung der Formel IX

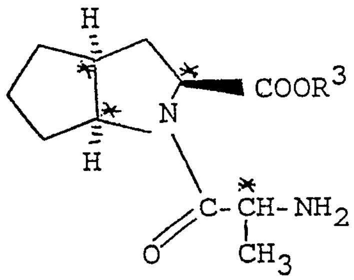

(IX)

in welcher die vier chiralen C-Atome (*) jeweils die S-Konfiguration aufweisen, z.B. analog der in US-Patent 4525301 beschriebenen Verfahrensweise in einem geeigneten Lösungsmittel bei einer Temperatur bis zum Siedepunkt des Reaktionsgemisches umsetzt,
oder daß man
f) eine Verbindung der Formel X

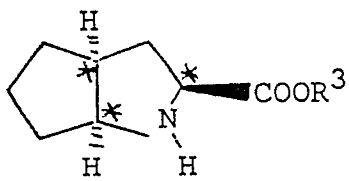

(X)

in welcher die drei chiralen C-Atome (*) jeweils die S-Konfiguration aufweisen und $R^3$ n-Octyl bedeutet, mit einer Verbindung der Formel XI

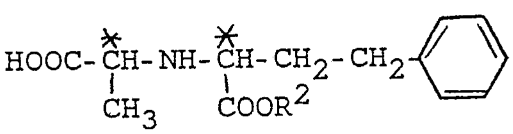

(XI)

in welcher $R^2$ wie oben unter (a) definiert ist, beispielsweise in Analogie zu bekannten Peptidkupplungsverfahren in einem organischen Lösungsmittel wie DMF, $CH_2Cl_2$, DMA in Gegenwart von Kupplungshilfsmitteln, wie Carbodiimiden (z.B. Dicyclohexylcarbodiimid), Diphenylphosphorylazid, Alkanphosphonsäureanhydriden, Dialkylphosphinosäureanhydriden oder N,N-Disuccinimidoylcarbonat in einem Lösungsmittel wie z.B. Acetonitril oder nach Aktivierung der Verbindungen der Formel X,z.B. durch Umsetzung mit Tetraethyldiphosphit oder nach Umwandlung der Verbindungen der Formel XI in Aktivester (z.B. mit 1-Hydroxybenzotriazol), in gemischte Anhydride (z.B. mit Chlorameisensäureestern), in Azide oder in Carbodiimid-Derivate (vgl. Schröder, Lübke, The Peptides, Band 1, New York 1965, Seiten 76-136) bei Temperaturen vorzugsweise zwischen -20 °C und dem Siedepunkt des Lösungsmittels umsetzt, erforderlichenfalls in einer erhaltenen Verbindung der Formel III ($R^2$ = Schutzgruppe) die Schutzgruppe $R^2$ in an sich bekannter Weise, zum Beispiel unter Anwendung dem Fachmann vertrauter Verseifungsmethoden (wie saure oder alkalische Hydrolyse) oder Hydriermethoden in einem geeigneten Lösungsmittel bei einer Temperatur bis zum Siedepunkt des Reaktionsgemisches abspaltet und die auf diese Weise erhaltenen Verbindung der Formel III ($R^2$ = Ethyl oder Wasserstoff) gegebenenfalls in ihr physiologisch verträgliches Salz überführt,

wobei, falls als Ausgangsmaterialien der Verfahrensvarianten (a)-(f) Stereoisomerengemische eingesetzt werden, daran anschließend in einer weiteren Trennstufe das all-S-Isomere der Formel III ($R^2$ = Wasserstoff oder Ethyl, $R^3$ = n-Octyl) abgetrennt wird.

Eine hydrogenolytisch abspaltbare Carboxyl-Schutzgruppe, wie Bzl, wird vorzugsweise durch Hydrogenolyse an einem geeigneten Katalysator wie z.B. Palladium auf Aktivkohle bei einem Druck von 0,2 bis 10 bar und einer Temperatur zwischen 0 °C und 100 °C in einem organischen Lösungsmittel abgespalten.

Leicht verseifbare aliphatische Reste, wie ($C_1$-$C_6$)-Alkyl, sind die bevorzugten basisch oder sauer abspaltbare Carboxyl-Schutzgruppen. Sie werden unter Anwendung dem Fachmann vertrauter Verseifungsmethoden (siehe z.B. Houben/Weyl, Methoden der Organischen Chemie, Band E 5/1, Seite 223-255), z.B. durch saure oder alkalische Hydrolyse abgespalten.

Die Verbindungen der Formel III ($R^2$ = Wasserstoff oder Ethyl, $R^3$ = Wasserstoff) sind bekannt (siehe z.B. EP-A 79022, US-Patent 4587258).

Verbindungen der Formel VI und VII sind bekannt und großteils käuflich erhältlich.

Verbindungen der Formel VIII werden aus den entsprechenden Hydroxyl-Verbindungen durch Überführung der Hydroxylgruppe in die $-OSO_2CF_3$-Gruppe nach gängigen Verfahren erhalten.

Aus Diastereomeren- bzw. Enantiomerengemischen können die obengenannten neuen all-S-Verbindungen durch Umkristallisieren oder durch Chromatographie, z.B. an Kieselgel, bzw. durch Salzbildung mit optisch aktiven Hilfsstoffen abgetrennt werden.

Die Erfindung betrifft auch Zwischenprodukte der Formel XII

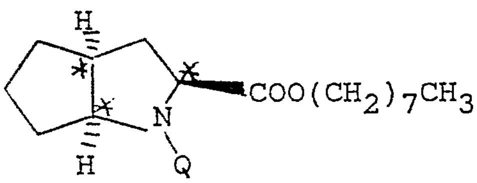

(XII)

in welcher die drei chiralen C-Atome jeweils die S-Konfiguration aufweisen und Q Wasserstoff oder den Rest XIII

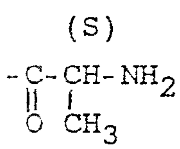

(XIII)

bedeutet und Gemische aus Verbindungen der Formel XII und ihren Stereoisomeren, sowie ein Verfahren zur Herstellung dieser Verbindungen das dadurch gekennzeichnet ist, daß man

a) eine Verbindung der Formel XIV

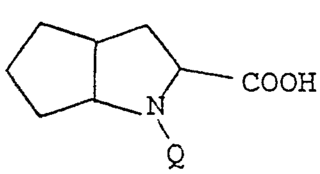

(XIV)

in der Q Wasserstoff oder $-CO-CH(CH_3)-NH_2$ bedeutet, mit n-Octanol oder $H_3C-[CH_2]_7-X$ wie oben bei Verfahrensvariante (a) oder (b) umsetzt oder

b) eine Verbindung der Formel XV

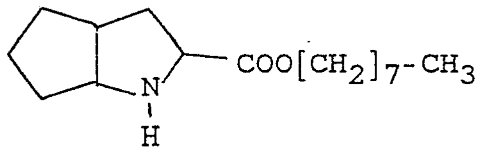

(XV)

wie oben bei Verfahrensvariante (f) mit einem aminogeschützten, beispielsweise mit Z oder Boc geschützten Alanin umsetzt und anschließend die Aminoschutzgruppe abspaltet und gewünschtenfalls das all-S-Isomer der Formel XII isoliert.

Verbindungen der Formel XIV sind beispielsweise aus EP-A-79022 oder US-Patent 4587258 bekannt.

Die neuen all-S-Verbindungen der Formel III ($R^2$ = H oder Ethyl, $R^3$ = n-Octyl) und ihre physiologisch verträglichen Salze weisen neben der oben erwähnten psychotropen und insbesondere anxiolytischen Wirkung eine starke nootrope, d.h. die cognitive Funktion verbessernde, Wirkung auf. Sie sind daher für die Behandlung cognitiver Dysfunktionen unterschiedlicher Genese, wie sie z.B. bei der Alzheimer'schen Krankheit oder der senilen Demenz auftreten, geeignet. Die nootrope Wirkung der erfindungsgemäßen Verbindungen wurde an Mäusen, die ein Körpergewicht von 20-25 g besaßen, im inhibitory (passive) avoidance test (step-through-Modell) geprüft. Eine modifizierte Form der von J. KOPP, Z. BODANECKY und M.E. JARVIK beschriebenen Testmethode wurde von J. BURES, O. BURESOVA und J. HUSTON in "Techniques and Basic Experiments for the Study of Brain and Behavior", Elsevier Scientific Publishers, Amsterdam (1983) beschrieben.

Entsprechend diesen Literaturangaben wird eine Substanz dann als nootrop wirksam bezeichnet, wenn sie bei den Versuchstieren die mittels eines elektroconvulsiven Schocks erzeugte Amnesie oder die mittels Scopolamin induzierte Amnesie aufzuheben vermag.

Die Versuche wurden nach modifizierten Testmethoden durchgeführt. Als Vergleichsverbindung diente das bekannte Nootropikum 2-Oxo-1-pyrrolidinylessigsäureamid (Piracetam). Die deutliche Überlegenheit der erfindungsgemäßen Verbindungen über die Vergleichssubstanz zeigte sich darin, daß die Scopolamin-induzierte Amnesie im inhibitory avoidance-Test sich mit einer MED (minimal effective Dosis) von 0,03-30 mg/kg p.o. aufheben läßt. Die Vergleichssubstanz hat eine MED von ca. 500-1000 mg/kg p.o.

Die Erfindung betrifft daher weiter die Anwendung der erfindungsgemäßen Verbindungen bei der Behandlung und Prophylaxe cognitiver Dysfunktionen.

Die Erfindung umfaßt weiterhin die genannten neuen Wirkstoffe enthaltende Arzneimittel, Verfahren zu deren Herstellung sowie dei Verwendung der erfindungsgemäßen Verbindungen bei der Herstellung von Arzneimitteln, die zur Behandlung und Prophylaxe der vorstehend genannten Krankheiten in Säugern wie Affen, Hunden, Katzen, Ratten, Menschen etc. eingesetzt werden.

Die Erfindung umfaßt weiterhin die genannten Verbindungen der Formeln I und II enthaltende Arzneimittel, Verfahren zu deren Herstellung sowie die Verwendung der erfindungsgemäßen Verbindungen bei der Herstellung von Arzneimitteln, die zur Behandlung und Prophylaxe von Störungen des zentralen Nervensystems, insbesondere von Angstzuständen geeignet sind.

In Ausübung der erfindungsgemäßen Methode können die oben beschriebenen Angiotensin-Converting-Enzyme-Inhibitoren der Formel I und II an Säugern wie Affen, Hunden, Katzen, Ratten, Menschen etc. angewendet werden.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt bis etwa 95%, vorteilhafterweise zwischen 10 und 75 %, beträgt.

Welche Hilfsstoffe für die gewünschte Arzneimittelformulierung geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können beispielsweise oral, rektal oder parenteral (z.B. intravenös oder subcutan) appliziert werden, wobei die orale Applikation bevorzugt ist.

Für eine orale Anwendungsform werden die aktiven Verbindungen mit den dafür geeigneten Zusatzstoffen wie Trägerstoffen, Stabilisatoren oder inerten Verdünnungsmitteln vermischt und durch die üblichen Methoden in geeignete Darreichungsformen gebracht wie Tabletten, Dragees, Steckkapseln, wässrige, alkoholische oder ölige Suspensionen oder wässrige, alkoholische oder ölige Lösungen. Als inerte Träger können z.B. Gummi arabicum, Magnesia, Magnesiumcarbonat, Milchzucker, Glucose oder Stärke, insbesondere Maisstärke verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht wie Sonnenblumenöl oder Lebertran.

Zur subcutanen oder intravenösen Applikation werden die aktiven Verbindungen oder deren physiologisch

verträgliche Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage Wasser, physiologische Kochsalzlösung oder Alkohole, z.B. Ethanol, Propanol, Glycerin, daneben auch Zuckerlösungen wie Glucose- oder Mannitlösungen oder auch eine Mischung aus den verschiedenen genannten Lösungsmitteln.

Die nachfolgenden Beispiele sollen die erfindungsgemäßen Verbindungen und Verfahren erläutern, ohne die Erfindung auf die hier stellvertretend genannten Substanzen bzw. Verfahren zu beschränken. Weiter werden Anwendungsformen zur Prophylaxe und Behandlung von Störungen des zentralen Nervensystems nach der erfindungsgemäßen Methode angegeben.

**Beispiel 1**

2-[N-(1S-Ethoxycarbonyl-3-phenylpropyl)-S-alanyl]-(1S,3S,5S)-2-azabicyclo[3.3.0]octan-3-carbonsäure-n-octylester

2.07 g (5 mmol) 2-[N-(1S-Ethoxycarbonyl-3-phenylpropyl)-S-alanyl]-(1S,3S,5S)-2-azabicyclo[3.3.0]-octan-3-carbonsäure (Ramipril) und 0,50 g (5 mmol) Kaliumhydrogencarbonat werden in 25 ml Dimethylformamid 1,5 Stunden bei 40 °C gerührt, nach Abkühlen auf Raumtemperatur eine Lösung von 1,16 g (6 mmol) 1-Bromoctan in 20 ml Dimethylformamid zugetropft und über Nacht bei Raumtemperatur gerührt. Durch Zugabe von 0,1 N HCl wird auf pH 6 gestellt, mit Wasser verdünnt, dreimal mit Methylenchlorid extrahiert und die vereinigten organischen Phasen getrocknet, eingeengt und durch Säulenchromatographie an 120 g Kieselgel (Laufmittel Toluol/Ethanol 98:2) gereinigt.
Ausbeute: 2,35 g (89 %) öliges Produkt;
$[\alpha]_D^{25}$ = -23,9° (c=1, Methanol)

**Beispiel 2**

2-[N-(1S-Ethoxycarbonyl-3-phenylpropyl)-S-alanyl]-(1S,3S,5S)-2-azabicyclo[3.3.0]octan-3-carbonsäure-n-octylester-hydrogenmaleinat

528 mg (1 mmol) eines nach Beispiel 1 gewonnenen Amins werden in 20 ml Ether gelöst und mit einer Lösung von 116 mg (1 mmol) Maleinsäure in 4 ml Aceton versetzt. Die Lösungsmittel werden abgedampft und der Rückstand mit Diisopropylether kristallisiert.
Ausbeute: 0,51 g (79 %) farblose Kristalle, Schmp. 89-90 °C

**Beispiel 3**

2-[N-(1S-Ethoxycarbonyl-3-phenylpropyl)-S-alanyl]-(1S,3S,5S)-2-azabicyclo[3.3.0]octan-3-carbonsäure-n-octylester

Zu einer Lösung von 1,97 g (7,5 mmol) Triphenylphosphin und 0,65 g (5 mmol) n-Octanol in 100 ml absolutem Tetrahydrofuran wird bei 0 °C eine Lösung von 1,31 g (7,5 mmol) Azodicarbonsäurediethylester in 10 ml absolutem Tetrahydrofuran zugetropft, 10 Minuten nachgerührt, dann bei 0 °C eine Lösung von 2,80 g (5 mmol) 2-[N-(1S-Ethoxycarbonyl-3-phenylpropyl)-S-alanyl]-(1S,3S,5S)-2-azabicyclo[3.3.0]octan-3-carbonsäure (Ramipril) in 25 ml absolutem Tetrahydrofuran zugegeben, eine Stunde bei 0 °C und über Nacht bei Raumtemperatur gerührt. Die Reaktionslösung wird eingeengt, in Essigester aufgenommen, zweimal mit 2 N Natronlauge und einmal mit Wasser gewaschen, getrocknet, eingeengt und das Rohprodukt (5,0 g) durch Flash-Chromatographie an 200 g Kieselgel (Laufmittel Methylenchlorid/Essigester 9:1) gereinigt. Man erhält 0,83 g (31 %) der Titelverbindung.

**Beispiel 4**

2-[N-(1S-Carboxy-3-phenylpropyl)-S-alanyl]-(1S,3S,5S)-2-azabicyclo[3.3.0]octan-3-carbonsäure-n-octylester

2.65 g (5 mmol) Ethylester aus Beispiel 1 werden in 18 ml Tetrahydrofuran gelöst, 7,5 ml 1N Natronlauge zugegeben und 48 Stunden bei Raumtemperatur gerührt. Man neutralisiert durch Zugabe von 7,5 ml 1N Salzsäure. Das Reaktionsgemisch wird eingeengt, der Rückstand in Wasser suspendiert, zweimal mit Essigester extrahiert, die vereinigten organischen Phasen mit gesättigter Natriumchloridlösung gewaschen, getrocknet, eingeengt und das Rohprodukt (2,05 g) durch Chromatographie an 80 g Kieselgel (Toluol/Ethanol 9:1) gereinigt. Das so erhaltene Produkt (1,15 g; 46 %) wird in 50 ml Petrolether verrieben, kaltgestellt, abgesaugt und getrocknet.
Ausbeute: 0,83 g farblose Kristalle; Schmp. 56-61 °C

**Beispiel 5**

2-[N-(1S-Ethoxycarbonyl-3-phenylpropyl)-S-alanyl]-(1S,3S,5S)-2-azabicyclo[3.3.0]octan-3-carbonsäure-n-octylester

1,40 g (2,8 mmol) Carbonsäure aus Beispiel 4 werden in 25 ml ethanolischer Salzsäure bei

Raumtemperatur 3 Tage lang gerührt. Dann wird eingeengt, der Rückstand in Essigester aufgenommen, dreimal mit gesättigter Natriumbicarbonatlösung und einmal mit Wasser gewaschen, getrocknet, eingeengt und das Rohprodukt durch Säulenchromatographie (Laufmittel Toluol/Ethanol 98:2) gereinigt. Man erhält 850 mg (57 %) der Titelverbindung.

**Beispiel 6**

2-[N-(1S-Ethoxycarbonyl-3-phenylpropyl)-S-alanyl]-(1S,3S,5S)-2-azabicyclo[3.3.0]octan-3-carbonsäure-n-octylester

2,50 g (5 mmol) Carbonsäure aus Beispiel 4 und 1,00 g (10 mmol) Kaliumhydrogencarbonat werden in 25 ml Dimethylformamid eine Stunde bei 40 °C gerührt, nach Abkühlen auf Raumtemperatur eine Lösung von 0,66 g (6 mmol) Bromethan in 20 ml Dimethylformamid zugetropft und über Nacht bei Raumtemperatur gerührt. Man gibt auf Wasser, extrahiert dreimal mit Essigester, wäscht die vereinigten organischen Phasen mehrfach mit Wasser, trocknet, engt ein und reinigt das Rohprodukt durch Säulenchromatographie an Kieselgel (Laufmittel Toluol/Ethanol 98:2). Man erhält 210 g (80 %) der Titelverbindung.

**Beispiel 7**

2-[N-(1S-Ethoxycarbonyl-3-phenylpropyl)-S-alanyl]-(1S,3S,5S)-2-azabicyclo[3.3.0]octan-3-carbonsäure-n-octylester

7a) 2-Tert.butyloxycarbonyl-(1S,3S,5S)-2-azabicyclo[3.3.0]octan-3-carbonsäurebenzylester

Zu einer Lösung von 40,0 g (0,163 mol) (1S,3S,5S)-2-Azabicyclo[3.3.0]octan-3-carbonsäurebenzylester und 23,4 ml (0,169 mol) absolutem Triethylamin in 300 ml absolutem Methylenchlorid wird bei 0 °C eine Lösung von 39,2 g (0,180 mol) Di-tert.butyldicarbonat in 60 ml absolutem Methylenchlorid langsam zugetropft, 15 Minuten bei 0 °C und eine Stunde bei Raumtemperatur nachgerührt. Die Reaktionslösung wird mit 10%iger Citronensäurelösung, gesättigter Natriumbicarbonatlösung und Wasser gewaschen, getrocknet und eingeengt.
Ausbeute: 55,6 g öliges Produkt
$[\alpha]_D^{25}$ = -1,2° (c=2, Methanol)

7b) 2-Tert.butyloxycarbonyl-(1S,3S,5S)-2-azabicyclo[3.3.0]octan-3-carbonsäure

55,6 g (0,16 mol) Benzylester aus Beispiel 7a) werden in 2 l Ethanol bei Raumtemperatur an 4 g Palladium/Kohle (10 %) während 2,5 Stunden hydriert. Der Katalysator wird abgesaugt und das Filtrat eingeengt.
Ausbeute: 37,3 g (90 %)
$[\alpha]_D^{25}$ = +22,7° (c=1, Methanol)

7c) 2-Tert.butyloxycarbonyl-(1S,3S,5S)-2-azabicyclo[3.3.0]octan-3-carbonsäure-n-octylester

32,3 g (0,127 mol) Säure aus Beispiel 7b) und 25,3 g (0,253 mol) Kaliumhydrogencarbonat werden in 500 ml Dimethylformamid 1,5 Stunden bei 40 °C gerührt. Nach Abkühlen werden 48,9 g (0,253 mol) 1-Bromoctan zugetropft und über Nacht bei Raumtemperatur gerührt. Die Reaktionsmischung wird auf Wasser gegeben, dreimal mit Essigester extrahiert, die vereinigten organischen Phasen mit gesättigter Natriumhydrogencarbonatlösung und Wasser gewaschen, getrocknet, eingeengt und das Rohprodukt (44,3 g) durch Flash-Chromatographie an Kieselgel (900 g; Laufmittel Toluol/Ethanol 95:5 bzw. 99,5:0,5) in zwei Portionen gereinigt.
Ausbeute: 35,4 g (76 %) öliges Produkts
$[\alpha]_D^{25}$ = +5,7° (c=1, Methanol)

7d) (1S,3S,5S)-2-Azabicyclo[3.3.0]octan-3-carbonsäure-n-octylester

2,6 g (7,0 mmol) BOC-Verbindung aus Beispiel 7c) werden bei 0 °C mit 9 ml Trifluoressigsäure 1,5 Stunden lang gerührt. Die überschüssige Säure wird im Vakuum abgedampft, der Rückstand in Wasser aufgenommen, mit Natriumbicarbonat basisch gestellt, mit Essigester extrahiert, die organische Phase noch einmal mit Wasser gewaschen, getrocknet, eingeengt und das Produkt rasch weiter umgesetzt.
Ausbeute: 1,8 g (95 %) öliges Produkt

7e)
2-[N-(1S-Ethoxycarbonyl-3-phenylpropyl)-S-alanyl]-(1S,3S,5S)-2-azabicyclo[3.3.0]octan-3-carbonsäure-n-octylester

Zu einer Lösung von 1,76 g (6,6 mmol) Amin aus Beispiel 7d) in 10 ml Methylenchlorid werden bei -10 °C nacheinander 6,44 g (56 mmol) N-Ethylmorpholin, 1,84 g (6,6 mmol) N-(1S-Ethoxycarbonyl-3-phenylpropyl)-S-alanin und 4 ml Propanphosphonsäureanhydrid (50 % in Methylenchlorid) zugetropft. Man rührt 3 Stunden bei 0 °C und 3 Stunden bei Raumtemperatur nach, engt ein, gibt den Rückstand auf Wasser, extrahiert mit Essigester und wäscht die organische Phase mit Wasser, 25 %iger Natriumhydrogensulfatlösung und gesättigter Natriumhydrogencarbonatlösung. Die Lösung wird getrocknet, eingeengt und durch

Säulenchromatographie an Kieselgel (Laufmittel Toluol/Ethanol 98:2) gereinigt. Man erhält 2,47g(71 %) der Titelverbindung.

**Beispiel 8**

2-[N-(1S-Ethoxycarbonyl-3-phenylpropyl)-S-alanyl]-(1S,3S,5S)-2-azabicyclo[3.3.0]octan-3-carbonsäure-n-octylester-hydrogenmaleinat

8a) (1S,3S,5S)-2-Azabicyclo[3.3.0]octan-3-carbonsäure-n-octylester-hydrochlorid
Zu einer Suspension von 10 g (64 mmol) (1S,3S,5S)-2-Azabicyclo[3.4.0]octan-3-carbonsäure in 100 ml destilliertem n-Octanol tropft man bei 40 °C 16,3 g (150 mmol) destilliertes Trimethylsilylchlorid und rührt über Nacht bei 40 °C. Die flüchtigen Bestandteile werden am Rotationsverdampfer entfernt, das Octanol durch Kurzwegdestillation im Hochvakuum abgetrennt, der Destillationsrückstand in Methylenchlorid aufgenommen, eingeengt und zweimal mit Diisopropylether verrieben. Man erhält 14,6 g (75 %) der Titelverbindung.
Schmp. 76-78 °C
$[\alpha]_D^{25}$ = -23,7° (c=1, Methanol)

8b)
2-[N-(1S-Ethoxycarbonyl-3-phenylpropyl)-S-alanyl]-(1S,3S,5S)-2-azabicyclo[3.3.0]octan-3-carbonsäure-n-octylester-hydrogenmaleinat
2,0 g (6,6 mmol) Amin-hydrochlorid aus Beispiel 8a) werden in 30 ml Methylenchlorid und 10 ml Wasser suspendiert und der pH durch Zugabe von gesättigter Kaliumcarbonatlösung auf 9-10 eingestellt. Dann werden 1,84 g (6,6 mmol) N-[1S-Ethoxycarbonyl-3-phenylpropyl)-S-alanin und einer Lösung von 4,1 ml Methylethylphosphinsäureanhydrid, in 4 ml Methylenchlorid nacheinander zugegeben. Man rührt über Nacht bei Raumtemperatur, verdünnt mit Methylenchlorid und Wasser, wäscht die organische Phase mit einem Kaliumsulfat/Kaliumhydrogensulfat-Puffer, Natriumbicarbonatlösung und Natriumchloridlösung, trocknet, engt ein und fällt aus dem Rohprodukt (2,92 g) durch Lösen in 33 ml Diisopropylether und Zugabe einer Lösung von 766 mg Maleinsäure in 4 ml Aceton die Titelverbindung. Man erhält 2,50 g (67 %).

**Beispiel 9**

2-[N-(1S-Ethoxycarbonyl-3-phenylpropyl)-S-alanyl]-(1S,3S,5S)-2-azabicyclo[3.3.0]octan-3-carbonsäure-n-octylester

9a) 2-[N-Tert.butyloxycarbonyl-S-alanyl]-(1S,3S,5S)-2-azabicyclo[3.3.0]octan-3-carbonsäure-n-octylester
Zu einem Gemisch aus 2,67 g (10 mmol) Amin aus Beispiel 7d), 1,89 g (10 mmol) BOC-S-Alanin und 1,00 g (10 mmol) Triethylamin in 50 ml absolutem Dimethylformamid tropft man in Eisbad langsam 10 ml einer Propanphosphonsäureanhydridlösung (50 % in Methylenchlorid) und rührt 4 Stunden bei Raumtemperatur. Die Lösung wird mit 200 ml Wasser versetzt, zweimal mit Essigester extrahiert, die vereinigten organischen Phasen mit Wasser, 10 %iger Zitronensäurelösung, gesättigter Natriumhydrogencarbonatlösung und gesättigter Natriumchloridlösung gewaschen, getrocknet, eingeengt und das Rohprodukt durch Säulenchromatographie an Kieselgel (Laufmittel Toluol/Ethanol 99:1) gereinigt. Man erhält 3,05 g (70 %) der Titelverbindung.
$[\alpha]_D^{25}$ = -39,9° (c=1, Methanol)

9b) 2-(S-Alanyl-(1S,3S,5S)-2-azabicyclo[3.3.0]octan-3-carbonsäure-n-octylester
6,70g (15,9 mmol) BOC-Verbindung aus Beispiel 9a) werden in 30 ml Trifluoressigsäure 90 Minuten bei 0 °C gerührt. Das Lösungsmittel wird abgedampft, der Rückstand in Ethanol aufgenommen, durch Zugabe von Kaliumcarbonat neutralisiert, filtriert und eingeengt. Man erhält 5,3 g (98 %) der Titelverbindung.

9c)
2-[N-(1S-Ethoxycarbonyl-3-phenylpropyl)-S-alanyl]-(1S,3S,5S)-2-azabicyclo[3.3.0]octan-3-carbonsäure-n-octylester
Zu einer Lösung von 1,35 g (4,0 mmol) Amin aus Beispiel 9b) in 25 ml absolutem Methylenchlorid gibt man bei 0 °C nacheinander 0,8g (8 mmol) Triethylamin und eine Lösung von 1,36 g (4 mmol) 4-Phenyl-(2R)-trifluormethylsulfonyloxybuttersäureethylester in 10 ml absolutem Methylenchlorid und rührt über Nacht bei Raumtemperatur. Die Reaktionslösung wird mit Wasser gewaschen, getrocknet, eingeengt und das Rohprodukt durch Säulenchromatographie an Kieselgel (Laufmittel Toluol/Ethanol 99,5:0,5, 99:1) gereinigt. Man erhält 0,28 g (13 %) der Titelverbindung.

**Beispiel 10**

Herstellung des erfindungsgemäß verwendeten Mittels zur oralen Anwendung in der Behandlung und Prophylaxe cognitiver Dysfunktionen, sowie zur Behandlung und Prophylaxe von Störungen des zentralen Nervensystems.

1000 Tabletten, die je 10 mg 2-[N-(1-S-Carbethoxy-3-phenyl-propyl)-S-alanyl]-1S,3S,5S-2-azabicyclo[3.3.0]octan-3-carbonsäure-n-octylester enthalten, werden mit den folgenden Hilfsmitteln hergestellt:

| | |
|---|---|
| 2-[N-(1-S-Carbethoxy-3-phenyl-propyl)-S-alanyl]-(1,3S,5S)-2-azabicyclo[3.3.0]octan-3-carbonsäure n-octylester | 10 g |
| Maisstärke | 140 g |
| Gelatine | 7,5 g |
| Mikrokristalline Cellulose | 2,5 g |
| Magnesiumstearat | 2,5 g |

2-[N-(1-S-Carbethoxy-3-phenyl-propyl)-S-alanyl]-(1S,3S,5S)-2-azabicyclo[3.3.0]octan-3-carbonsäure-n-octylester und Maisstärke werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das entstandene Granulat wird zu 1000 Tabletten gepreßt, wobei jede Tablette 10 mg der Wirksubstanz enthält. Diese Tabletten können für die oben genannten Indikationen verwendet werden.

**Beispiel 11**

Analog Beispiel 10 werden 1000 Tabletten hergestellt, die je 10 mg 1′-[N-(1-S-Carbethoxy-3-phenylpropyl)-S-alanyl]-(3′S,5′S)-Spirobicyclo[2.2.2]octan-2,3′-pyrrolidin-5′-carbonsäure enthalten.

**Beispiel 12**

Gelatine-Kapseln, die je 10 mg 1′-[N-(1-S-Carbethoxy-3-phenylpropyl)-S-alanyl]-(3′S,5′S)-spirobicyclo[2.2.2]octan-2,3′-pyrrolidin-5′-carbonsäure enthalten, werden mit der folgenden Mischung gefüllt:

| | |
|---|---|
| 1′-[N-(1-S-Carbethoxy-3-phenylpropyl)-S-alanyl]-(3′S,5′S)-spirobicyclo[2.2.2]octan-2,3′-pyrrolidin-5′-carbonsäure | 10 mg |
| Magnesiumstearat | 1 mg |
| Lactose | 214 mg |

Diese Kapseln können zur Behandlung und Prophylaxe von Störungen des zentralen Nervensystems verwendet werden.

**Beispiel 13**

Die Herstellung einer Injektionslösung wird im folgenden beschrieben:

| | |
|---|---|
| 2-[N-1-S-Carboxy-3-phenyl-propyl)-S-alanyl]-1S,3S,5S)-2-asabicyclo[3.3.0]octan-3-carbonsäure | 250 mg |
| Methylparaben | 5 g |
| Propylparaben | 1 g |
| Natriumchlorid | 25 g |
| Wasser für Injektion | 5 l |

2-[N-(1-S-Carboxy-3-phenyl-propyl)-S-alanyl]-(1S,3S,5S)-2-azabicyclo[3.3.0]octan-3-carbonsäure, die Konservierungsstoffe und Natriumchlorid werden in 3 l Wasser für Injektion gelöst und auf 5 l mit Wasser für Injektion aufgefüllt. Die Lösung wird steril gefiltert und aseptisch in vorsterilisierte Flaschen gefüllt, die mit sterilisierten Gummikappen verschlossen werden. Jede Flasche enthält 5 ml Lösung.

**Beispiel 14**

Tabletten, die zur Behandlung oder Prophylaxe von Störungen des zentralen Nervensystems verwendet werden können, werden wie in Beispiel 10 beschrieben hergestellt, mit der Ausnahme, daß anstelle von

2-[N-(1-S-Carbethoxy-3-phenyl-propyl)-S-alanyl]-(1S,3S,5S)-2-azabicyclo[3.3.0]octan-3S-carbonsäure-n-octylester 2-[N-(1-S-Carboxy-3-phenyl-propyl)-S-alanyl]-(1S,3S,5S)-2-azabicyclo[3.3.0]octan-3-carbonsäure oder 1-[N-(1-S-Carboxy-3-phenyl-propyl)-S-alanyl]-(2S,3aR,7aS)-octahydroindol-2-carbonsäure oder
1-[N-(1-S-Carbethoxy-3-phenyl-propyl)-S-alanyl]-cis-2,3,3a,4,5,7a-hexahydro[1H]indol-2-S-endo-carbonsäure oder
1-[N-(1-S-Carboxy-3-phenyl-propyl)-S-alanyl]-cis-2,3,3a,4,5,7a-hexahydro[1H]indol-2S-endo-carbonsäure oder
2-[N-(1-S-Carboxy-3-phenyl-propyl)-S-lysyl]-(1S,3S,5S)-2-azabicyclo[3.3.0]octan-3-carbonsäure oder
2-[N-(1-S-Carbethoxy-3-cyclohexyl-propyl)-S-alanyl]-(1S,3S,5S)-2-azabicyclo[3.3.0]octan-3-carbonsäure oder
N-(1-S-Carboxy-3-cyclohexyl-propyl)-S-lysyl-(1S,3S,5S)-2- azabicyclo[3.3.0]octan-3-carbonsäure oder
1′-[N-(1-S-Carbethoxy-3-phenyl-propyl)-S-alanyl]-exo-spirobicyclo[2.2.2]octan-2,3′-pyrrolidin-5′-S-carbonsäure oder
(S,S,S)-1-Methyl-2-(1-carbethoxy-3-phenyl-propyl)-2H-undecahydro-cyclopenta[4.5]pyrrolo[1,2-a]pyrazin-3.8-dion oder
1′-[N-(1-S-Carbethoxy-3-phenylpropyl)-S-alanyl]-endo-spirobicyclo[2.2.2]octan-2,3′-pyrrolidinyl-5′-S-carbonsäure oder
2-[N-(1-S-Carbethoxy-3-phenylpropyl)-S-alanyl]-cis-endo-2-azabicyclo[3.3.0]octan-3-S-carbonsäure-n-octylester oder
2-[N-(1-S-Carbethoxy-3-phenylpropyl)-S-alanyl]-cis-endo-2-azabicyclo[3.3.0]octan-3-S-carbonsäuredecylester oder
2-[N-(1-S-Carbethoxy-3-phenylpropyl)-S-alanyl]-cis-endo-2-azabicyclo[3.3.0]octan-3-S-carbonsäure-(5-nonyl)ester oder
2-[N-(1-S-Octyloxy-3-phenylpropyl)-S-alanyl]-cis-endo-2-azabicyclo[3.3.0]propan-3-S-carbonsäureoctylester oder
2-[N-(1-S-Menthyloxy-3-phenylpropyl)-S-alanyl]-cis-endo-2-azabicyclo[3.3.0]octan-3-S-carbonsäure-benzhydrylester
verwendet werden.

**Beispiel 15**

Eine Injektionslösung wird analog der in Beispiel 13 beschriebenen Vorschrift hergestellt, mit der Ausnahme, daß anstelle von 2-[N-(1-S-Carbethoxy-3-phenyl-propyl)-S-alanyl]-(1S,3S,5S)-2-azabicyclo[3.3.0]octan-3-carbonsäure oder
2-[N-(1-S-Carboxy-3-phenyl-propyl)-S-alanyl]-(1S,3S,5S)-2-azabicyclo[3.3.0]octan-3-carbonsäure oder
1-[N-(1-S-Carbethoxy-3-phenyl-propyl)-S-alanyl]-(2S,3aR,7aS)-octahydroindol-2-carbonsäurehydrochlorid oder
1-[N-(1-S-Carboxy-3-phenyl-propyl)-S-alanyl]-(2S,3aR,7aS)-octahydroindol-2-carbonsäure oder
1-[N-(1-S-Carbethoxy-3-cyclohexyl-propyl)-S-alanyl]-cis-2,3,3a,4,5,7a-hexahydro[1H]indol-2-S-endo-carbonsäure oder
1-[N-(1-S-Carboxy-3-phenyl-propyl)-S-alanyl]-cis-2,3,3a,4,5,7a-hexahydro[1H]indol-2-S-endo-carbonsäure oder
2-[N-(1-Carboxy-3-phenyl-propyl)-S-lysyl]-(1S,3S,5S)-2- azabicyclo[3.3.0]octan-3-carbonsäure oder
2-[N-(1-S-Carbethoxy-3-cyclohexyl-propyl)-S-alanyl]-(1S,3S,5S)-2-azabicyclo[3.3.0]octan-3-carbonsäure oder
2-[N-(1-S-Carboxy-3-cyclohexyl-propyl)-S-lysyl]-(1S,3S,5S)-2-azabicyclo[3.3.0]octan-3-carbonsäure oder
1′[N-(1-S-Carboxy-3-phenylpropyl)-S-alanyl]-endo-spirobicyclo[2.2.2]octan-2,3′-pyrrolidin-5′-S-carbonsäure
1′[N-(1-S-Carboxy-3-phenylpropyl)-S-alanyl]-exo-spirobicyclo[2.2.2]octan-2,3′-pyrrolidin-5′-S-carbonsäure
angewendet werden.

**Beispiel 16**

Herstellung des erfindungsgemäß verwendeten Mittels zur oralen Anwendung in der Behandlung und Prophylaxe cognitiver Dysfunktionen, sowie zur Behandlung und Prophylaxe von Störungen des zentralen Nervensystems.

1000 Tabletten, die je 15 mg 2-[N-(1-S-Carbethoxy-3-phenyl-propyl)-S-alanyl]-1S,3S,5S-2-azabicyclo[3.3.0]octan-3-carbonsäure-n-octylester-Maleinat enthalten, werden mit den folgenden Hilfsmitteln hergestellt:

| | |
|---|---|
| 2-[N-(1-S-Carbethoxy-3-phenyl-propyl)-S-alanyl]-(1S,3S,5S)-2-azabicyclo[3.3.0]octan-3-carbonsäure n-octylester-Maleinat | 15 g |
| Maisstärke | 200 g |
| Gelatine | 10 g |
| Mikrokristalline Cellulose | 4 g |
| Magnesiumstearat | 4 g |

2-[N-(1-S-Carbethoxy-3-phenyl-propyl)-S-alanyl]-(1S,3S,5S)-2-azabicyclo[3.3.0]octan-3-carbonsäure-n-octylester-Maleinat und Maisstärke werden mit einer wäßrigen Gelatine-Lösung gemischt. Die Mischung wird getrocknet und zu einem Granulat vermahlen. Mikrokristalline Cellulose und Magnesiumstearat werden mit den Granulat vermischt. Das entstandene Granulat wird zu 1000 Tabletten gepreßt, wobei jede Tablette 15 mg der Wirksubstanz enthält.
Diese Tabletten können für die oben genannten Indikationen verwendet werden.

**Beispiel 17**

Herstellung des erfindungsgemäß verwendeten Mittels zur oralen Anwendung in der Behandlung und Prophylaxe cognitiver Dysfunktionen, sowie zur Behandlung und Prophylaxe von Störungen des zentralen Nervensystems.

1000 Tabletten, die je 8 mg 2-[N-(1-S-Carboxy-3-phenyl-propyl)-S-alanyl]-1S,3S,5S-2-azabicyclo[3.3.0]octan-3-carbonsäure-n-octylester enthalten, werden mit den folgenden Hilfsmitteln hergestellt:

| | |
|---|---|
| 2-[N-(1-S- Carboxy -3-phenyl-propyl)-S-alanyl]-(1S,3S,5S)-2-azabicyclo[3.3.0]octan-3-carbonsäure n-octylester | 8 g |
| Maisstärke | 120 g |
| Gelatine | 7 g |
| Mikrokristalline Cellulose | 2 g |
| Magnesiumstearat | 2 g |

2-[N-(1-S-Carboxy-3-phenyl-propyl)-S-alanyl]-(1S,3S,5S)-2-azabicyclo[3.3.0]octan-3-carbonsäure-n-octylester und Maisstärke werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das entstandene Granulat wird zu 1000 Tabletten gepreßt, wobei jede Tablette 8 mg der Wirksubstanz enthält.
Diese Tabletten können für die oben genannten Indikationen verwendet werden.

**Patentansprüche**

1. Verwendung eines ACE-Inhibitors zur Herstellung eines Arzneimittels mit psychotroper Wirkung.
2. Verwendung eines ACE-Inhibitors zur Herstellung eines Arzneimittels mit anxiolytischer Wirkung.
3. Verwendung gemäß Anspruch 1 oder 2, dadurch gekennzeichnet, daß Angiotensin-Converting-Enzyme-Inhibitoren der Formel I

$X^1 - X^2$ (I)

in welcher

$X^1$ 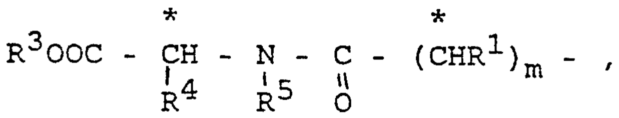

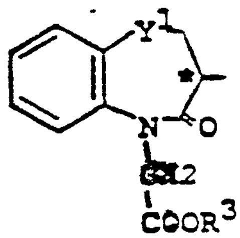 , 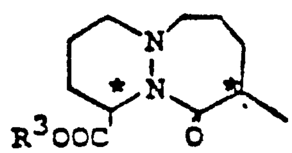 oder 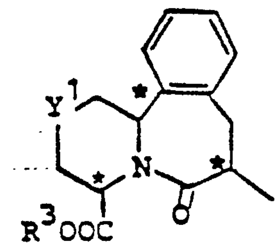 ,

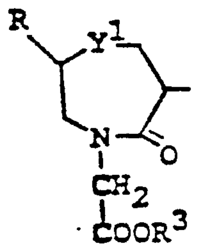 bedeutet,

$X^2$ $-CH_2SH$ , 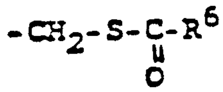 , 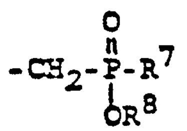 oder

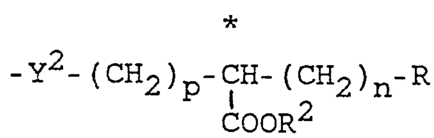 bedeutet,

$Y^1$ für -S- oder $-CH_2-$ steht,
$Y^2$ für $-NR^9-$ oder $-CH_2-$ steht,
m = 0 oder 1 ist,
n = 0, 1 oder 2 ist,
p = 0 oder 1 ist,
R = Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclisch-aliphatischen Rest mit 4 - 20 C-Atomen,
einen gegebenenfalls substituierten heteroaromatischen oder heteroaromatisch-($C_1$-$C_8$)-aliphatischen Rest mit 5 - 12 Ringatomen, oder
einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,

einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder,
falls nicht von vorstehenden Definitionen schon umfaßt, die erforderlichenfalls geschützte Seitenkette einer natürlich vorkommenden α-Aminosäure bedeutet,
$R^2$ und $R^3$ gleich oder verschieden sind und
Wasserstoff, einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen Rest der Formel

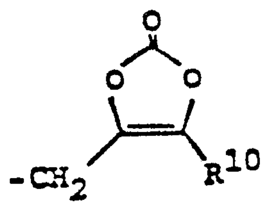

worin $R^{10}$ Wasserstoff, einen aliphatischen Rest mit 1-6 C-Atomen oder einen gegebenenfalls substituierten aromatischen Rest mit 6-12 C-Atomen bedeutet,
einen Rest der Formel

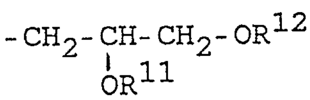 oder 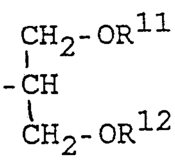

worin $R^{11}$ und $R^{12}$ gleich oder verschieden unabhängig voneinander Wasserstoff, einen gegebenenfalls substituierten Alkylrest mit 1-23 C-Atomen oder einen gegebenenfalls substituierten Acylrest mit 1-23 C-Atomen bedeuten,
bedeuten,
$R^4$ für Wasserstoff oder ($C_1$-$C_6$)Alkyl und
$R^5$ für ($C_1$-$C_6$)Alkyl, ($C_3$-$C_6$)-Cycloalkyl oder

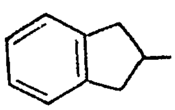 stehen oder

$R^4$ und $R^5$ zusammen mit den sie tragenden Atomen ein mono-, bi-, oder tricyclisches heterocyclisches Ringsystem mit 3 bis 15 Ring-C-Atomen bilden,
$R^6$ Wasserstoff, Amino, ($C_1$-$C_6$)-Alkyl, ($C_6$-$C_{12}$)-Aryl oder ($C_7$-$C_{13}$)-Aralkyl,
$R^7$ ($C_1$-$C_6$)-Alkyl oder ($C_7$-$C_{13}$)-Aralkyl, vorzugsweise $-(CH_2)_4-C_6H_5$,
$R^8$ ($C_1$-$C_6$)-Alkyl, das gegebenenfalls durch ($C_1$-$C_6$)-Alkanoyloxy monosubstituiert ist, vorzugsweise 2-Methyl-1-propionyloxy-propyl, und
$R^9$ Wasserstoff oder ($C_1$-$C_6$)-Alkyl bedeuten;
oder deren physiologisch unbedenkliche Salze verwendet werden.

4. Verwendung gemäß Anspruch 1 oder 2, dadurch gekennzeichnet, daß Angiotensin-Converting-Enzyme-Inhibitoren der Formel II

$$R^3OOC-\overset{*}{C}H(R^4)-N(R^5)-C(=O)-\overset{*}{C}H(R^1)-NH-\overset{*}{C}H(COOR^2)-(CH_2)_n-R \qquad (II)$$

in welcher
n = 1 oder 2 ist,
R = Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclisch-aliphatischen Rest mit 7 - 14 C-Atomen,
einen gegebenenfalls substituierten heteroaromatischen oder heteroaromatisch-($C_1$-$C_8$)-aliphatischen Rest mit 5 - 12 Ringatomen, oder
einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder,
falls nicht von vorstehenden Definitionen schon umfaßt, die erforderlichenfalls geschützte Seitenkette einer natürlich vorkommenden α-Aminosäure bedeutet,
$R^2$ und $R^3$ gleich oder verschieden sind und
Wasserstoff, einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7-32 C-Atomen,
einen Rest der Formel

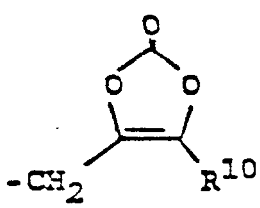

worin $R^{10}$ Wasserstoff, einen aliphatischen Rest mit 1-6 C-Atomen oder einen gegebenenfalls substituierten aromatischen Rest mit 6-12 C-Atomen bedeutet,
einen Rest der Formel

$$-CH_2-CH(OR^{11})-CH_2-OR^{12} \quad \text{oder} \quad -CH(CH_2-OR^{11})(CH_2-OR^{12})$$

worin $R^{11}$ und $R^{12}$ gleich oder verschieden unabhängig voneinander Wasserstoff, einen gegebenenfalls substituierten Alkylrest mit 1-23 C-Atomen oder einen gegebenenfalls substituierten Acylrest mit 1-23 C-Atomen bedeuten,

bedeuten,
und
$R^4$ und $R^5$ zusammen mit den sie tragenden Atomen ein mono-, bi- oder tricyclisches heterocyclisches Ringsystem mit 3 bis 15 Ring-C-Atomen bilden,
oder deren physiologisch verträglichen Salze verwendet werden.

5. Verwendung gemäß einem oder mehreren der Ansprüche 1 bis 4, dadurch gekennzeichnet, daß Verbindungen der Formel I, vorzugsweise solche der Formel II verwendet werden, in welchen

a) n = 1 oder 2 ist;

b) R

1. Wasserstoff bedeutet;
2. Alkyl mit 1 - 18 C-Atomen bedeutet;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine ganze Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (C-2) stehen;
5. Aryl mit 6 - 12 C-Atomen bedeutet, das durch ($C_1$-$C_8$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_8$)-alkylamino, ($C_1$-$C_4$)-Alkanoylamino, Methylendioxy, Carboxy, Cyano und/oder Sulfamoyl, mono-, di- oder trisubstituiert sein kann;
6. falls n = 2 ist, ($C_6$-$C_{12}$)-Aryl-($C_1$-$C_8$)-alkyl oder Di-($C_6$-$C_{12}$)-aryl-($C_1$-$C_8$)-alkyl bedeutet, die im Arylteil jeweils wie unter b)5. beschrieben substituiert sein können; oder
7. Alkoxy mit 1 - 4 C-Atomen;
8. Aryloxy mit 6 - 12 C-Atomen, das wie unter b)5. beschrieben substituiert sein kann;
9. mono- bzw. bicyclisches Heteroaryloxy oder Heteroaryl-($C_1$-$C_8$)-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen, die im Heteroaryl wie unter b)5. beschrieben substituiert sein können;
10. Amino-($C_1$-$C_8$)-alkyl;
11. ($C_1$-$C_4$)-Alkanoylamino-($C_1$-$C_8$)-alkyl;
12. ($C_7$-$C_{13}$)-Aroylamino-($C_1$-$C_8$)-alkyl;
13. ($C_1$-$C_4$)-Alkoxy-carbonylamino-($C_1$-$C_8$)-alkyl;
14. ($C_6$-$C_{12}$)-Aryl-($C_1$-$C_4$)-alkoxycarbonylamino-($C_1$-$C_8$)-alkyl;
15. ($C_6$-$C_{12}$)-Aryl-($C_1$-$C_4$)-alkylamino-($C_1$-$C_8$)-alkyl;
16. ($C_1$-$C_4$)-Alkylamino-($C_1$-$C_8$)-alkyl;
17. Di-($C_1$-$C_4$)-alkylamino-($C_1$-$C_8$)-alkyl;
18. Guanidino-($C_1$-$C_8$)-alkyl,
19. Imidazolyl;
20. Indolyl;
21. ($C_1$-$C_4$)-Alkylthio;
22. falls n = 2 ist, ($C_1$-$C_4$)-Alkylthio-($C_1$-$C_8$)-alkyl;
23. ($C_6$-$C_{12}$)-Arylthio-($C_1$-$C_8$)-alkyl; das im Arylteil wie unter b) 5. beschrieben, substituiert sein kann,
24. ($C_6$-$C_{12}$)-Aryl-($C_1$-$C_8$)-alkylthio, das im Arylteil wie unter b) 5. beschrieben substituiert sein kann;
25. falls n = 2 ist, Carboxy-($C_1$-$C_8$)-alkyl;
26. Carboxy;
27. Carbamoyl;
28. falls n = 2 ist, Carbamoyl-($C_1$-$C_8$)-alkyl;
29. ($C_1$-$C_4$)-Alkoxy-carbonyl-($C_1$-$C_8$)-alkyl;
30. falls n = 2 ist, ($C_6$-$C_{12}$)-Aryloxy-($C_1$-$C_8$)-alkyl, das im Arylteil wie unter b) 5. beschrieben substituiert sein kann; oder
31. ($C_6$-$C_{12}$)-Aryl-($C_1$-$C_8$)-alkoxy, das im Arylteil wie unter b) 5. beschrieben substituiert sein kann, bedeutet;

c) $R^1$

1. Wasserstoff bedeutet;
2. Alkyl mit 1 - 18 C-Atomen bedeutet;
3. einen aliphatischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;

5. Aryl mit 6 - 12 C-Atomen,
das wie unter b) 5. beschrieben substituiert sein kann;
6. $(C_6-C_{12})$-Aryl-$(C_1-C_8)$-alkyl oder $(C_7-C_{13})$-Aroyl-$(C_1-C_8)$-alkyl,
die beide im Arylteil wie unter b) 5. beschrieben substituiert sein können;
7. mono- bzw. bicyclisches, gegebenenfalls teilhydriertes Heteroaryl oder Heteroaryl-$(C_1-C_8)$-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen,
die im Heteroaryl wie bei Aryl unter b) 5. beschrieben substituiert sein können; oder
8. falls von c) 1. - 8. noch nicht umfaßt,
die gegebenenfalls geschützte Seitenkette einer natürlich vorkommenden α-Aminosäure der Formel $R^1$-$CH(NH_2)$-COOH bedeutet;
d) $R^2$ und $R^3$ gleich oder verschieden sind und
1. Wasserstoff bedeutet;
2. Alkyl mit 1 - 18 C-Atomen;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;
5. Di-$(C_1-C_4)$-alkylamino-$(C_1-C_8)$-alkyl;
6. $(C_1-C_5)$-Alkanoyloxy-$(C_1-C_8)$-alkyl;
7. $(C_1-C_6)$-Alkoxy-carbonyloxy-$(C_1-C_8)$-alkyl;
8. $(C_7-C_{13})$-Aroyloxy-$(C_1-C_8)$-alkyl;
9. $(C_6-C_{12})$-Aryloxycarbonyloxy$(C_1-C_8)$-alkyl;
10 Aryl mit 6 - 12 C-Atomen;
11. $(C_7-C_{20})$-Aralkyl;
12. Phthalidyl;
13. einen Rest der Formel

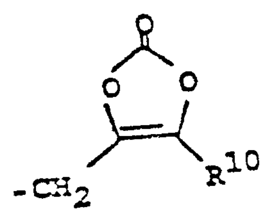

bedeutet,
worin $R^{10}$ Wasserstoff, $(C_1-C_6)$-Alkyl oder Aryl mit 6-12 C-Atomen ist,
14. einen Rest der Formel

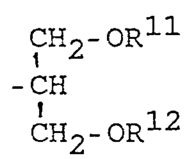

bedeutet, worin $R^{11}$ und $R^{12}$ gleich oder verschieden unabhängig voneinander Wasserstoff, einen gegebenenfalls substituierten Alkylrest mit 1-23 C-Atomen oder einen gegebenenfalls substituierten Acylrest mit 1-23 C-Atomen bedeuten,
wobei die unter d) 8., 9., 10. und 11. genannten Reste wie unter b) 5. beschreiben im Arylteil substituiert sein können; und
e) $R^4$ und $R^5$ zusammen mit den sie tragenden Atomen ein mono-, bi- oder tricyclisches heterocyclisches Ringsystem mit 3 bis 15 Ring-C-Atomen bilden.

6. Verwendung gemäß einem oder mehreren der Ansprüche 1 bis 5, dadurch gekennzeichnet, daß Verbindungen der Formel I, vorzugsweise solche der Formel II verwendet werden, in welchen
n = 1 oder 2 ist
R Wasserstoff,
Alkyl mit 1 - 8 C-Atomen,

Alkenyl mit 2 - 6 C-Atomen,
Cycloalkyl mit 3 - 9 C-Atomen,
Aryl mit 6 - 12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl, mono-, di- oder trisubstituiert sein kann,
Alkoxy mit 1 - 4 C-Atomen,
Aryloxy mit 6 - 12 C-Atomen,
das wie oben bei Aryl beschrieben substituiert sein kann,
mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen,
das wie oben bei Aryl beschrieben substituiert sein kann,
Amino-($C_1$-$C_4$)-alkyl,
($C_1$-$C_4$)-Alkanoylamino-($C_1$-$C_4$)alkyl,
($C_7$-$C_{13}$)-Aroylamino-($C_1$-$C_4$)-alkyl,
($C_1$-$C_4$)-Alkoxy-carbonylamino-($C_1$-$C_4$)-alkyl,
($C_6$-$C_{12}$)-Aryl-($C_1$-$C_4$)-alkoxycarbonylamino-($C_1$-$C_4$)-alkyl,
($C_6$-$C_{12}$)-Aryl-($C_1$-$C_4$)-alkylamino-($C_1$-$C_4$)-alkyl,
($C_1$-$C_4$)-Alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,
Imidazolyl, Indolyl,
($C_1$-$C_4$)-Alkylthio,
($C_1$-$C_4$)-Alkylthio-($C_1$-$C_4$)-alkyl,
($C_6$-$C_{12}$)-Arylthio-($C_1$-$C_4$)-alkyl,
das im Arylteil wie oben bei Aryl beschrieben, substituiert sein kann,
($C_6$-$C_{12}$)-Aryl-($C_1$-$C_4$)-alkylthio,
das im Arylteil wie oben bei Aryl beschrieben substituiert sein kann,
Carboxy-($C_1$-$C_4$)-alkyl,
Carboxy, Carbamoyl,
Carbamoyl-($C_1$-$C_4$)-alkyl,
($C_1$-$C_4$)-Alkoxy-carbonyl-($C_1$-$C_4$)-alkyl,
($C_6$-$C_{12}$)-Aryloxy-($C_1$-$C_4$)-alkyl,
das im Arylteil wie oben bei Aryl beschrieben substituiert sein kann oder
($C_6$-$C_{12}$)-Aryl-($C_1$-$C_4$)-alkoxy,
das im Arylteil wie oben bei Aryl beschrieben substituiert sein kann,
$R^1$ Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
($C_3$-$C_9$)-Cycloalkyl-($C_1$-$C_4$)-alkyl,
($C_5$-$C_9$)-Cycloalkenyl-($C_1$-$C_4$)-alkyl,
gegebenenfalls teilhydriertes Aryl mit 6 - 12 C-Atomen, das wie oben bei R beschrieben substituiert sein kann,
($C_6$-$C_{12}$)-Aryl-($C_1$-$C_4$)-alkyl oder ($C_7$-$C_{13}$)-Aroyl-($C_1$ oder $C_2$)alkyl
die beide wie das vorstehende Aryl substituiert sein können
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann oder
die gegebenenfalls geschützte Seitenkette einer natürliche vorkommenden $\alpha$-Aminosäure $R^1$-CH($NH_2$)-COOH bedeuten,
$R^2$ und $R^3$ gleich oder verschieden sind und
Wasserstoff,
Alkyl mit 1 - 12 C-Atomen,
Alkenyl mit 2 - 12 C-Atomen,
Di-($C_1$-$C_4$)-alkylamino-($C_1$-$C_8$)-alkyl,
($C_1$-$C_5$)-Alkanoyloxy-($C_1$-$C_8$)-alkyl,
($C_1$-$C_6$)-Alkoxy-carbonyloxy-($C_1$-$C_8$)-alkyl,
($C_7$-$C_{13}$)-Aroyloxy-($C_1$-$C_8$)-alkyl,
($C_6$-$C_{12}$)-Aryloxycarbonyloxy($C_1$-$C_8$)-alkyl,
Aryl mit 6 - 12 C-Atomen,
($C_6$-$C_{12}$)-Aryl-($C_1$-$C_8$)-alkyl,

($C_3$-$C_9$)-Cycloalkyl oder
$C_3$-$C_9$)-Cycloalkyl-($C_1$-$C_8$)-alkyl
bedeuten und
$R^4$ und $R^5$ die oben angegebene Bedeutung haben.

7. Verwendung gemäß einem oder mehreren der Ansprüche 1 bis 6, dadurch gekennzeichnet, daß Verbindungen der Formel I, vorzugsweise solche der Formel II verwendet werden, in welchen
n = 1 oder 2 ist,
R ($C_1$-$C_6$)-Alkyl, ($C_2$-$C_6$)-Alkenyl, ($C_3$-$C_9$)-Cycloalkyl, Amino-($C_1$-$C_4$)-alkyl, ($C_2$-$C_5$)-Acylamino-($C_1$-$C_4$)-alkyl, ($C_7$-$C_{13}$)-Aroylamino-($C_1$-$C_4$)-alkyl, ($C_1$-$C_4$)-Alkoxycarbonylamino-($C_1$-$C_4$)-alkyl, ($C_6$-$C_{12}$)-Aryl- ($C_1$-$C_4$)-alkoxycarbonylamino-($C_1$-$C_4$)- alkyl, ($C_6$-$C_{12}$)-Aryl, das durch ($C_1$-$C_4$)-Alkyl, ($C_1$-$C_4$)-Alkoxy, Hydroxy Halogen, Nitro, Amino, ($C_1$-$C_4$)-Alkylamino, Di-($C_1$-$C_4$)-alkylamino und/oder Methylendioxy mono-, di- oder trisubstituiert sein kann, oder 3-Indolyl, insbesondere Methyl, Ethyl, Cyclohexyl, tert. Butoxycarbonylamino-($C_1$-$C_4$)-alkyl, Benzoyloxycarbonylamino-($C_1$-$C_4$)-alkyl oder Phenyl, das durch Phenyl, ($C_1$-$C_2$)-Alkyl, ($C_1$ oder $C_2$)-Alkoxy, Hydroxy, Fluor, Chlor, Brom, Amino, ($C_1$-$C_4$)-Alkylamino, Di-($C_1$-$C_4$)alkylamino, Nitro und/oder Methylendioxy mono- oder disubstituiert oder im Falle von Methoxy, trisubstituiert sein kann, bedeutet,
$R^1$ Wasserstoff oder ($C_1$-$C_6$)-Alkyl, das gegebenenfalls durch Amino, ($C_1$-$C_6$)-Acylamino oder Benzoylamino substituiert sein kann, ($C_2$-$C_6$)-Alkenyl, ($C_3$-$C_9$)-Cycloalkyl, ($C_5$-$C_9$)-Cycloalkenyl, ($C_3$-$C_7$)-Cycloalkyl-($C_1$-$C_4$)-alkyl, ($C_6$-$C_{12}$)-Aryl oder teilhydriertes Aryl, das jeweils durch ($C_1$-$C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)-alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,
$R^2$ und $R^3$ gleiche oder verschiedene Reste Wasserstoff, ($C_1$-$C_{12}$)-Alkyl, ($C_2$-$C_{12}$)-Alkenyl oder ($C_6$-$C_{12}$)-Aryl-($C_1$-$C_8$)-alkyl, bedeuten und
$R^4$ und $R^5$ die oben angegebene Bedeutung haben.

8. Verwendung gemäß einem oder mehreren der Ansprüche 1 bis 7, dadurch gekennzeichnet, daß Verbindungen der Formel II verwendet werden, worin $R^4$ und $R^5$ zusammen mit den sie tragenden Atomen ein mono-, bi- oder tricyclisches Ringsystem mit 3 bis 15 Ring-C-Atomen, bis zu 2 Ring-S-Atomen und bis zu 2 Ring-N-Atomen vorzugsweise aus der Reihe Pyrrolidin, Tetrahydroisochinolin, Decahydroisochinolin, Octahydroindol, Indolin, Octahydrocyclopenta[b]pyrrol, 2-Azaspiro[4.5]decan, 2-Azaspiro[4.4]nonan, Spiro[(bicyclo[2.2.1]heptan)-2,3′-pyrrolidin], Spiro[(bicyclo-[2.2.2]octan)-2,3′-pyrrolidin], 2-Azatricyclo[4.3.0.1$^{6,9}$]decan, Decahydrocyclohepta[b]pyrrol, Octahydroisoindol, Octahydrocyclopenta[c]pyrrol, 2,3,3a,4,5,7a-Hexahydroindol, 1,2,3,3a,4,6a-Hexahydrocyclopenta[b]pyrrol und 2-Azabicyclo[3.1.0]hexan bilden.

9. Verwendung in einem Verfahren zur Herstellung eines pharmazeutischen Mittels gemäß einem oder mehreren der Ansprüche 1 bis 8, dadurch gekennzeichnet, daß man den Wirkstoff mit den geeigneten Träger-, Hilfs- und/oder Zusatzstoffen in eine geeignete Darreichungsform bringt.

10. Verbindung der Formel III

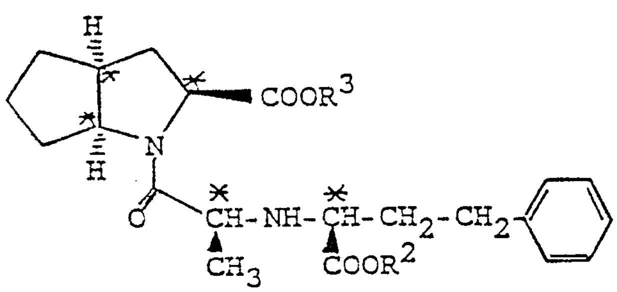

(III)

in welcher die fünf chiralen C-Atome (*) jeweils die S-Konfiguration aufweisen,
$R^2$ Ethyl oder Wasserstoff bedeutet und
$R^3$ n-Octyl bedeutet,
sowie deren physiologisch verträglichen Salze.

11. Verbindung der Formel III gemäß Anspruch 10, worin $R^2$ Ethyl bedeutet, sowie deren physiologisch verträglichen Salze.

12. Verbindung der Formel III gemäß Anspruch 10, worin $R^2$ Wasserstoff bedeutet, sowie deren physiologisch verträglichen Salze.

13. Maleinat der Verbindung gemäß Anspruch 11.

14. Verfahren zur Herstellung einer Verbindung der Formel III gemäß einem der Ansprüche 10 bis 13, dadurch gekennzeichnet, daß man

a) eine Verbindung der Formel III, in welcher die fünf chiralen C-Atome (*) jeweils die S-Konfiguration aufweisen, $R^2$ Ethyl oder eine basisch, sauer oder hydrogenolytisch leicht abspaltbare Carboxyl-Schutzgruppe bedeutet und $R^3$ Wasserstoff bedeutet, mit n-Octanol umsetzt,
oder daß man

b) eine Verbindung der Formel III, in der die Konfiguration sowie $R^2$ und $R^3$ wie oben unter (a) definiert sind, mit einer Verbindung der Formel VI

$H_3C-[CH_2-]_7X$ (VI)

in der X eine Abgangsgruppe, die nucleophil verdrängt werden kann, bedeutet, unter Bedingungen einer nucleophilen Substitution umsetzt,
oder daß man

c) eine Verbindung der Formel III, die wie oben unter (a) konfiguriert ist und in welcher $R^2$ Wasserstoff bedeutet und $R^3$ n-Octyl bedeutet, mit Ethanol wie unter Verfahrensvariante (a) beschrieben, umsetzt,
oder daß man

d) eine Verbindung der Formel III, in der die Konfiguration, $R^2$ und $R^3$ wie oben unter (c) definiert sind, mit einer Verbindung der Formel VII

$H_3C-CH_2-X$ (VII)

in der X eine Abgangsgruppe, die nucleophil verdrängt werden kann, bedeutet, wie unter Verfahrensvariante (b) beschrieben, umsetzt,
oder daß man

e) eine Verbindung der Formel VIII

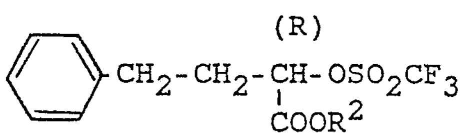

(VIII)

in der $R^2$ wie oben unter (a) definiert ist, umsetzt mit einer Verbindung der Formel IX

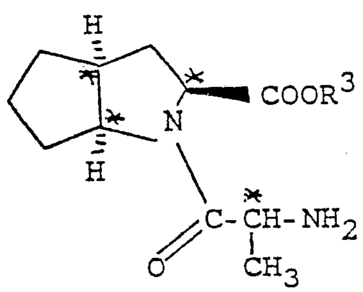

(IX)

in welcher die vier chiralen C-Atome (*) jeweils die S-Konfiguration aufweisen,
oder daß man

f) eine Verbindung der Formel X

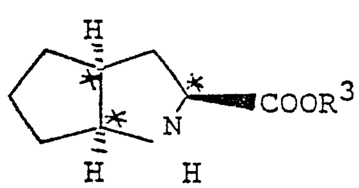

(X)

in welcher die drei chiralen C-Atome (*) jeweils die S-Konfiguration aufweisen und $R^3$ n-Octyl bedeutet, umsetzt mit einer Verbindung der Formel XI

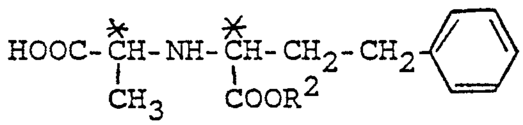

(XI)

in welcher $R^2$ wie oben unter (a) definiert ist, erforderlichenfalls in einer erhaltenen Verbindung der Formel III ($R^2$ = Schutzgruppe) die Schutzgruppe $R^2$ in an sich bekannter Weise abspaltet und die auf diese Weise erhaltenen Verbindung der Formel III ($R^2$ = Ethyl oder Wasserstoff) gegebenenfalls in ihr physiologisch verträgliches Salz überführt,
wobei, falls als Ausgangsmaterialien der Verfahrensvarianten (a)-(f) Stereoisomerengemische eingesetzt werden, daran anschließend in einer weiteren Trennstufe das all-S-Isomere der Formel III ($R^2$ = Wasserstoff oder Ethyl, $R^3$ = n-Octyl) abgetrennt wird.

15. Verbindung der Formel XII

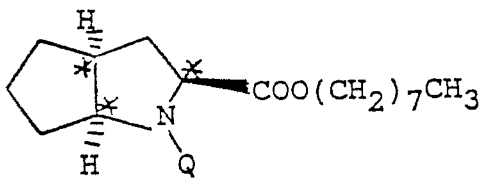

(XII)

in welcher die drei chiralen C-Atome jeweils die S-Konfiguration aufweisen und Q Wasserstoff oder den Rest XIII

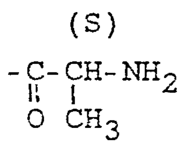

(XIII)

bedeutet.

16. Stereoisomerengemisch, enthaltend eine Verbindung gemäß Anspruch 15.

17. Verfahren zur Herstellung einer Verbindung gemäß Anspruch 15 oder 16, dadurch gekennzeichnet, daß man

a) eine Verbindung der Formel XIV

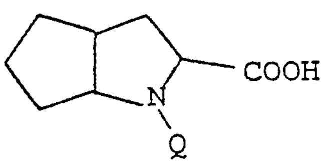

(XIV)

in der Q Wasserstoff oder $-CO-CH(CH_3)-NH_2$ bedeutet, mit n-Octanol oder $H_3C-[CH_2]_7-X$ analog Anspruch 14, Verfahrensvariante (a) oder (b) umsetzt oder

b) eine Verbindung der Formel XV

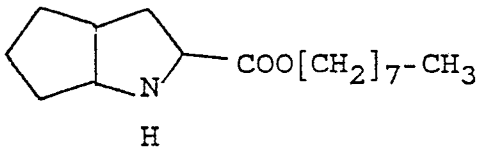 (XV)

analog Anspruch 14, Verfahrensvariante (f) mit einem aminogeschützten Alanin umsetzt und anschließend die Aminoschutzgruppe abspaltet und gewünschtenfalls das all-S-Isomer der Formel XII isoliert.

18. Verbindung gemäß einem der Anspruch 10 bis 13 zur Anwendung als Heilmittel.
19. Verbindung gemäß Anspruch 18 zur Anwendung als Nootropikum.
20. Verbindung gemäß Anspruch 18 zur Anwendung Psychotropikum, vorzugsweise Anxiolytikum.

**Patentansprüche für folgenden Vertragsstaat: ES**

1. Verwendung eines ACE-Inhibitors zur Herstellung eines Arzneimittels mit psychotroper Wirkung.
2. Verwendung eines ACE-Inhibitors zur Herstellung eines Arzneimittels mit anxiolytischer Wirkung.
3. Verwendung gemäß Anspruch 1 oder 2, dadurch gekennzeichnet, daß Angiotensin-Converting-Enzyme-Inhibitoren der Formel I

$X^1 - X^2$ (I)

in welcher

$X^1$ 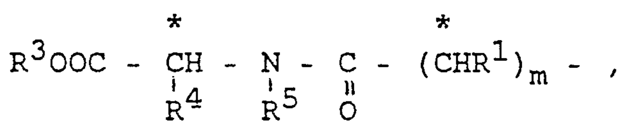

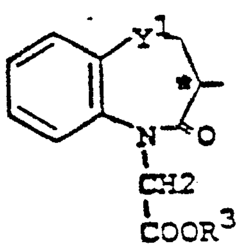 , 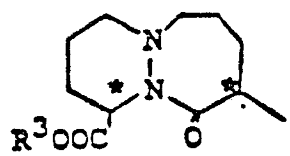 oder 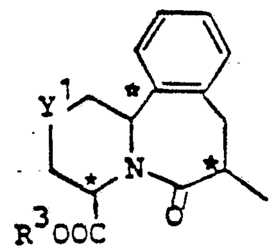 ,

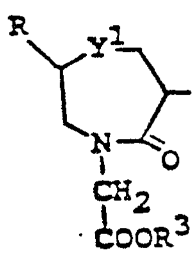 bedeutet,

$X^2$ $-CH_2SH$ , 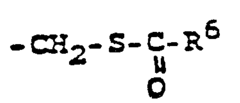 , 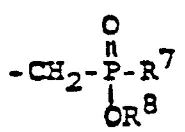 oder

$$-Y^2-(CH_2)_p-\overset{*}{C}H(COOR^2)-(CH_2)_n-R \quad \text{bedeutet,}$$

$Y^1$ für -S- oder $-CH_2-$ steht,
$Y^2$ für $-NR^9-$ oder $-CH_2-$ steht,
m = 0 oder 1 ist,
n = 0, 1 oder 2 ist,
p = 0 oder 1 ist,
R = Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclisch-aliphatischen Rest mit 4 - 20 C-Atomen,
einen gegebenenfalls substituierten heteroaromatischen oder heteroaromatisch-($C_1$-$C_8$)-aliphatischen Rest mit 5 - 12 Ringatomen, oder
einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder,
falls nicht von vorstehenden Definitionen schon umfaßt, die erforderlichenfalls geschützte Seitenkette einer natürlich vorkommenden α-Aminosäure bedeutet,
$R^2$ und $R^3$ gleich oder verschieden sind und
Wasserstoff, einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen Rest der Formel

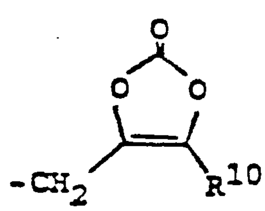

worin $R^{10}$ Wasserstoff, einen aliphatischen Rest mit 1-6 C-Atomen oder einen gegebenenfalls substituierten aromatischen Rest mit 6-12 C-Atomen bedeutet,
einen Rest der Formel

$$-CH_2-CH(OR^{11})-CH_2-OR^{12} \quad \text{oder} \quad -CH(CH_2-OR^{11})(CH_2-OR^{12})$$

worin $R^{11}$ und $R^{12}$ gleich oder verschieden unabhängig voneinander Wasserstoff, einen gegebenenfalls substituierten Alkylrest mit 1-23 C-Atomen oder einen gegebenenfalls substituierten Acylrest mit 1-23 C-Atomen bedeuten,
bedeuten,
$R^4$ für Wasserstoff oder $(C_1\text{-}C_6)$Alkyl und
$R^5$ für $(C_1\text{-}C_6)$Alkyl, $(C_3\text{-}C_6)$-Cycloalkyl oder

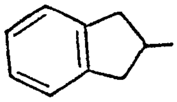 stehen oder

$R^4$ und $R^5$ zusammen mit den sie tragenden Atomen ein mono-, bi-, oder tricyclisches heterocyclisches Ringsystem mit 3 bis 15 Ring-C-Atomen bilden,
$R^6$ Wasserstoff, Amino, $(C_1\text{-}C_6)$-Alkyl, $(C_6\text{-}C_{12})$-Aryl oder $(C_7\text{-}C_{13})$-Aralkyl,
$R^7$ $(C_1\text{-}C_6)$-Alkyl oder $(C_7\text{-}C_{13})$-Aralkyl, vorzugsweise $-(CH_2)_4\text{-}C_6H_5$,
$R^8$ $(C_1\text{-}C_6)$-Alkyl, das gegebenenfalls durch $(C_1\text{-}C_6)$-Alkanoyloxy monosubstituiert ist, vorzugsweise 2-Methyl-1-propionyloxy-propyl, und
$R^9$ Wasserstoff oder $(C_1\text{-}C_6)$-Alkyl bedeuten;
oder deren physiologisch unbedenkliche Salze verwendet werden.

4. Verwendung gemäß Anspruch 1 oder 2, dadurch gekennzeichnet, daß Angiotensin-Converting-Enzyme-Inhibitoren der Formel II

$$R^3OOC - \overset{*}{\underset{R^4}{CH}} - \underset{R^5}{N} - \underset{O}{\overset{}{C}} - \overset{*}{\underset{R^1}{CH}} - NH - \overset{*}{\underset{COOR^2}{CH}} - (CH_2)_n - R \qquad (II)$$

in welcher
n = 1 oder 2 ist,
R = Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclisch-aliphatischen Rest mit 7 - 14 C-Atomen,
einen gegebenenfalls substituierten heteroaromatischen oder heteroaromatisch-$(C_1\text{-}C_8)$-aliphatischen Rest mit 5 - 12 Ringatomen, oder
einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-$(C_1\text{-}C_8)$-aliphatischen Rest mit 5 - 12 Ringatomen oder,
falls nicht von vorstehenden Definitionen schon umfaßt, die erforderlichenfalls geschützte Seitenkette einer natürlich vorkommenden α-Aminosäure bedeutet,
$R^2$ und $R^3$ gleich oder verschieden sind und
Wasserstoff, einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7-32 C-Atomen,
einen Rest der Formel

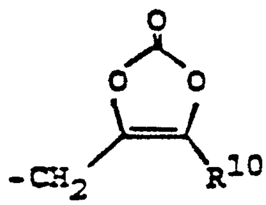

worin $R^{10}$ Wasserstoff, einen aliphatischen Rest mit 1-6 C-Atomen oder einen gegebenenfalls substituierten aromatischen Rest mit 6-12 C-Atomen bedeutet,
einen Rest der Formel

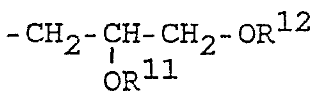

oder

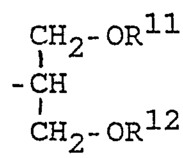

worin $R^{11}$ und $R^{12}$ gleich oder verschieden unabhängig voneinander Wasserstoff, einen gegebenenfalls substituierten Alkylrest mit 1-23 C-Atomen oder einen gegebenenfalls substituierten Acylrest mit 1-23 C-Atomen bedeuten,
bedeuten,
und
$R^4$ und $R^5$ zusammen mit den sie tragenden Atomen ein mono-, bi- oder tricyclisches heterocyclisches Ringsystem mit 3 bis 15 Ring-C-Atomen bilden,
oder deren physiologisch verträglichen Salze verwendet werden.

5. Verwendung gemäß einem oder mehreren der Ansprüche 1 bis 4, dadurch gekennzeichnet, daß Verbindungen der Formel I, vorzugsweise solche der Formel II verwendet werden, in welchen

a) n = 1 oder 2 ist;

b) R

1. Wasserstoff bedeutet;
2. Alkyl mit 1 - 18 C-Atomen bedeutet;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine ganze Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (C-2) stehen;
5. Aryl mit 6 - 12 C-Atomen bedeutet,
das durch $(C_1-C_8)$-Alkyl, $(C_1-C_4)$-Alkoxy, Hydroxy, Halogen, Nitro, Amino, Aminomethyl, $(C_1-C_4)$-Alkylamino, Di-$(C_1-C_4)$-alkylamino, $(C_1-C_4)$-Alkanoylamino, Methylendioxy, Carboxy, Cyano und/oder Sulfamoyl, mono-, di- oder trisubstituiert sein kann;
6. falls n = 2 ist, $(C_6-C_{12})$-Aryl-$(C_1-C_8)$-alkyl oder Di-$(C_6-C_{12})$-aryl-$(C_1-C_8)$-alkyl bedeutet,
die im Arylteil jeweils wie unter b)5. beschrieben substituiert sein können; oder
7. Alkoxy mit 1 - 4 C-Atomen;
8. Aryloxy mit 6 - 12 C-Atomen,
das wie unter b)5. beschrieben substituiert sein kann;
9. mono- bzw. bicyclisches Heteroaryloxy oder Heteroaryl-$(C_1-C_8)$-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen,
die im Heteroaryl wie unter b)5. beschrieben substituiert sein können;
10. Amino-$(C_1-C_8)$-alkyl;
11. $(C_1-C_4)$-Alkanoylamino-$(C_1-C_8)$-alkyl;
12. $(C_7-C_{13})$-Aroylamino-$(C_1-C_8)$-alkyl;
13. $(C_1-C_4)$-Alkoxy-carbonylamino-$(C_1-C_8)$-alkyl;
14. $(C_6-C_{12})$-Aryl-$(C_1-C_4)$-alkoxycarbonylamino-$(C_1-C_8)$-alkyl;
15. $(C_6-C_{12})$-Aryl-$(C_1-C_4)$-alkylamino-$(C_1-C_8)$-alkyl;
16. $(C_1-C_4)$-Alkylamino-$(C_1-C_8)$-alkyl;
17. Di-$(C_1-C_4)$-alkylamino-$(C_1-C_8)$-alkyl;
18. Guanidino-$(C_1-C_8)$-alkyl,

19. Imidazolyl;
20. Indolyl;
21. ($C_1$-$C_4$)-Alkylthio;
22. falls n = 2 ist, ($C_1$-$C_4$)-Alkylthio-($C_1$-$C_8$)-alkyl;
23. ($C_6$-$C_{12}$)-Arylthio-($C_1$-$C_8$)-alkyl;
das im Arylteil wie unter b) 5. beschrieben, substituiert sein kann,
24. ($C_6$-$C_{12}$)-Aryl-($C_1$-$C_8$)-alkylthio,
das im Arylteil wie unter b) 5. beschrieben substituiert sein kann;
25. falls n = 2 ist, Carboxy-($C_1$-$C_8$)-alkyl;
26. Carboxy;
27. Carbamoyl;
28. falls n = 2 ist, Carbamoyl-($C_1$-$C_8$)-alkyl;
29. ($C_1$-$C_4$-Alkoxy-carbonyl-($C_1$-$C_8$-alkyl;
30. falls n = 2 ist, ($C_6$-$C_{12}$)-Aryloxy-($C_1$-$C_8$)-alkyl,
das im Arylteil wie unter b) 5. beschrieben substituiert sein kann; oder
31. ($C_6$-$C_{12}$)-Aryl-($C_1$-$C_8$)-alkoxy,
das im Arylteil wie unter b) 5. beschrieben substituiert sein kann, bedeutet;

c) $R^1$
1. Wasserstoff bedeutet;
2. Alkyl mit 1 - 18 C-Atomen bedeutet;
3. einen aliphatischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;
5. Aryl mit 6 - 12 C-Atomen,
das wie unter b) 5. beschrieben substituiert sein kann ;
6. ($C_6$-$C_{12}$)-Aryl-($C_1$-$C_8$)-alkyl oder ($C_7$-$C_{13}$)-Aroyl-($C_1$-$C_8$)-alkyl,
die beide im Arylteil wie unter b) 5. beschrieben substituiert sein können;
7. mono- bzw. bicyclisches, gegebenenfalls teilhydriertes Heteroaryl oder Heteroaryl-($C_1$-$C_8$)-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen,
die im Heteroaryl wie bei Aryl unter b) 5. beschrieben substituiert sein können; oder
8. falls von c) 1. - 8. noch nicht umfaßt,
die gegebenenfalls geschützte Seitenkette einer natürlich vorkommenden α-Aminosäure der Formel $R^1$-CH($NH_2$)-COOH bedeutet;

d) $R^2$ und $R^3$ gleich oder verschieden sind und
1. Wasserstoff bedeutet;
2. Alkyl mit 1 - 18 C-Atomen;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedetuet, der gegebenen falls verzweigt ist, worin c für eine ganze Zahl 3 bis 20 und d für eine gerade Zahl 0 bis (c-2) stehen;
5. Di-($C_1$-$C_4$)-alkylamino-($C_1$-$C_8$)-alkyl;
6. ($C_1$-$C_5$)-Alkanoyloxy-($C_1$-$C_8$)-alkyl;
7. ($C_1$-$C_6$)-Alkoxy-carbonyloxy-($C_1$-$C_8$)-alkyl;
8. ($C_7$-$C_{13}$)-Aroyloxy-($C_1$-$C_8$)-alkyl;
9. $C_6$-$C_{12}$)-Aryloxycarbonyloxy($C_1$-$C_8$)-alkyl;
10. Aryl mit 6 - 12 C-Atomen;
11. ($C_7$-$C_{20}$)-Aralkyl;
12. Phthalidyl;
13. einen Rest der Formel

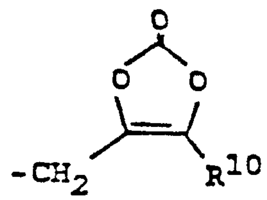

bedeutet,
worin $R^{10}$ Wasserstoff, $(C_1-C_6)$-Alkyl oder Aryl mit 6-12 C-Atomen ist,
14. einen Rest der Formel

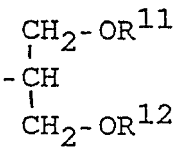

bedeutet, worin $R^{11}$ und $R^{12}$ gleich oder verschieden unabhängig voneinander Wasserstoff, einen gegebenenfalls substituierten Alkylrest mit 1-23 C-Atomen oder einen gegebenenfalls substituierten Acylrest mit 1-23 C-Atomen bedeuten,
wobei die unter d) 8., 9., 10. und 11. genannten Reste wie unter b) 5. beschreiben im Arylteil substituiert sein können; und
e) $R^4$ und $R^5$ zusammen mit den sie tragenden Atomen ein mono-, bi- oder tricyclisches heterocyclisches Ringsystem mit 3 bis 15 Ring-C-Atomen bilden.

6. Verwendung gemäß einem oder mehreren der Ansprüche 1 bis 5, dadurch gekennzeichnet, daß Verbindungen der Formel I, vorzugsweise solche der Formel II verwendet werden, in welchen
n = 1 oder 2 ist
R Wasserstoff,
Alkyl mit 1 - 8 C-Atomen,
Alkenyl mit 2 - 6 C-Atomen,
Cycloalkyl mit 3 - 9 C-Atomen,
Aryl mit 6 - 12 C-Atomen,
das durch $(C_1-C_4)$-Alkyl, $(C_1-C_4)$-Alkoxy, Hydroxy, Halogen, Nitro, Amino, Aminomethyl $(C_1-C_4)$-Alkylamino, Di-$(C_1-C_4)$-alkylamino, $(C_1-C_4)$-Alkanoylamino, Methylendioxy, Carboxy, Cyano und/oder Sulfamoyl, mono-, di- oder trisubstituiert sein kann,
Alkoxy mit 1 - 4 C-Atomen,
Aryloxy mit 6 - 12 C-Atomen,
das wie oben bei Aryl beschrieben substituiert sein kann,
mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen,
das wie oben bei Aryl beschrieben substituiert sein kann,
Amino-$(C_1-C_4)$-alkyl,
$(C_1-C_4)$-Alkanoylamino-$(C_1-C_4)$alkyl,
$(C_7-C_{13})$-Aroylamino-$(C_1-C_4)$-alkyl,
$(C_1-C_4)$-Alkoxy-carbonylamino-$(C_1-C_4)$-alkyl,
$(C_6-C_{12})$-Aryl-$(C_1-C_4)$-alkoxycarbonylamino-$(C_1-C_4)$-alkyl,
$(C_6-C_{12})$-Aryl-$(C_1-C_4)$-alkylamino-$(C_1-C_4)$-alkyl,
$(C_1-C_4)$-Alkylamino-$(C_1-C_4)$-alkyl,
Di-$(C_1-C_4)$-alkylamino-$(C_1-C_4)$-alkyl,
Guanidino-$(C_1-C_4)$-alkyl,
Imidazolyl, Indolyl,
$(C_1-C_4)$-Alkylthio,
$(C_1-C_4)$-Alkylthio-$(C_1-C_4)$-alkyl,
$(C_6-C_{12})$-Arylthio-$(C_1-C_4)$-alkyl,
das im Arylteil wie oben bei Aryl beschrieben, substituiert sein kann,
$(C_6-C_{12})$-Aryl-$(C_1-C_4$-alkylthio,
das im Arylteil wie oben bei Aryl beschrieben substituiert sein kann,
Carboxy-$(C_1-C_4)$-alkyl,
Carboxy, Carbamoyl,

Carbamoyl-($C_1$-$C_4$)-alkyl,
($C_1$-$C_4$)-Alkoxy-carbonyl-($C_1$-$C_4$)-alkyl,
($C_6$-$C_{12}$)-Aryloxy-($C_1$-$C_4$)-alkyl,
das im Arylteil wie oben bei Aryl beschrieben substituiert sein kann oder
($C_6$-$C_{12}$)-Aryl-($C_1$-$C_4$)-alkoxy,
das im Arylteil wie oben bei Aryl beschrieben substituiert sein kann,
$R^1$ Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
($C_3$-$C_9$)-Cycloalkyl-($C_1$-$C_4$)-alkyl,
($C_5$-$C_9$)-Cycloalkenyl-($C_1$-$C_4$)-alkyl,
gegebenenfalls teilhydriertes Aryl mit 6 - 12 C-Atomen, das wie oben bei R beschrieben substituiert sein kann,
($C_6$-$C_{12}$)-Aryl-($C_1$-$C_4$)-alkyl oder ($C_7$-$C_{13}$)-Aroyl-($C_1$ oder $C_2$)alkyl
die beide wie das vorstehende Aryl substituiert sein können
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann oder
die gegenbenenfalls geschützte Seitenkette einer natürliche vorkommenden α-Aminosäure $R^1$-CH($NH_2$)-COOH bedeuten,
$R^2$ und $R^3$ gleich oder verschieden sind und
Wasserstoff,
Alkyl mit 1 - 12 C-Atomen,
Alkenyl mit 2 - 12 C-Atomen,
Di-($C_1$-$C_4$)-alkylamino-($C_1$-$C_8$)-alkyl,
($C_1$-$C_5$)-Alkanoyloxy-($C_1$-$C_8$)-alkyl,
($C_1$-$C_6$)-Alkoxy-carbonyloxy-($C_1$-$C_8$)-alkyl,
($C_7$-$C_{13}$)-Aroyloxy-($C_1$-$C_8$)-alkyl,
($C_6$-$C_{12}$)-Aryloxycarbonyloxy($C_1$-$C_8$)-alkyl,
Aryl mit 6 - 12 C-Atomen,
($C_6$-$C_{12}$)-Aryl-($C_1$-$C_8$)-alkyl,
($C_3$-$C_9$)-Cycloalkyl oder
($C_3$-$C_9$)-Cycloalkyl-($C_1$-$C_8$)-alkyl
bedeuten und
$R^4$ und $R^5$ die oben angegebene Bedeutung haben.

7. Verwendung gemäß einem oder mehreren der Ansprüche 1 bis 6, dadurch gekennzeichnet, daß Verbindungen der Formel I, vorzugsweise solche der Formel II verwendet werden, in welchen
n= 1 oder 2 ist,
R ($C_1$-$C_6$)-Alkyl, ($C_2$-$C_6$)-Alkenyl, ($C_3$-$C_9$)-Cycloalkyl, Amino-($C_1$-$C_4$)-alkyl, ($C_2$-$C_5$)-Acylamino-($C_1$-$C_4$)-alkyl, ($C_7$-$C_{13}$)-Aroylamino-($C_1$-$C_4$)-alkyl, ($C_1$-$C_4$)-Alkoxycarbonylamino-($C_1$-$C_4$)-alkyl, ($C_6$-$C_{12}$)-Aryl-($C_1$-$C_4$)-alkoxycarbonylamino-($C_1$-$C_4$)-alkyl, ($C_6$-$C_{12}$)-Aryl, das durch ($C_1$-$C_4$)-Alkyl, ($C_1$-$C_4$)-Alkoxy, Hydroxy, Halogen, Nitro, Amino, ($C_1$-$C_4$)-Alkylamino, Di-($C_1$-$C_4$)-alkylamino und/oder Methylendioxy mono-, di- oder trisubstituiert sein kann, oder 3-Indolyl, insbesondere Methyl, Ethyl, Cyclohexyl, tert. Butoxycarbonylamino-($C_1$-$C_4$)-alkyl, Benzoyloxycarbonylamino-($C_1$-$C_4$)-alkyl oder Phenyl, das durch Phenyl, ($C_1$-$C_2$)-Alkyl, ($C_1$ oder $C_2$)-Alkoxy, Hydroxy, Fluor, Chlor, Brom, Amino, ($C_1$-$C_4$)-Alkylamino, Di-($C_1$-$C_4$)alkylamino, Nitro und/oder Methylendioxy mono- oder disubstituiert oder im Falle von Methoxy, trisubstituiert sein kann, bedeutet,
$R^1$ Wasserstoff oder ($C_1$-$C_6$)-Alkyl, das gegebenenfalls durch Amino, ($C_1$-$C_6$)-Acylamino oder Benzoylamino substituiert sein kann, ($C_2$-$C_6$)-Alkenyl, ($C_3$-$C_9$)-Cycloalkyl, ($C_5$-$C_9$)-Cycloalkenyl, ($C_3$-$C_7$)-Cycloalkyl-($C_1$-$C_4$)-alkyl, ($C_6$-$C_{12}$)-Aryl oder teilhydriertes Aryl, das jeweils durch ($C_1$-$C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)-alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,
$R^2$ und $R^3$ gleiche oder verschiedene Reste Wasserstoff, ($C_1$-$C_{12}$)-Alkyl, ($C_2$-$C_{12}$)-Alkenyl oder ($C_6$-$C_{12}$)-Aryl-($C_1$-$C_8$)-alkyl, bedeuten und
$R^4$ und $R^5$ die oben angegebene Bedeutung haben.

8. Verwendung gemäß einem oder mehreren der Ansprüche 1 bis 7, dadurch gekennzeichnet, daß Verbindungen der Formel II verwendet werden, worin $R^4$ und $R^5$ zusammen mit den sie tragenden Atomen ein mono-, bi- oder tricyclisches Ringsystem mit 3 bis 15 Ring-C-Atomen, bis zu 2 Ring-S-Atomen und bis zu 2 Ring-N-Atomen vorzugsweise aus der Reihe Pyrrolidin, Tetrahydroisochinolin, Decahydroisochinolin, Octahydroindol, Indolin, Octahydrocyclopenta[b]pyrrol, 2-Azaspiro[4.5]decan, 2-Azaspiro[4.4]nonan, Spiro[(bicyclo[2.2.1]heptan)-2,3′-pyrrolidin], Spiro[(bicyclo-[2.2.2]octan)-2,3′-pyrrolidin], 2-Azatricyclo[4.3.0.$1^{6,9}$]decan, Decahydrocyclohepta[b]pyrrol, Octahydroisoindol, Octahydrocyclopenta[c]pyrrol, 2,3,3a,4,5,7a-Hexahydroindol, 1,2,3,3a,4,6a-Hexahydrocyclopenta[b]pyrrol und 2-Azabicyclo[3.1.0]hexan bilden.

9. Verwendung in einem Verfahren zur Herstellung eines pharmazeutischen Mittels gemäß einem oder mehreren der Ansprüche 1 bis 8, dadurch gekennzeichnet, daß man den Wirkstoff mit den geeigneten Träger-, Hilfs- und/oder Zusatzstoffen in eine geeignete Darreichungsform bringt.

10. Verfahren zur Herstellung einer Verbindung der Formel III

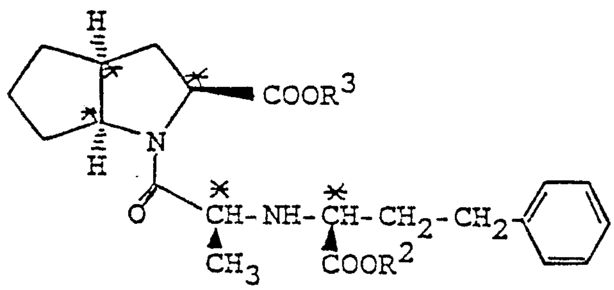

(III)

in welcher die fünf chiralen C-Atome (*) jeweils die S-Konfiguration aufweisen,
$R^2$ Ethyl oder Wasserstorff bedeutet und
$R^3$ n-Octyl bedeutet,
sowie deren physiologisch verträglichen Salzen, dadurch gekennzeichnet, daß man

a) eine Verbindung der Formel III, in welcher die fünf chiralen C-Atome (*) jeweils die S-Konfiguration aufweisen, $R^2$ Ethyl oder eine basisch, sauer oder hydrogenolytisch leicht abspaltbare Carboxyl-Schutzgruppe bedeutet und $R^3$ Wasserstoff bedeutet, mit n-Octanol umsetzt,
oder daß man

b) eine Verbindung der Formel III, in der die Konfiguration sowie $R^2$ und $R^3$ wie oben unter (a) definiert sind, mit einer Verbindung der Formel VI
$H_3C$-[$CH_2$-]$_7$X (VI)
in der X eine Abgangsgruppe, die nucleophil verdrängt werden kann, bedeutet, unter Bedingungen einer nucleophilen Substitution umsetzt,
oder daß man

c) eine Verbindung der Formel III, die wie oben unter (a) konfiguriert ist und in welcher $R^2$ Wasserstoff bedeutet und $R^3$ n-Octyl bedeutet, mit Ethanol wie unter Verfahrensvariante (a) beschrieben, umsetzt,
oder daß man

d) eine Verbindung der Formel III, in der die Konfiguration, $R^2$ und $R^3$ wie oben unter (c) definiert sind, mit einer Verbindung der Formel VII
$H_3C-CH_2-X$ (VII)
in der X eine Abgangsgruppe, die nucleophil verdrängt werden kann, bedeutet, wie unter Verfahrensvariante (b) beschrieben, umsetzt,
oder daß man

e) eine Verbindung der Formel VIII

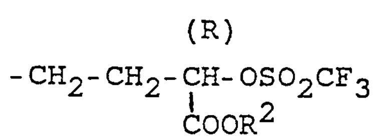

(VIII)

in der $R^2$ wie oben unter (a) definiert ist, umsetzt mit einer Verbindung der Formel IX

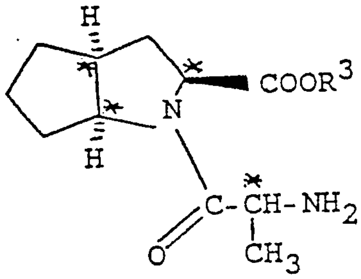

(IX)

in welcher die vier chiralen C-Atome (*) jeweils die S-Konfiguration aufweisen, oder daß man

f) eine Verbindung der Formel X

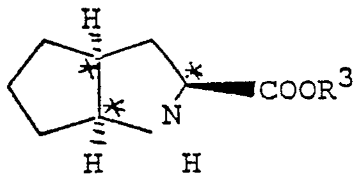

(X)

in welcher die drei chiralen C-Atome (*) jeweils die S-Konfiguration aufweisen und $R^3$ n-Octyl bedeutet, umsetzt mit einer Verbindung der Formel XI

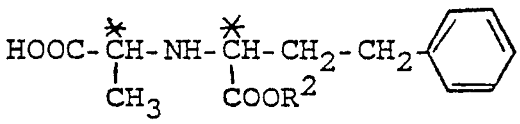

(XI)

in welcher $R^2$ wie oben unter (a) definiert ist, erforderlichenfalls in einer erhaltenen Verbindung der Formel III ($R^2$ = Schutzgruppe) die Schutzgruppe $R^2$ in an sich bekannter Weise abspaltet und die auf diese Weise erhaltenen Verbindung der Formel III ($R^2$ = Ethyl oder Wasserstoff) gegebenenfalls in ihr physiologisch verträgliches Salz überführt,

wobei, falls als Ausgangsmaterialien der Verfahrensvarianten (a)-(f) Stereoisomerengemische eingesetzt werden, daran anschließend in einer weiteren Trennstufe das all-S-Isomere der Formel III ($R^2$ = Wasserstoff oder Ethyl, $R^3$ = n-Octyl) abgetrennt wird.

11. Verfahren gemäß Anspruch 10, dadurch gekennzeichnet, daß eine Verbindung der Formel III hergestellt wird, worin $R^2$ Ethyl bedeutet, oder deren physiologisch verträglichen Salze.

12. Verfahren gemäß Anspruch 10, dadurch gekennzeichnet, daß eine Verbindung der Formel III hergestellt wird, worin $R^2$ Wasserstoff bedeutet, oder deren physiologisch verträglichen Salze.

13. Verfahren gemäß Anspruch 11, dadurch gekennzeichnet, daß ein Maleinat hergestellt wird.

14. Verfahren zur Herstellung einer Verbindung der Formel XII

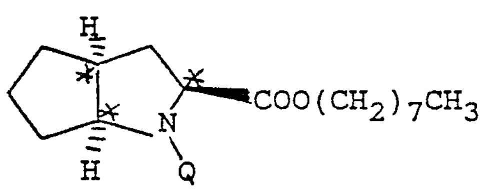

(XII)

in welcher die drei chiralen C-Atome jeweils die S-Konfiguration aufweisen und Q Wasserstoff oder den Rest XIII

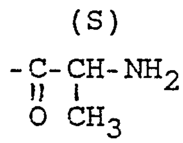

(XIII)

bedeutet, dadurch gekennzeichnet, daß man

a) eine Verbindung der Formel XIV

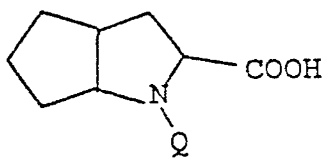

(XIV)

in der Q Wasserstoff oder $-CO-CH(CH_3)-NH_2$ bedeutet, mit n-Octanol oder $H_3C-[CH_2]_7-X$ analog Anspruch 10, Verfahrensvariante (a) oder (b) umsetzt oder

b) eine Verbindung der Formel XV

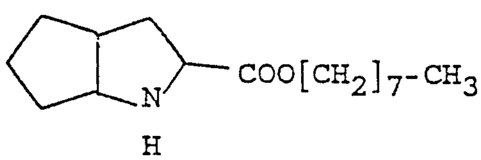

(XV)

analog Anspruch 10, Verfahrensvariante (f) mit einem aminogeschützten Alanin umsetzt und anschließend die Aminoschutzgruppe abspaltet und gewünschtenfalls das all-S-Isomer der Formel XII isoliert.

15. Verfahren gemäß Anspruch 14, dadurch gekennzeichnet, daß ausgehend von (einem) Stereoisomerengemisch(en) der Verbindung XIV oder XV ein Stereoisomerengemisch enthaltend eine Verbindung der Formel XII hergestellt wird.

**Patentansprüche für folgenden Vertragsstaat: GR**

1. Verwendung eines ACE-Inhibitors zur Herstellung eines Arzneimittels mit psychotroper Wirkung.
2. Verwendung eines ACE-Inhibitors zur Herstellung eines Arzniemittels mit anxiolytischer Wirkung.
3. Verwendung gemäß Anspruch 1 oder 2, dadurch gekennzeichnet, daß Angiotensin-Converting-Enzyme-Inhibitoren der Formel I

$X^1 - X^2$ (I)

in welcher

$X^1$ $R^3OOC - \overset{*}{C}H(R^4) - N(R^5) - \overset{*}{C}(=O) - (CHR^1)_m -$ ,

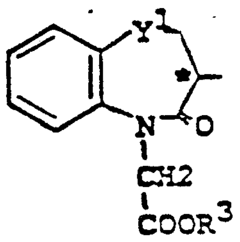 ,

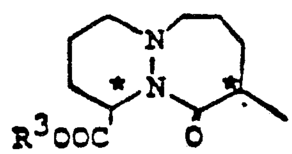

oder

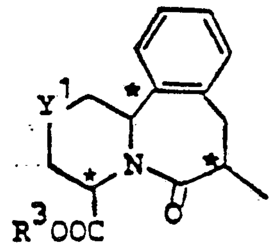 ,

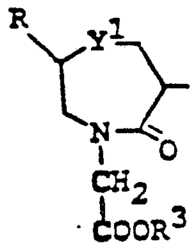

bedeutet,

$X^2$ $-CH_2SH$ , $-CH_2-S-C(=O)-R^6$ , $-CH_2-P(=O)(OR^8)-R^7$ oder

$-Y^2-(CH_2)_p-\overset{*}{C}H(COOR^2)-(CH_2)_n-R$ bedeutet,

$Y^1$ für -S- oder $-CH_2-$ steht,
$Y^2$ für $-NR^9-$ oder $-CH_2-$ steht,
m = 0 oder 1 ist,
n = 0, 1 oder 2 ist,
p = 0 oder 1 ist,
R = Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclisch-aliphatischen Rest mit 4 - 20 C-Atomen,
einen gegebenenfalls substituierten heteroaromatischen oder heteroaromatisch-($C_1$-$C_8$)-aliphatischen Rest mit 5 - 12 Ringatomen, oder
einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,

einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder,
falls nicht von vorstehenden Definitionen schon umfaßt, die erforderlichenfalls geschützte Seitenkette einer natürlich vorkommenden α-Aminosäure bedeutet,
$R^2$ und $R^3$ gleich oder verschieden sind und
Wasserstoff, einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen Rest der Formel

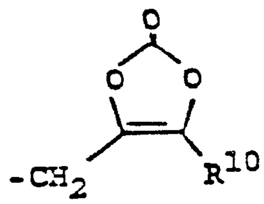

worin $R^{10}$ Wasserstoff, einen aliphatischen Rest mit 1-6 C-Atomen oder einen gegebenenfalls substituierten aromatischen Rest mit 6-12 C-Atomen bedeutet,
einen Rest der Formel

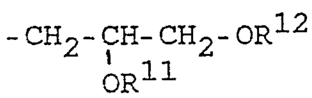
oder
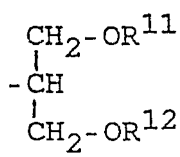

worin $R^{11}$ und $R^{12}$ gleich oder verschieden unabhängig voneinander Wasserstoff, einen gegebenenfalls substituierten Alkylrest mit 1-23 C-Atomen oder einen gegebenenfalls substituierten Acylrest mit 1-23 C-Atomen bedeuten,
bedeuten,
$R^4$ für Wasserstoff oder ($C_1$-$C_6$)Alkyl und
$R^5$ für ($C_1$-$C_6$)Alkyl, ($C_3$-$C_6$)-Cycloalkyl oder

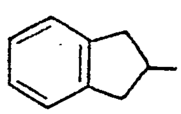
stehen oder

$R^4$ und $R^5$ zusammen mit den sie tragenden Atomen ein mono-, bi-, oder tricyclisches heterocyclisches Ringsystem mit 3 bis 15 Ring-C-Atomen bilden,
$R^6$ Wasserstoff, Amino, ($C_1$-$C_6$)-Alkyl, ($C_6$-$C_{12}$)-Aryl oder ($C_7$-$C_{13}$)-Aralkyl,
$R^7$ ($C_1$-$C_6$)-Alkyl oder ($C_7$-$C_{13}$)-Aralkyl, vorzugsweise - $(CH_2)_4$-$C_6H_5$,
$R^8$ ($C_1$-$C_6$)-Alkyl, das gegebenenfalls durch ($C_1$-$C_6$)-Alkanoyloxy monosubstituiert ist, vorzugsweise 2-Methyl-1-propionyloxy-propyl, und
$R^9$ Wasserstoff oder ($C_1$-$C_6$)-Alkyl bedeuten;
oder deren physiologisch unbedenkliche Salze verwendet werden.

4. Verwendung gemäß Anspruch 1 oder 2, dadurch gekennzeichnet, daß Angiotensin-Converting-Enzyme-Inhibitoren der Formel II

$$R^3OOC-\overset{*}{C}H(R^4)-N(R^5)-C(=O)-\overset{*}{C}H(R^1)-NH-\overset{*}{C}H(COOR^2)-(CH_2)_n-R \qquad (II)$$

in welcher
$n = 1$ oder 2 ist,
R = Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclisch-aliphatischen Rest mit 7 - 14 C-Atomen,
einen gegebenenfalls substituierten heteroaromatischen oder heteroaromatisch-($C_1$-$C_8$)-aliphatischen Rest mit 5 - 12 Ringatomen, oder
einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder,
falls nicht von vorstehenden Definitionen schon umfaßt, die erforderlichenfalls geschützte Seitenkette einer natürlich vorkommenden α-Aminosäure bedeutet,
$R^2$ und $R^3$ gleich oder verschieden sind und
Wasserstoff, einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7-32 C-Atomen,
einen Rest der Formel

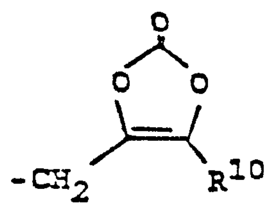

worin $R^{10}$ Wasserstoff, einen aliphatischen Rest mit 1-6 C-Atomen oder einen gegebenenfalls substituierten aromatischen Rest mit 6-12 C-Atomen bedeutet,
einen Rest der Formel

$$-CH_2-CH(OR^{11})-CH_2-OR^{12} \quad \text{oder} \quad -CH(CH_2-OR^{11})(CH_2-OR^{12})$$

worin $R^{11}$ und $R^{12}$ gleich oder verschieden unabhängig voneinander Wasserstoff, einen gegebenenfalls substituierten Alkylrest mit 1-23 C-Atomen oder einen gegebenenfalls substituierten Acylrest mit 1-23 C-Atomen bedeuten,

bedeuten,
und
$R^4$ und $R^5$ zusammen mit den sie tragenden Atomen ein mono-, bi- oder tricyclisches heterocyclisches Ringsystem mit 3 bis 15 Ring-C-Atomen bilden,
oder deren physiologisch verträglichen Salze verwendet werden.

5. Verwendung gemäß einem oder mehreren der Ansprüche 1 bis 4, dadurch gekennzeichnet, daß Verbindungen der Formel I, vorzugsweise solche der Formel II verwendet werden, in welchen

a) n= 1 oder 2 ist;
b) R
1. Wasserstoff bedeutet;
2. Alkyl mit 1 - 18 C-Atomen bedeutet;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine ganze Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (C-2) stehen;
5. Aryl mit 6 - 12 C-Atomen bedeutet,
das durch ($C_1$-$C_8$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl, mono-, di- oder trisubstituiert sein kann;
6. falls n= 2 ist, ($C_6$-$C_{12}$)-Aryl-($C_1$-$C_8$)-alkyl oder Di-($C_6$-$C_{12}$)-aryl-($C_1$-$C_8$)-alkyl bedeutet,
die im Arylteil jeweils wie unter b)5. beschrieben substituiert sein können; oder
7. Alkoxy mit 1 - 4 C-Atomen;
8. Aryloxy mit 6 - 12 C-Atomen,
das wie unter b)5. beschrieben substituiert sein kann;
9. mono- bzw. bicyclisches Heteroaryloxy oder Heteroaryl-($C_1$-$C_8$)-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen,
die im Heteroaryl wie unter b)5. beschrieben substituiert sein können;
10. Amino-($C_1$-$C_8$)-alkyl;
11. ($C_1$-$C_4$)-Alkanoylamino-($C_1$-$C_8$)-alkyl;
12. ($C_7$-$C_{13}$)-Aroylamino-($C_1$-$C_8$)-alkyl;
13. ($C_1$-$C_4$)-Alkoxy-carbonylamino-($C_1$-$C_8$)-alkyl;
14. ($C_6$-$C_{12}$)-Aryl-($C_1$-$C_4$)-alkoxycarbonylamino-($C_1$-$C_8$)-alkyl;
15. ($C_6$-$C_{12}$)-Aryl-($C_1$-$C_4$)-alkylamino-($C_1$-$C_8$)-alkyl;
16. ($C_1$-$C_4$)-Alkylamino-($C_1$-$C_8$)-alkyl;
17. Di-($C_1$-$C_4$)-alkylamino-($C_1$- $C_8$)-alkyl;
18. Guanidino-($C_1$-$C_8$)-alkyl,
19. Imidazolyl;
20. Indolyl;
21. ($C_1$-$C_4$)-Alkylthio;
22. falls n= 2 ist, ($C_1$-$C_4$)-Alkylthio-($C_1$-$C_8$)-alkyl;
23. ($C_6$-$C_{12}$)-Arylthio-($C_1$-$C_8$)-alkyl;
das im Arylteil wie unter b) 5. beschrieben, substituiert sein kann,
24. ($C_6$-$C_{12}$)-Aryl-($C_1$-$C_8$)-alkylthio,
das im Arylteil wie unter b) 5. beschrieben substituiert sein kann;
25. falls n= 2 ist, Carboxy-($C_1$-$C_8$)-alkyl;
26. Carboxy;
27. Carbamoyl;
28. falls n= 2 ist, Carbamoyl-($C_1$-$C_8$)-alkyl;
29. ($C_1$-$C_4$-Alkoxy-carbonyl-($C_1$-$C_8$-alkyl;
30. falls n= 2 ist, ($C_6$-$C_{12}$)-Aryloxy-($C_1$-$C_8$)-alkyl,
das im Arylteil wie unter b) 5. beschrieben substituiert sein kann; oder
31. ($C_6$-$C_{12}$)-Aryl-($C_1$-$C_8$)-alkoxy,
das im Arylteil wie unter b) 5. beschrieben substituiert sein kann, bedeutet;
c) $R^1$
1. Wasserstoff bedeutet;
2. Alkyl mit 1 - 18 C-Atomen bedeutet;
3. einen aliphatischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;

5. Aryl mit 6 - 12 C-Atomen,
das wie unter b) 5. beschrieben substituiert sein kann ;
6. ($C_6$-$C_{12}$)-Aryl-($C_1$-$C_8$)-alkyl oder ($C_7$-$C_{13}$)-Aroyl-($C_1$-$C_8$)-alkyl,
die beide im Arylteil wie unter b) 5. beschrieben substituiert sein können;
7. mono- bzw. bicyclisches, gegebenenfalls teilhydriertes Heteroaryl oder Heteroaryl-($C_1$-$C_8$)-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen,
die im Heteroaryl wie bei Aryl unter b) 5. beschrieben substituiert sein können; oder
8. falls von c) 1. - 8. noch nicht umfaßt,
die gegebenenfalls geschützte Seitenkette einer natürlich vorkommenden α-Aminosäure der Formel $R^1$-$CH(NH_2)$-COOH bedeutet;
d) $R^2$ und $R^3$ gleich oder verschieden sind und
1. Wasserstoff bedeutet;
2. Alkyl mit 1 - 18 C-Atomen;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedetuet, der gegebenen falls verzweigt ist, worin c für eine ganze Zahl 3 bis 20 und d für eine gerade Zahl 0 bis (c-2) stehen;
5. Di-($C_1$-$C_4$)-alkylamino-($C_1$-$C_8$)-alkyl;
6. ($C_1$-$C_5$)-Alkanoyloxy-($C_1$-$C_8$)-alkyl;
7. ($C_1$-$C_6$)-Alkoxy-carbonyloxy-($C_1$-$C_8$)-alkyl;
8. ($C_7$-$C_{13}$)-Aroyloxy-($C_1$-$C_8$)-alkyl;
9. $C_6$-$C_{12}$)-Aryloxycarbonyloxy($C_1$-$C_8$)-alkyl;
10. Aryl mit 6 - 12 C-Atomen;
11. ($C_7$-$C_{20}$)-Aralkyl;
12. Phthalidyl;
13. einen Rest der Formel

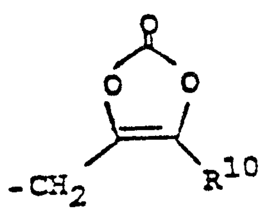

bedeutet,
worin $R^{10}$ Wasserstoff, ($C_1$-$C_6$)-Alkyl oder Aryl mit 6-12 C-Atomen ist,
14. einen Rest der Formel

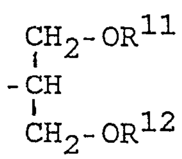

bedeutet, worin $R^{11}$ und $R^{12}$ gleich oder verschieden unabhängig voneinander Wasserstoff, einen gegebenenfalls substituierten Alkylrest mit 1-23 C-Atomen oder einen gegebenenfalls substituierten Acylrest mit 1-23 C-Atomen bedeuten,
wobei die unter d) 8., 9., 10. und 11. genannten Reste wie unter b) 5. beschreiben im Arylteil substituiert sein können; und
e) $R^4$ und $R^5$ zusammen mit den sie tragenden Atomen ein mono-, bi- oder tricyclisches heterocyclisches Ringsystem mit 3 bis 15 Ring-C-Atomen bilden.

6. Verwendung gemäß einem oder mehreren der Ansprüche 1 bis 5, dadurch gekennzeichnet, daß Verbindungen der Formel I, vorzugsweise solche der Formel II verwendet werden, in welchen
n = 1 oder 2 ist
R Wasserstoff,
Alkyl mit 1 - 8 C-Atomen,

Alkenyl mit 2 - 6 C-Atomen,
Cycloalkyl mit 3 - 9 C-Atomen,
Aryl mit 6 - 12 C-Atomen,
das durch ($C_1$-$C_4$)-Alkyl, ($C_1$-$C_4$)-Alkoxy, Hydroxy, Halogen, Nitro, Amino, Aminomethyl ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl, mono-, di- oder trisubstituiert sein kann,
Alkoxy mit 1 - 4 C-Atomen,
Aryloxy mit 6 - 12 C-Atomen,
das wie oben bei Aryl beschrieben substituiert sein kann,
mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen,
das wie oben bei Aryl beschrieben substituiert sein kann,
Amino-($C_1$-$C_4$)-alkyl,
($C_1$-$C_4$)-Alkanoylamino-($C_1$-$C_4$)alkyl,
($C_7$-$C_{13}$)-Aroylamino-($C_1$-$C_4$)-alkyl,
($C_1$-$C_4$)-Alkoxy-carbonylamino-($C_1$-$C_4$)-alkyl,
($C_6$-$C_{12}$)-Aryl-($C_1$-$C_4$)-alkoxycarbonylamino-($C_1$-$C_4$)-alkyl,
($C_6$-$C_{12}$)-Aryl-($C_1$-$C_4$)-alkylamino-($C_1$-$C_4$)-alkyl,
($C_1$-$C_4$)-Alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,
Imidazolyl, Indolyl,
($C_1$-$C_4$)-Alkylthio,
($C_1$-$C_4$)-Alkylthio-($C_1$-$C_4$)-alkyl,
($C_6$-$C_{12}$)-Arylthio-($C_1$-$C_4$)-alkyl,
das im Arylteil wie oben bei Aryl beschrieben, substituiert sein kann,
($C_6$-$C_{12}$)-Aryl-($C_1$-$C_4$-alkylthio,
das im Arylteil wie oben bei Aryl beschrieben substituiert sein kann,
Carboxy-($C_1$-$C_4$)-alkyl,
Carboxy, Carbamoyl,
Carbamoyl-($C_1$-$C_4$)-alkyl,
($C_1$-$C_4$)-Alkoxy-carbonyl-($C_1$-$C_4$)-alkyl,
($C_6$-$C_{12}$)-Aryloxy-($C_1$-$C_4$)-alkyl,
das im Arylteil wie oben bei Aryl beschrieben substituiert sein kann oder
($C_6$-$C_{12}$)-Aryl-($C_1$-$C_4$)-alkoxy,
das im Arylteil wie oben bei Aryl beschrieben substituiert sein kann,
$R^1$ Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
($C_3$-$C_9$)-Cycloalkyl-($C_1$-$C_4$)-alkyl,
($C_5$-$C_9$)-Cycloalkenyl-($C_1$-$C_4$)-alkyl,
gegebenenfalls teilhydriertes Aryl mit 6 - 12 C-Atomen, das wie oben bei R beschrieben substituiert sein kann,
($C_6$-$C_{12}$)-Aryl-($C_1$-$C_4$)-alkyl oder ($C_7$-$C_{13}$)-Aroyl-($C_1$ oder $C_2$)alkyl
die beide wie das vorstehende Aryl substituiert sein können
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann oder
die gegenbenenfalls geschützte Seitenkette einer natürliche vorkommenden $\alpha$-Aminosäure $R^1$-CH($NH_2$)-COOH bedeuten,
$R^2$ und $R^3$ gleich oder verschieden sind und
Wasserstoff,
Alkyl mit 1 - 12 C-Atomen,
Alkenyl mit 2 - 12 C-Atomen,
Di-($C_1$-$C_4$)-alkylamino-($C_1$-$C_8$)-alkyl,
($C_1$-$C_5$)-Alkanoyloxy-($C_1$-$C_8$)-alkyl,
($C_1$-$C_6$)-Alkoxy-carbonyloxy-($C_1$-$C_8$)-alkyl,
($C_7$-$C_{13}$)-Aroyloxy-($C_1$-$C_8$)-alkyl,
($C_6$-$C_{12}$)-Aryloxycarbonyloxy($C_1$-$C_8$)-alkyl,
Aryl mit 6 - 12 C-Atomen,
($C_6$-$C_{12}$)-Aryl-($C_1$-$C_8$)-alkyl,

($C_3$-$C_9$)-Cycloalkyl oder
($C_3$-$C_9$)-Cycloalkyl-($C_1$-$C_8$)-alkyl
bedeuten und
$R^4$ und $R^5$ die oben angegebene Bedeutung haben.

7. Verwendung gemäß einem oder mehreren der Ansprüche 1 bis 6, dadurch gekennzeichnet, daß Verbindungen der Formel I, vorzugsweise solche der Formel II verwendet werden, in welchen
n = 1 oder 2 ist,
R ($C_1$-$C_6$)-Alkyl, ($C_2$-$C_6$)-Alkenyl, ($C_3$-$C_9$)-Cycloalkyl, Amino-($C_1$-$C_4$)-alkyl, ($C_2$-$C_5$)-Acylamino-($C_1$-$C_4$)-alkyl, ($C_7$-$C_{13}$)-Aroylamino-($C_1$-$C_4$)-alkyl, ($C_1$-$C_4$)-Alkoxycarbonylamino-($C_1$-$C_4$)-alkyl, ($C_6$-$C_{12}$)-Aryl-($C_1$-$C_4$)-alkoxycarbonylamino-($C_1$-$C_4$)-alkyl, ($C_6$-$C_{12}$)-Aryl, das durch ($C_1$-$C_4$)-Alkyl, ($C_1$-$C_4$)-Alkoxy, Hydroxy, Halogen, Nitro, Amino, ($C_1$-$C_4$)-Alkylamino, Di-($C_1$-$C_4$)-alkylamino und/oder Methylendioxy mono-, di- oder trisubstituiert sein kann, oder 3-Indolyl, insbesondere Methyl, Ethyl, Cyclohexyl, tert. Butoxycarbonylamino-($C_1$-$C_4$)-alkyl, Benzoyloxycarbonylamino-($C_1$-$C_4$)-alkyl oder Phenyl, das durch Phenyl, ($C_1$-$C_2$)-Alkyl, ($C_1$ oder $C_2$)-Alkoxy, Hydroxy, Fluor, Chlor, Brom, Amino, ($C_1$-$C_4$)-Alkylamino, Di-($C_1$-$C_4$)alkylamino, Nitro und/oder Methylendioxy mono- oder disubstituiert oder im Falle von Methoxy, trisubstituiert sein kann, bedeutet,
$R^1$ Wasserstoff oder ($C_1$-$C_6$)-Alkyl, das gegebenenfalls durch Amino, ($C_1$-$C_6$)-Acylamino oder Benzoylamino substituiert sein kann, ($C_2$-$C_6$)-Alkenyl, ($C_3$-$C_9$)-Cycloalkyl, ($C_5$-$C_9$)-Cycloalkenyl, ($C_3$-$C_7$)-Cycloalkyl-($C_1$-$C_4$)-alkyl, ($C_6$-$C_{12}$)-Aryl oder teilhydriertes Aryl, das jeweils durch ($C_1$-$C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)-alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,
$R^2$ und $R^3$ gleiche oder verschiedene Reste Wasserstoff, ($C_1$-$C_{12}$)-Alkyl, ($C_2$-$C_{12}$)-Alkenyl oder ($C_6$-$C_{12}$)-Aryl-($C_1$-$C_8$)-alkyl, bedeuten und
$R^4$ und $R^5$ die oben angegebene Bedeutung haben.

8. Verwendung gemäß einem oder mehreren der Ansprüche 1 bis 7, dadurch gekennzeichnet, daß Verbindungen der Formel II verwendet werden, worin $R^4$ und $R^5$ zusammen mit den sie tragenden Atomen ein mono-, bi- oder tricyclisches Ringsystem mit 3 bis 15 Ring-C-Atomen, bis zu 2 Ring-S-Atomen und bis zu 2 Ring-N-Atomen vorzugsweise aus der Reihe Pyrrolidin, Tetrahydroisochinolin, Decahydroisochinolin, Octahydroindol, Indolin, Octahydrocyclopenta[b]pyrrol, 2-Azaspiro[4.5]decan, 2-Azaspiro[4.4]nonan, Spiro[(bicyclo[2.2.1]heptan)-2,3'-pyrrolidin], Spiro[(bicyclo-[2.2.2]octan)-2,3'-pyrrolidin], 2-Azatricyclo[4.3.0.1$^{6,9}$]decan, Decahydrocyclohepta[b]pyrrol, Octahydroisoindol, Octahydrocyclopenta[c]pyrrol, 2,3,3a,4,5,7a-Hexahydroindol, 1,2,3,3a,4,6a-Hexahydrocyclopenta[b]pyrrol und 2-Azabicyclo[3.1.0]hexan bilden.

9. Verwendung in einem Verfahren zur Herstellung eines pharmazeutischen Mittels gemäß einem oder mehreren der Ansprüche 1 bis 8, dadurch gekennzeichnet, daß man den Wirkstoff mit den geeigneten Träger-, Hilfs- und/oder Zusatzstoffen in eine geeignete Darreichungsform bringt.

10. Verfahren zur Herstellung einer Verbindung der Formel III

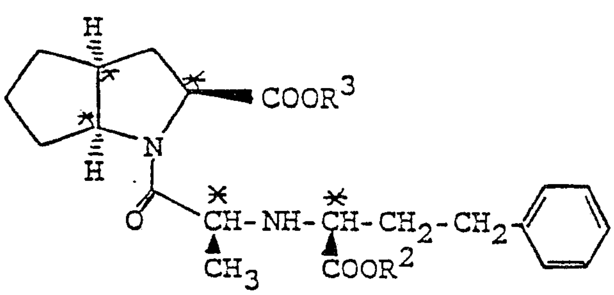

(III)

in welcher die fünf chiralen C-Atome (*) jeweils die S-Konfiguration aufweisen,
$R^2$ Ethyl oder Wasserstorff bedeutet und
$R^3$ n-Octyl bedeutet,
sowie deren physiologisch verträglichen Salzen, dadurch gekennzeichnet, daß man

a) eine Verbindung der Formel III, in welcher die fünf chiralen C-Atome (*) jeweils die S-Konfiguration aufweisen, $R^2$ Ethyl oder eine basisch, sauer oder hydrogenolytisch leicht abspaltbare Carboxyl-Schutzgruppe bedeutet und $R^3$ Wasserstoff bedeutet, mit n-Octanol umsetzt, oder daß man

b) eine Verbindung der Formel III, in der die Konfiguration sowie $R^2$ und $R^3$ wie oben unter (a) definiert sind, mit einer Verbindung der Formel VI

$H_3C-[CH_2-]_7X$ (VI)

in der X eine Abgangsgruppe, die nucleophil verdrängt werden kann, bedeutet, unter Bedingungen einer nucleophilen Substitution umsetzt,

oder daß man

c) eine Verbindung der Formel III, die w oben unter (a) konfiguriert ist und in welcher $R^2$ Wasserstoff bedeutet und $R^3$ n-Octyl bedeutet, mit Ethanol wie unter Verfahrensvariante (a) beschrieben, umsetzt,

oder daß man

d) eine Verbindung der Formel III, in der die Konfiguration, $R^2$ und $R^3$ wie oben unter (c) definiert sind, mit einer Verbindung der Formel VII

$H_3C-CH_2-X$ (VII)

in der X eine Abgangsgruppe, die nucleophil verdrängt werden kann, bedeutet, wie unter Verfahrensvariante (b) beschrieben, umsetzt,

oder daß man

e) eine Verbindung der Formel VIII

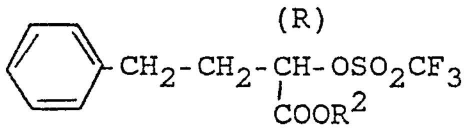

(VIII)

in der $R^2$ wie oben unter (a) definiert ist, umsetzt mit einer Verbindung der Formel IX

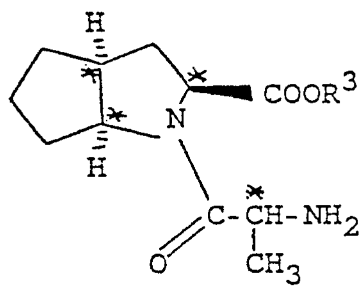

(IX)

in welcher die vier chiralen C-Atome (*) jeweils die S-Konfiguration aufweisen,

oder daß man

f) eine Verbindung der Formel X

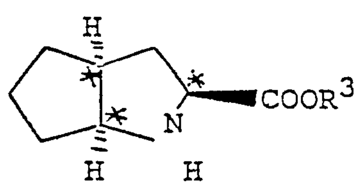

(X)

in welcher die drei chiralen C-Atome (*) jeweils die S-Konfiguration aufweisen und $R^3$ n-Octyl bedeutet, umsetzt mit einer Verbindung der Formel XI

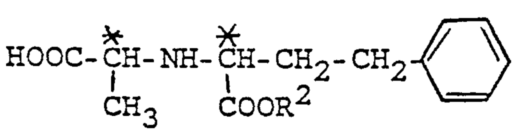

(XI)

in welcher $R^2$ wie oben unter (a) definiert ist, erforderlichenfalls in einer erhaltenen Verbindung der Formel III ($R^2$ = Schutzgruppe) die Schutzgruppe $R^2$ in an sich bekannter Weise abspaltet und die auf diese Weise erhaltenen Verbindung der Formel III ($R^2$ = Ethyl oder Wasserstoff) gegebenenfalls in ihr physiologisch verträgliches Salz überführt,
wobei, falls als Ausgangsmaterialien der Verfahrensvarianten (a)-(f) Stereoisomerengemische eingesetzt werden, daran anschließend in einer weiteren Trennstufe das all-S-Isomere der Formel III ($R^2$ = Wasserstoff oder Ethyl, $R^3$ = n-Octyl) abgetrennt wird.

11. Verfahren gemäß Anspruch 10, dadurch gekennzeichnet, daß eine Verbindung der Formel III hergestellt wird, worin $R^2$ Ethyl bedeutet, sowie deren physiologisch verträglichen Salze.

12. Verfahren gemäß Anspruch 10, dadurch gekennzeichnet, daß eine Verbindung der Formel III hergestellt wird, worin $R^2$ Wasserstoff bedeutet, oder deren physiologisch verträglichen Salze.

13. Verfahren gemäß Anspruch 11, dadurch gekennzeichnet, daß ein Maleinat hergestellt wird.

14. Verbindung der Formel XII

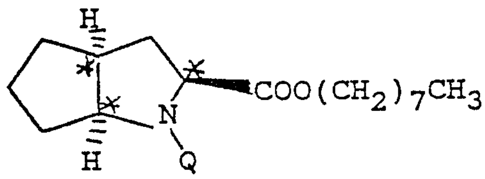

(XII)

in welcher die drei chiralen C-Atome jeweils die S-Konfiguration aufweisen und Q Wasserstoff oder den Rest XIII

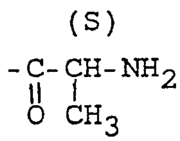

(XIII)

bedeutet.

15. Stereoisomerengemisch, enthaltend eine Verbindung gemäß Anspruch 14.

16. Verfahren zur Herstellung einer Verbindung gemäß Anspruch 14 oder 15, dadurch gekennzeichnet, daß man

a) eine Verbindung der Formel XIV

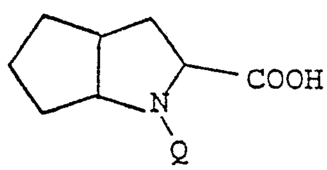

(XIV)

in der Q Wasserstoff oder $-CO-CH(CH_3)-NH_2$ bedeutet, mit n-Octanol oder $H_3C-[CH_2]_7-X$ analog Anspruch 10, Verfahrensvariante (a) oder (b) umsetzt oder

b) eine Verbindung der Formel XV

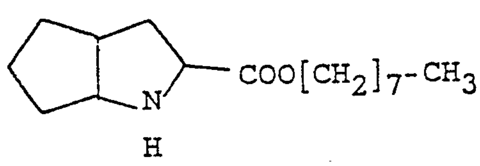

(XV)

analog Anspruch 10, Verfahrensvariante (f) mit einem aminogeschützten Alanin umsetzt und

anschließend die Aminoschutzgruppe abspaltet und gewünschtenfalls das all-S-Isomer der Formel XII isoliert.